# EUROPEAN PATENT SPECIFICATION

(11) **EP 1 682 577 B1**
(45) Date of publication and mention of the grant of the patent: **18.04.2012**
(21) Application number: 04800492.3
(22) Date of filing: 01.11.2004
(51) Int. Cl.: C07K 14/705, G01N 33/52, G01N 33/566, G01N 33/53

(54) **CELL-BASED ASSAYS EMPLOYING VOLTAGE AND CALCIUM DYES**
ASSAYS AUF ZELLBASIS UNTER ANWENDUNG VON SPANNUNG UND CALCIUMFARBSTOFFEN
ANALYSES CELLULAIRES UTILISANT LA TENSION ET DES COLORANTS A BASE DE CALCIUM

(30) Priority: 30.10.2003 US 515442 P; 20.07.2004 US 589012 P
(43) Date of publication of application: 26.07.2006
(73) Proprietor: Atto Bioscience, Inc., Rockville, MD 20850 (US)
(72) Inventor: YAO, Yong, Rockville, MD 20850 (US); CAO, Liang, Rockville, MD 20850 (US); LU, Jianming, Rockville, MD 20850 (US); LLORENTE, Isabel, Rockville, MD 20850 (US)
(74) Representative: Tollervey, Rebecca Marie
(86) International application number: PCT/US2004/036022
(87) International publication number: WO 2005/041758

(56) References cited:
- WO-A2-98/58074
- WO-A2-03/038039
- US-A- 6 113 856
- US-A1- 2003 157 571
- GAVAZZO PAOLA ET AL: "A point mutation in the pore region alters gating, Ca2+ blockage, and permeation of olfactory cyclic nucleotide-gated channels" JOURNAL OF GENERAL PHYSIOLOGY, vol. 116, no. 3, September 2000 (2000-09), pages 311-325, XP002412015 ISSN: 0022-1295
- SCOTT S P ET AL: 'Three Residues Predicted by Molecular Modeling To Interact with the Purine Moiety Alter Ligand Binding and Channel Gating in Cyclic Nucleotide-Gated Channels.' BIOCHEMISTRY. vol. 37, 15 September 1998, pages 17239 - 17252, XP002995967

### SEQUENCE LISTING

<110> BD Biosciences Inc.
<120> Novel Cell-Based Assays Employing Voltage and Calcium Dyes
<130> 062614-5002-WO
<150> US 60/589,012
   <151> 2004-07-20
<150> US 60/515,442
   <151> 2003-10-30
<160> 23
<170> PatentIn version 3.1
<210> 1
   <211> 1995
   <212> DNA
   <213> Rattus norvegicus
<220>
   <221> CDS
   <222> (1) .. (1992)
   <223>
<400> 1
   atg atg acc gaa aaa tcc aat ggt gtg aaa agc tct cca gct aat aac       48
   Met Met Thr Glu Lys Ser Asn Gly Val Lys Ser Ser Pro Ala Asn Asn
   1               5                   10                  15
   cat aac cat cat cct cct cct tct atc aag gcc aat ggc aaa gat gac       96
   His Asn His His Pro Pro Pro Ser Ile Lys Ala Asn Gly Lys Asp Asp
               20                  25                  30
   cac agg gca gga agc aga cca cag tct gtg gca gct gat gat gac act      144
   His Arg Ala Gly Ser Arg Pro Gln Ser Val Ala Ala Asp Asp Asp Thr
           35                  40                  45
   tct cca gaa cta caa agg ctg gca gag atg gat acc cct cgg agg ggg      192
   Ser Pro Glu Leu Gln Arg Leu Ala Glu Met Asp Thr Pro Arg Arg Gly
       50                  55                  60
   agg ggt ggc ttc caa agg att gtt cgc ctg gtg ggg gtc atc agg gac      240
   Arg Gly Gly Phe Gln Arg Ile Val Arg Leu Val Gly Val Ile Arg Asp
   65                  70                  75                  80
   tgg gcc aac aag aat ttc cgt gaa gag gaa cca agg cct gac tcc ttc      288
   Trp Ala Asn Lys Asn Phe Arg Glu Glu Glu Pro Arg Pro Asp Ser Phe
                   85                  90                  95
   cta gag cgt ttc cgt ggg cca gaa ctc cag act gtg aca acc cat cag      336
   Leu Glu Arg Phe Arg Gly Pro Glu Leu Gln Thr Val Thr Thr His Gln
   100 105 110
   ggg gat gac aaa ggc ggc aag gac ggc gag gga aag ggc acc aaa aag 384
   Gly Asp Asp Lys Gly Gly Lys Asp Gly Glu Gly Lys Gly Thr Lys Lys
   115 120 125
   aaa ttt gaa ctg ttt gtt ttg gac cca gcc gga gac tgg tat tac cgt 432
   Lys Phe Glu Leu Phe Val Leu Asp Pro Ala Gly Asp Trp Tyr Tyr Arg
   130 135 140
   tgg ttg ttt gtc att gcc atg cct gtt ctt tac aac tgg tgc ctg ttg 480
   Trp Leu Phe Val Ile Ala Met Pro Val Leu Tyr Asn Trp Cys Leu Leu
   145 150 155 160
   gtg gcc aga gcc tgc ttc agt gat cta cag aga aac tat ttt gtg gta 528
   Val Ala Arg Ala Cys Phe Ser Asp Leu Gln Arg Asn Tyr Phe Val Val
   165 170 175
   tgg ctg gtg ctg gac tac ttc tca gac act gtc tat atc gca gac ctc 576
   Trp Leu Val Leu Asp Tyr Phe Ser Asp Thr Val Tyr Ile Ala Asp Leu
   180 185 190
   atc att cgg ctg cgc aca ggc ttc cta gaa cag ggg ctc ttg gtc aaa 624
   Ile Ile Arg Leu Arg Thr Gly Phe Leu Glu Gln Gly Leu Leu Val Lys
   195 200 205
   gat ccc aag aaa ttg cga gac aac tat att cac act ttg cag ttc aaa 672
   Asp Pro Lys Lys Leu Arg Asp Asn Tyr Ile His Thr Leu Gln Phe Lys
   210 215 220
   ttg gat gtg gct tct atc att ccc act gac ctt atc tat ttt gct gtg 720
   Leu Asp Val Ala Ser Ile Ile Pro Thr Asp Leu Ile Tyr Phe Ala Val
   225 230 235 240
   ggt atc cac agc cct gag gta cgc ttc aac cgt cta tta cac ttt gcc 768
   Gly Ile His Ser Pro Glu Val Arg Phe Asn Arg Leu Leu His Phe Ala
   245 250 255
   cgt atg ttt gag ttc ttt gac cgc act gag aca cgc acc agc tac ccc 816
   Arg Met Phe Glu Phe Phe Asp Arg Thr Glu Thr Arg Thr Ser Tyr Pro
   260 265 270
   aac atc ttc cga atc agc aat ctg gtc ctt tac atc ttg gtc atc atc 864
   Asn Ile Phe Arg Ile Ser Asn Leu Val Leu Tyr Ile Leu Val Ile Ile
   275 280 285
   cac tgg aat gct tgt att tat tat gtt att tct aag tcc att ggc ttt 912
   His Trp Asn Ala Cys Ile Tyr Tyr Val Ile Ser Lys Ser Ile Gly Phe
   290 295 300
   gga gtt gac acc tgg gtt tac ccc aac att act gac cct gaa tat ggc 960
   Gly Val Asp Thr Trp Val Tyr Pro Asn Ile Thr Asp Pro Glu Tyr Gly
   305 310 315 320
   tac ctg gct aga gag tac att tac tgt ctt tac tgg tcc aca ctg acc 1008
   Tyr Leu Ala Arg Glu Tyr Ile Tyr Cys Leu Tyr Trp Ser Thr Leu Thr
   325 330 335
   ctc acc acc att gga gag aca cca ccc cct gta aag gat gag gag tac 1056
   Leu Thr Thr Ile Gly Glu Thr Pro Pro Pro Val Lys Asp Glu Glu Tyr
   340 345 350
   cta ttt gtc atc ttt gac ttc ttg att ggt gtc ctc atc ttt gcc act      1104
   Leu Phe Val Ile Phe Asp Phe Leu Ile Gly Val Leu Ile Phe Ala Thr
           355                 360                 365
   att gtg gga aat gtg ggc tcc atg atc tcc aac atg aat gcc aca cga      1152
   Ile Val Gly Asn Val Gly Ser Met Ile Ser Asn Met Asn Ala Thr Arg
       370                 375                 380
   gca gag ttc cag gcc aag att gat gct gtc aaa cac tac atg cag ttc      1200
   Ala Glu Phe Gln Ala Lys Ile Asp Ala Val Lys His Tyr Met Gln Phe
   385                 390                 395                 400
   cga aag gtc agc aaa gac atg gaa gcc aag gtc atc aaa tgg ttt gac      1248
   Arg Lys Val Ser Lys Asp Met Glu Ala Lys Val Ile Lys Trp Phe Asp
                   405                 410                 415
   tac ttg tgg acc aat aag aag aca gta gat gaa cga gaa gtc ctc aag      1296
   Tyr Leu Trp Thr Asn Lys Lys Thr Val Asp Glu Arg Glu Val Leu Lys
               420                 425                 430
   aac ctg cca gca aag ctc agg gct gag ata gcc att aat gtt cac ttg      1344
   Asn Leu Pro Ala Lys Leu Arg Ala Glu Ile Ala Ile Asn Val His Leu
           435                 440                 445
   tcc act ctg aag aaa gtg cgc ata ttc cag gat tgt gaa gct ggc cta      1392
   Ser Thr Leu Lys Lys Val Arg Ile Phe Gln Asp Cys Glu Ala Gly Leu
       450                 455                 460
   ctg gtg gaa ctg gta ctg aag ctt cgt cct cag gtc ttt agt cct gga      1440
   Leu Val Glu Leu Val Leu Lys Leu Arg Pro Gln Val Phe Ser Pro Gly
   465                 470                 475                 480
   gat tat att tgc cgt aag ggg gac att ggc aag gaa atg tac atc atc      1488
   Asp Tyr Ile Cys Arg Lys Gly Asp Ile Gly Lys Glu Met Tyr Ile Ile
                   485                 490                 495
   aag gag ggc aag ttg gca gtg gta gct gat gat ggc gtg act cag tat      1536
   Lys Glu Gly Lys Leu Ala Val Val Ala Asp Asp Gly Val Thr Gln Tyr
               500                 505                 510
   gcc ttg ctc tca gct ggg agc tgc ttt ggt gag att agt atc ctt aac      1584
   Ala Leu Leu Ser Ala Gly Ser Cys Phe Gly Glu Ile Ser Ile Leu Asn
           515                 520                 525
   att aag ggt agc aaa atg ggc aat cga cgt act gct aat atc cgt agc      1632
   Ile Lys Gly Ser Lys Met Gly Asn Arg Arg Thr Ala Asn Ile Arg Ser
       530                 535                 540
   ctg ggc tac tca gat ctc ttc tgc ttg tcc aag gac gat ctt atg gaa      1680
   Leu Gly Tyr Ser Asp Leu Phe Cys Leu Ser Lys Asp Asp Leu Met Glu
   545                 550                 555                 560
   gct gta act gag tat cct gat gcc aag aag gtc ctg gag gaa cgg ggt      1728
   Ala Val Thr Glu Tyr Pro Asp Ala Lys Lys Val Leu Glu Glu Arg Gly
                   565                 570                 575
   agg gag atc ctg atg aag gaa ggt cta ctg gat gag aat gaa gtg gca      1776
   Arg Glu Ile Leu Met Lys Glu Gly Leu Leu Asp Glu Asn Glu Val Ala
               580                 585                 590
   gct agt atg gag gta gat gtt cag gag aag ctg gaa cag ttg gag aca        1824
   Ala Ser Met Glu Val Asp Val Gln Glu Lys Leu Glu Gln Leu Glu Thr
           595                 600                 605
   aac atg gat acc ttg tac act cgc ttt gcc cgc ctg ctg gct gag tac        1872
   Asn Met Asp Thr Leu Tyr Thr Arg Phe Ala Arg Leu Leu Ala Glu Tyr
       610                 615                 620
   act ggg gcc cag cag aag ctc aag caa cgc atc aca gtg cta gag acc        1920
   Thr Gly Ala Gln Gln Lys Leu Lys Gln Arg Ile Thr Val Leu Glu Thr
   625                 630                 635                 640
   aag atg aaa cag aac cat gag gat gat tat cta tca gat ggg ata aac        1968
   Lys Met Lys Gln Asn His Glu Asp Asp Tyr Leu Ser Asp Gly Ile Asn
                   645                 650                 655
   act cct gag cca act gct gct gaa taa                                    1995
   Thr Pro Glu Pro Thr Ala Ala Glu
               660
<210> 2
   <211> 664
   <212> PRT
   <213> Rattus norvegicus
<400> 2
   Met Met Thr Glu Lys Ser Asn Gly Val Lys Ser Ser Pro Ala Asn Asn
   1 5 10 15
   His Asn His His Pro Pro Pro Ser Ile Lys Ala Asn Gly Lys Asp Asp
   20 25 30
   His Arg Ala Gly Ser Arg Pro Gln Ser Val Ala Ala Asp Asp Asp Thr
   35 40 45
   Ser Pro Glu Leu Gln Arg Leu Ala Glu Met Asp Thr Pro Arg Arg Gly
   50 55 60
   Arg Gly Gly Phe Gln Arg Ile Val Arg Leu Val Gly Val Ile Arg Asp
   65 70 75 80
   Trp Ala Asn Lys Asn Phe Arg Glu Glu Glu Pro Arg Pro Asp Ser Phe
   85 90 95
   Leu Glu Arg Phe Arg Gly Pro Glu Leu Gln Thr Val Thr Thr His Gln
   100 105 110
   Gly Asp Asp Lys Gly Gly Lys Asp Gly Glu Gly Lys Gly Thr Lys Lys
   115 120 125
   Lys Phe Glu Leu Phe Val Leu Asp Pro Ala Gly Asp Trp Tyr Tyr Arg
   130 135 140
   Trp Leu Phe Val Ile Ala Met Pro Val Leu Tyr Asn Trp Cys Leu Leu
   145 150 155 160
   Val Ala Arg Ala Cys Phe Ser Asp Leu Gln Arg Asn Tyr Phe Val Val
   165 170 175
   Trp Leu Val Leu Asp Tyr Phe Ser Asp Thr Val Tyr Ile Ala Asp Leu
   180 185 190
   Ile Ile Arg Leu Arg Thr Gly Phe Leu Glu Gln Gly Leu Leu Val Lys
   195 200 205
   Asp Pro Lys Lys Leu Arg Asp Asn Tyr Ile His Thr Leu Gln Phe Lys
   210 215 220
   Leu Asp Val Ala Ser Ile Ile Pro Thr Asp Leu Ile Tyr Phe Ala Val
   225 230 235 240
   Gly Ile His Ser Pro Glu Val Arg Phe Asn Arg Leu Leu His Phe Ala
   245 250 255
   Arg Met Phe Glu Phe Phe Asp Arg Thr Glu Thr Arg Thr Ser Tyr Pro
   260 265 270
   Asn Ile Phe Arg Ile Ser Asn Leu Val Leu Tyr Ile Leu Val Ile Ile
   275 280 285
   His Trp Asn Ala Cys Ile Tyr Tyr Val Ile Ser Lys Ser Ile Gly Phe
   290 295 300
   Gly Val Asp Thr Trp Val Tyr Pro Asn Ile Thr Asp Pro Glu Tyr Gly
   305 310 315 320
   Tyr Leu Ala Arg Glu Tyr Ile Tyr Cys Leu Tyr Trp Ser Thr Leu Thr
   325 330 335
   Leu Thr Thr Ile Gly Glu Thr Pro Pro Pro Val Lys Asp Glu Glu Tyr
   340 345 350
   Leu Phe Val Ile Phe Asp Phe Leu Ile Gly Val Leu Ile Phe Ala Thr
   355 360 365
   Ile Val Gly Asn Val Gly Ser Met Ile Ser Asn Met Asn Ala Thr Arg
   370 375 380
   Ala Glu Phe Gln Ala Lys Ile Asp Ala Val Lys His Tyr Met Gln Phe
   385 390 395 400
   Arg Lys Val Ser Lys Asp Met Glu Ala Lys Val Ile Lys Trp Phe Asp
   405 410 415
   Tyr Leu Trp Thr Asn Lys Lys Thr Val Asp Glu Arg Glu Val Leu Lys
   420 425 430
   Asn Leu Pro Ala Lys Leu Arg Ala Glu Ile Ala Ile Asn Val His Leu
   435 440 445
   Ser Thr Leu Lys Lys Val Arg Ile Phe Gln Asp Cys Glu Ala Gly Leu
   450 455 460
   Leu Val Glu Leu Val Leu Lys Leu Arg Pro Gln Val Phe Ser Pro Gly
   465 470 475 480
   Asp Tyr Ile Cys Arg Lys Gly Asp Ile Gly Lys Glu Met Tyr Ile Ile
   485 490 495
   Lys Glu Gly Lys Leu Ala Val Val Ala Asp Asp Gly Val Thr Gln Tyr
   500 505 510
   Ala Leu Leu Ser Ala Gly Ser Cys Phe Gly Glu Ile Ser Ile Leu Asn
           515                 520                 525
   Ile Lys Gly Ser Lys Met Gly Asn Arg Arg Thr Ala Asn Ile Arg Ser
       530                 535                 540
   Leu Gly Tyr Ser Asp Leu Phe Cys Leu Ser Lys Asp Asp Leu Met Glu
   545                 550                 555                 560
   Ala Val Thr Glu Tyr Pro Asp Ala Lys Lys Val Leu Glu Glu Arg Gly
                   565                 570                 575
   Arg Glu Ile Leu Met Lys Glu Gly Leu Leu Asp Glu Asn Glu Val Ala
               580                 585                 590
   Ala Ser Met Glu Val Asp Val Gln Glu Lys Leu Glu Gln Leu Glu Thr
           595                 600                 605
   Asn Met Asp Thr Leu Tyr Thr Arg Phe Ala Arg Leu Leu Ala Glu Tyr
       610                 615                 620
   Thr Gly Ala Gln Gln Lys Leu Lys Gln Arg Ile Thr Val Leu Glu Thr
   625                 630                 635                 640
   Lys Met Lys Gln Asn His Glu Asp Asp Tyr Leu Ser Asp Gly Ile Asn
                   645                 650                 655
   Thr Pro Glu Pro Thr Ala Ala Glu
               660
<210> 3
   <211> 1995
   <212> DNA
   <213> Rattus norvegicus
<220>
   <221> CDS
   <222> (1) .. (1992)
   <223>
<400> 3
   atg atg acc gaa aaa tcc aat ggt gtg aaa agc tct cca gct aat aac      48
   Met Met Thr Glu Lys Ser Asn Gly Val Lys Ser Ser Pro Ala Asn Asn
   1               5                   10                  15
   cat aac cat cat cct cct cct tct atc aag gcc aat ggc aaa gat gac      96
   His Asn His His Pro Pro Pro Ser Ile Lys Ala Asn Gly Lys Asp Asp
               20                  25                  30
   cac agg gca gga agc aga cca cag tct gtg gca gct gat gat gac act     144
   His Arg Ala Gly Ser Arg Pro Gln Ser Val Ala Ala Asp Asp Asp Thr
           35                  40                  45
   tct cca gaa cta caa agg ctg gca gag atg gat acc cct cgg agg ggg     192
   Ser Pro Glu Leu Gln Arg Leu Ala Glu Met Asp Thr Pro Arg Arg Gly
       50                  55                  60
   agg ggt ggc ttc caa agg att gtt cgc ctg gtg ggg gtc atc agg gac     240
   Arg Gly Gly Phe Gln Arg Ile Val Arg Leu Val Gly Val Ile Arg Asp
   65                  70                  75                  80
   tgg gcc aac aag aat ttc cgt gaa gag gaa cca agg cct gac tcc ttc      288
   Trp Ala Asn Lys Asn Phe Arg Glu Glu Glu Pro Arg Pro Asp Ser Phe
                   85                  90                  95
   cta gag cgt ttc cgt ggg cca gaa ctc cag act gtg aca acc cat cag      336
   Leu Glu Arg Phe Arg Gly Pro Glu Leu Gln Thr Val Thr Thr His Gln
               100                 105                 110
   ggg gat gac aaa ggc ggc aag gac ggc gag gga aag ggc acc aaa aag      384
   Gly Asp Asp Lys Gly Gly Lys Asp Gly Glu Gly Lys Gly Thr Lys Lys
           115                 120                 125
   aaa ttt gaa ctg ttt gtt ttg gac cca gcc gga gac tgg tat tac cgt      432
   Lys Phe Glu Leu Phe Val Leu Asp Pro Ala Gly Asp Trp Tyr Tyr Arg
       130                 135                 140
   tgg ttg ttt gtc att gcc atg cct gtt ctt tac aac tgg tgc ctg ttg      480
   Trp Leu Phe Val Ile Ala Met Pro Val Leu Tyr Asn Trp Cys Leu Leu
   145                 150                 155                 160
   gtg gcc aga gcc tgc ttc agt gat cta cag aga aac tat ttt gtg gta      528
   Val Ala Arg Ala Cys Phe Ser Asp Leu Gln Arg Asn Tyr Phe Val Val
                   165                 170                 175
   tgg ctg gtg ctg gac tac ttc tca gac act gtc tat atc gca gac ctc      576
   Trp Leu Val Leu Asp Tyr Phe Ser Asp Thr Val Tyr Ile Ala Asp Leu
               180                 185                 190
   atc att cgg ctg cgc aca ggc ttc cta gaa cag ggg ctc ttg gtc aaa      624
   Ile Ile Arg Leu Arg Thr Gly Phe Leu Glu Gln Gly Leu Leu Val Lys
           195                 200                 205
   gat ccc aag aaa ttg cga gac aac tat att cac act ttg cag ttc aaa      672
   Asp Pro Lys Lys Leu Arg Asp Asn Tyr Ile His Thr Leu Gln Phe Lys
       210                 215                 220
   ttg gat gtg gct tct atc att ccc act gac ctt atc tat ttt gct gtg      720
   Leu Asp Val Ala Ser Ile Ile Pro Thr Asp Leu Ile Tyr Phe Ala Val
   225                 230                 235                 240
   ggt atc cac agc cct gag gta cgc ttc aac cgt cta tta cac ttt gcc      768
   Gly Ile His Ser Pro Glu Val Arg Phe Asn Arg Leu Leu His Phe Ala
                   245                 250                 255
   cgt atg ttt gag ttc ttt gac cgc act gag aca cgc acc agc tac ccc      816
   Arg Met Phe Glu Phe Phe Asp Arg Thr Glu Thr Arg Thr Ser Tyr Pro
               260                 265                 270
   aac atc ttc cga atc agc aat ctg gtc ctt tac atc ttg gtc atc atc      864
   Asn Ile Phe Arg Ile Ser Asn Leu Val Leu Tyr Ile Leu Val Ile Ile
           275                 280                 285
   cac tgg aat gct tgt att tat tat gtt att tct aag tcc att ggc ttt      912
   His Trp Asn Ala Cys Ile Tyr Tyr Val Ile Ser Lys Ser Ile Gly Phe
       290                 295                 300
   gga gtt gac acc tgg gtt tac ccc aac att act gac cct gaa tat ggc      960
   Gly Val Asp Thr Trp Val Tyr Pro Asn Ile Thr Asp Pro Glu Tyr Gly
   305                 310                 315                 320
   tac ctg gct aga gag tac att tac tgt ctt tac tgg tcc aca ctg acc      1008
   Tyr Leu Ala Arg Glu Tyr Ile Tyr Cys Leu Tyr Trp Ser Thr Leu Thr
                   325                 330                 335
   ctc acc acc att gga gag aca cca ccc cct gta aag gat gag gag tac      1056
   Leu Thr Thr Ile Gly Glu Thr Pro Pro Pro Val Lys Asp Glu Glu Tyr
               340                 345                 350
   cta ttt gtc atc ttt gac ttc ttg att ggt gtc ctc atc ttt gcc act      1104
   Leu Phe Val Ile Phe Asp Phe Leu Ile Gly Val Leu Ile Phe Ala Thr
           355                 360                 365
   att gtg gga aat gtg ggc tcc atg atc tcc aac atg aat gcc aca cga      1152
   Ile Val Gly Asn Val Gly Ser Met Ile Ser Asn Met Asn Ala Thr Arg
       370                 375                 380
   gca gag ttc cag gcc aag att gat gct gtc aaa cac tac atg cag ttc      1200
   Ala Glu Phe Gln Ala Lys Ile Asp Ala Val Lys His Tyr Met Gln Phe
   385                 390                 395                 400
   cga aag gtc agc aaa gac atg gaa gcc aag gtc atc aaa tgg ttt gac      1248
   Arg Lys Val Ser Lys Asp Met Glu Ala Lys Val Ile Lys Trp Phe Asp
                   405                 410                 415
   tac ttg tgg acc aat aag aag aca gta gat gaa cga gaa gtc ctc aag      1296
   Tyr Leu Trp Thr Asn Lys Lys Thr Val Asp Glu Arg Glu Val Leu Lys
               420                 425                 430
   aac ctg cca gca aag ctc agg gct gag ata gcc att aat gtt cac ttg      1344
   Asn Leu Pro Ala Lys Leu Arg Ala Glu Ile Ala Ile Asn Val His Leu
           435                 440                 445
   tcc act ctg aag aaa gtg cgc ata ttc cag gat tgt gaa gct ggc cta      1392
   Ser Thr Leu Lys Lys Val Arg Ile Phe Gln Asp Cys Glu Ala Gly Leu
       450                 455                 460
   ctg gtg gaa ctg gta ctg aag ctt cgt cct cag gtc ttt agt cct gga      1440
   Leu Val Glu Leu Val Leu Lys Leu Arg Pro Gln Val Phe Ser Pro Gly
   465                 470                 475                 480
   gat tat att tgc cgt aag ggg gac att ggc aag gaa atg tac atc atc      1488
   Asp Tyr Ile Cys Arg Lys Gly Asp Ile Gly Lys Glu Met Tyr Ile Ile
                   485                 490                 495
   aag gag ggc aag ttg gca gtg gta gct gat gat ggc gtg act cag tat      1536
   Lys Glu Gly Lys Leu Ala Val Val Ala Asp Asp Gly Val Thr Gln Tyr
               500                 505                 510
   gcc ttg ctc tca gct ggg agc tgc ttt ggt gag att agt atc ctt aac      1584
   Ala Leu Leu Ser Ala Gly Ser Cys Phe Gly Glu Ile Ser Ile Leu Asn
           515                 520                 525
   att aag ggt agc aaa atg ggc aat cga cgt act gct aat atc cgt agc      1632
   Ile Lys Gly Ser Lys Met Gly Asn Arg Arg Thr Ala Asn Ile Arg Ser
       530                 535                 540
   ctg ggc tac tca gat ctc ttc tgc ttg tcc aag gac gat ctt atg gaa      1680
   Leu Gly Tyr Ser Asp Leu Phe Cys Leu Ser Lys Asp Asp Leu Met Glu
   545                 550                 555                 560
   gct gta act gag gct cct gat gcc aag aag gtc ctg gag gaa cgg ggt      1728
   Ala Val Thr Glu Ala Pro Asp Ala Lys Lys Val Leu Glu Glu Arg Gly
                   565                 570                 575
   agg gag atc ctg atg aag gaa ggt cta ctg gat gag aat gaa gtg gca      1776
   Arg Glu Ile Leu Met Lys Glu Gly Leu Leu Asp Glu Asn Glu Val Ala
               580                 585                 590
   gct agt atg gag gta gat gtt cag gag aag ctg gaa cag ttg gag aca      1824
   Ala Ser Met Glu Val Asp Val Gln Glu Lys Leu Glu Gln Leu Glu Thr
           595                 600                 605
   aac atg gat acc ttg tac act cgc ttt gcc cgc ctg ctg gct gag tac      1872
   Asn Met Asp Thr Leu Tyr Thr Arg Phe Ala Arg Leu Leu Ala Glu Tyr
       610                 615                 620
   act ggg gcc cag cag aag ctc aag caa cgc atc aca gtg cta gag acc      1920
   Thr Gly Ala Gln Gln Lys Leu Lys Gln Arg Ile Thr Val Leu Glu Thr
   625                 630                 635                 640
   aag atg aaa cag aac cat gag gat gat tat cta tca gat ggg ata aac      1968
   Lys Met Lys Gln Asn His Glu Asp Asp Tyr Leu Ser Asp Gly Ile Asn
                   645                 650                 655
   act cct gag cca act gct gct gaa taa                                  1995
   Thr Pro Glu Pro Thr Ala Ala Glu
               660
<210> 4
   <211> 664
   <212> PRT
   <213> Rattus norvegicus
<400> 4
   Met Met Thr Glu Lys Ser Asn Gly Val Lys Ser Ser Pro Ala Asn Asn
   1               5                   10                  15
   His Asn His His Pro Pro Pro Ser Ile Lys Ala Asn Gly Lys Asp Asp
               20                  25                  30
   His Arg Ala Gly Ser Arg Pro Gln Ser Val Ala Ala Asp Asp Asp Thr
           35                  40                  45
   Ser Pro Glu Leu Gln Arg Leu Ala Glu Met Asp Thr Pro Arg Arg Gly
       50                  55                  60
   Arg Gly Gly Phe Gln Arg Ile Val Arg Leu Val Gly Val Ile Arg Asp
   65                  70                  75                  80
   Trp Ala Asn Lys Asn Phe Arg Glu Glu Glu Pro Arg Pro Asp Ser Phe
                   85                  90                  95
   Leu Glu Arg Phe Arg Gly Pro Glu Leu Gln Thr Val Thr Thr His Gln
               100                 105                 110
   Gly Asp Asp Lys Gly Gly Lys Asp Gly Glu Gly Lys Gly Thr Lys Lys
           115                 120                 125
   Lys Phe Glu Leu Phe Val Leu Asp Pro Ala Gly Asp Trp Tyr Tyr Arg
       130                 135                 140
   Trp Leu Phe Val Ile Ala Met Pro Val Leu Tyr Asn Trp Cys Leu Leu
   145 150 155 160
   Val Ala Arg Ala Cys Phe Ser Asp Leu Gln Arg Asn Tyr Phe Val Val
   165 170 175
   Trp Leu Val Leu Asp Tyr Phe Ser Asp Thr Val Tyr Ile Ala Asp Leu
   180 185 190
   Ile Ile Arg Leu Arg Thr Gly Phe Leu Glu Gln Gly Leu Leu Val Lys
   195 200 205
   Asp Pro Lys Lys Leu Arg Asp Asn Tyr Ile His Thr Leu Gln Phe Lys
   210 215 220
   Leu Asp Val Ala Ser Ile Ile Pro Thr Asp Leu Ile Tyr Phe Ala Val
   225 230 235 240
   Gly Ile His Ser Pro Glu Val Arg Phe Asn Arg Leu Leu His Phe Ala
   245 250 255
   Arg Met Phe Glu Phe Phe Asp Arg Thr Glu Thr Arg Thr Ser Tyr Pro
   260 265 270
   Asn Ile Phe Arg Ile Ser Asn Leu Val Leu Tyr Ile Leu Val Ile Ile
   275 280 285
   His Trp Asn Ala Cys Ile Tyr Tyr Val Ile Ser Lys Ser Ile Gly Phe
   290 295 300
   Gly Val Asp Thr Trp Val Tyr Pro Asn Ile Thr Asp Pro Glu Tyr Gly
   305 310 315 320
   Tyr Leu Ala Arg Glu Tyr Ile Tyr Cys Leu Tyr Trp Ser Thr Leu Thr
   325 330 335
   Leu Thr Thr Ile Gly Glu Thr Pro Pro Pro Val Lys Asp Glu Glu Tyr
   340 345 350
   Leu Phe Val Ile Phe Asp Phe Leu Ile Gly Val Leu Ile Phe Ala Thr
   355 360 365
   Ile Val Gly Asn Val Gly Ser Met Ile Ser Asn Met Asn Ala Thr Arg
   370 375 380
   Ala Glu Phe Gln Ala Lys Ile Asp Ala Val Lys His Tyr Met Gln Phe
   385 390 395 400
   Arg Lys Val Ser Lys Asp Met Glu Ala Lys Val Ile Lys Trp Phe Asp
   405 410 415
   Tyr Leu Trp Thr Asn Lys Lys Thr Val Asp Glu Arg Glu Val Leu Lys
   420 425 430
   Asn Leu Pro Ala Lys Leu Arg Ala Glu Ile Ala Ile Asn Val His Leu
   435 440 445
   Ser Thr Leu Lys Lys Val Arg Ile Phe Gln Asp Cys Glu Ala Gly Leu
   450 455 460
   Leu Val Glu Leu Val Leu Lys Leu Arg Pro Gln Val Phe Ser Pro Gly
   465                 470                 475                 480
   Asp Tyr Ile Cys Arg Lys Gly Asp Ile Gly Lys Glu Met Tyr Ile Ile
                   485                 490                 495
   Lys Glu Gly Lys Leu Ala Val Val Ala Asp Asp Gly Val Thr Gln Tyr
               500                 505                 510
   Ala Leu Leu Ser Ala Gly Ser Cys Phe Gly Glu Ile Ser Ile Leu Asn
           515                 520                 525
   Ile Lys Gly Ser Lys Met Gly Asn Arg Arg Thr Ala Asn Ile Arg Ser
       530                 535                 540
   Leu Gly Tyr Ser Asp Leu Phe Cys Leu Ser Lys Asp Asp Leu Met Glu
   545                 550                 555                 560
   Ala Val Thr Glu Ala Pro Asp Ala Lys Lys Val Leu Glu Glu Arg Gly
                   565                 570                 575
   Arg Glu Ile Leu Met Lys Glu Gly Leu Leu Asp Glu Asn Glu Val Ala
               580                 585                 590
   Ala Ser Met Glu Val Asp Val Gln Glu Lys Leu Glu Gln Leu Glu Thr
           595                 600                 605
   Asn Met Asp Thr Leu Tyr Thr Arg Phe Ala Arg Leu Leu Ala Glu Tyr
       610                 615                 620
   Thr Gly Ala Gln Gln Lys Leu Lys Gln Arg Ile Thr Val Leu Glu Thr
   625                 630                 635                 640
   Lys Met Lys Gln Asn His Glu Asp Asp Tyr Leu Ser Asp Gly Ile Asn
                   645                 650                 655
   Thr Pro Glu Pro Thr Ala Ala Glu
               660
<210> 5
   <211> 1995
   <212> DNA
   <213> Rattus norvegicus
<220>
   <221> CDS
   <222> (1) .. (1992)
   <223>
<400> 5
   atg atg acc gaa aaa tcc aat ggt gtg aaa agc tct cca gct aat aac        48
   Met Met Thr Glu Lys Ser Asn Gly Val Lys Ser Ser Pro Ala Asn Asn
   1               5                   10                  15
   cat aac cat cat cct cct cct tct atc aag gcc aat ggc aaa gat gac        96
   His Asn His His Pro Pro Pro Ser Ile Lys Ala Asn Gly Lys Asp Asp
               20                  25                  30
   cac agg gca gga agc aga cca cag tct gtg gca gct gat gat gac act       144
   His Arg Ala Gly Ser Arg Pro Gln Ser Val Ala Ala Asp Asp Asp Thr
               35                  40                  45
   tct cca gaa cta caa agg ctg gca gag atg gat acc cct cgg agg ggg      192
   Ser Pro Glu Leu Gln Arg Leu Ala Glu Met Asp Thr Pro Arg Arg Gly
       50                  55                  60
   agg ggt ggc ttc caa agg att gtt cgc ctg gtg ggg gtc atc agg gac      240
   Arg Gly Gly Phe Gln Arg Ile Val Arg Leu Val Gly Val Ile Arg Asp
   65                  70                  75                  80
   tgg gcc aac aag aat ttc cgt gaa gag gaa cca agg cct gac tcc ttc      288
   Trp Ala Asn Lys Asn Phe Arg Glu Glu Glu Pro Arg Pro Asp Ser Phe
                   85                  90                  95
   cta gag cgt ttc cgt ggg cca gaa ctc cag act gtg aca acc cat cag      336
   Leu Glu Arg Phe Arg Gly Pro Glu Leu Gln Thr Val Thr Thr His Gln
               100                 105                 110
   ggg gat gac aaa ggc ggc aag gac ggc gag gga aag ggc acc aaa aag      384
   Gly Asp Asp Lys Gly Gly Lys Asp Gly Glu Gly Lys Gly Thr Lys Lys
           115                 120                 125
   aaa ttt gaa ctg ttt gtt ttg gac cca gcc gga gac tgg tat tac cgt      432
   Lys Phe Glu Leu Phe Val Leu Asp Pro Ala Gly Asp Trp Tyr Tyr Arg
       130                 135                 140
   tgg ttg ttt gtc att gcc atg cct gtt ctt tac aac tgg tgc ctg ttg      480
   Trp Leu Phe Val Ile Ala Met Pro Val Leu Tyr Asn Trp Cys Leu Leu
   145                 150                 155                 160
   gtg gcc aga gcc tgc ttc agt gat cta cag aga aac tat ttt gtg gta      528
   Val Ala Arg Ala Cys Phe Ser Asp Leu Gln Arg Asn Tyr Phe Val Val
                   165                 170                 175
   tgg ctg gtg ctg gac tac ttc tca gac act gtc tat atc gca gac ctc      576
   Trp Leu Val Leu Asp Tyr Phe Ser Asp Thr Val Tyr Ile Ala Asp Leu
               180                 185                 190
   atc att cgg ctg cgc aca ggc ttc cta gaa cag ggg ctc ttg gtc aaa      624
   Ile Ile Arg Leu Arg Thr Gly Phe Leu Glu Gln Gly Leu Leu Val Lys
           195                 200                 205
   gat ccc aag aaa ttg cga gac aac tat att cac act ttg cag ttc aaa      672
   Asp Pro Lys Lys Leu Arg Asp Asn Tyr Ile His Thr Leu Gln Phe Lys
       210                 215                 220
   ttg gat gtg gct tct atc att ccc act gac ctt atc tat ttt gct gtg      720
   Leu Asp Val Ala Ser Ile Ile Pro Thr Asp Leu Ile Tyr Phe Ala Val
   225                 230                 235                 240
   ggt atc cac agc cct gag gta cgc ttc aac cgt cta tta cac ttt gcc      768
   Gly Ile His Ser Pro Glu Val Arg Phe Asn Arg Leu Leu His Phe Ala
                   245                 250                 255
   cgt atg ttt gag ttc ttt gac cgc act gag aca cgc acc agc tac ccc      816
   Arg Met Phe Glu Phe Phe Asp Arg Thr Glu Thr Arg Thr Ser Tyr Pro
               260                 265                 270
   aac atc ttc cga atc agc aat ctg gtc ctt tac atc ttg gtc atc atc      864
   Asn Ile Phe Arg Ile Ser Asn Leu Val Leu Tyr Ile Leu Val Ile Ile
           275                 280                 285
   cac tgg aat gct tgt att tat tat gtt att tct aag tcc att ggc ttt      912
   His Trp Asn Ala Cys Ile Tyr Tyr Val Ile Ser Lys Ser Ile Gly Phe
       290                 295                 300
   gga gtt gac acc tgg gtt tac ccc aac att act gac cct gaa tat ggc      960
   Gly Val Asp Thr Trp Val Tyr Pro Asn Ile Thr Asp Pro Glu Tyr Gly
   305                 310                 315                 320
   tac ctg gct aga gag tac att tac tgt ctt tac tgg tcc aca ctg acc     1008
   Tyr Leu Ala Arg Glu Tyr Ile Tyr Cys Leu Tyr Trp Ser Thr Leu Thr
                   325                 330                 335
   ctc acc acc att gga gag aca cca ccc cct gta aag gat gag gag tac     1056
   Leu Thr Thr Ile Gly Glu Thr Pro Pro Pro Val Lys Asp Glu Glu Tyr
               340                 345                 350
   cta ttt gtc atc ttt gac ttc ttg att ggt gtc ctc atc ttt gcc act     1104
   Leu Phe Val Ile Phe Asp Phe Leu Ile Gly Val Leu Ile Phe Ala Thr
           355                 360                 365
   att gtg gga aat gtg ggc tcc atg atc tcc aac atg aat gcc aca cga     1152
   Ile Val Gly Asn Val Gly Ser Met Ile Ser Asn Met Asn Ala Thr Arg
       370                 375                 380
   gca gag ttc cag gcc aag att gat gct gtc aaa cac tac atg cag ttc     1200
   Ala Glu Phe Gln Ala Lys Ile Asp Ala Val Lys His Tyr Met Gln Phe
   385                 390                 395                 400
   cga aag gtc agc aaa gac atg gaa gcc aag gtc atc aaa tgg ttt gac     1248
   Arg Lys Val Ser Lys Asp Met Glu Ala Lys Val Ile Lys Trp Phe Asp
                   405                 410                 415
   tac ttg tgg acc aat aag aag aca gta gat gaa cga gaa gtc ctc aag     1296
   Tyr Leu Trp Thr Asn Lys Lys Thr Val Asp Glu Arg Glu Val Leu Lys
               420                 425                 430
   aac ctg cca gca aag ctc agg gct gag ata gcc att aat gtt cac ttg     1344
   Asn Leu Pro Ala Lys Leu Arg Ala Glu Ile Ala Ile Asn Val His Leu
           435                 440                 445
   tcc act ctg aag aaa gtg cgc ata ttc cag gat tgg gaa gct ggc cta     1392
   Ser Thr Leu Lys Lys Val Arg Ile Phe Gln Asp Trp Glu Ala Gly Leu
       450                 455                 460
   ctg gtg gaa ctg gta ctg aag ctt cgt cct cag gtc ttt agt cct gga     1440
   Leu Val Glu Leu Val Leu Lys Leu Arg Pro Gln Val Phe Ser Pro Gly
   465                 470                 475                 480
   gat tat att tgc cgt aag ggg gac att ggc aag gaa atg tac atc atc     1488
   Asp Tyr Ile Cys Arg Lys Gly Asp Ile Gly Lys Glu Met Tyr Ile Ile
                   485                 490                 495
   aag gag ggc aag ttg gca gtg gta gct gat gat ggc gtg act cag tat     1536
   Lys Glu Gly Lys Leu Ala Val Val Ala Asp Asp Gly Val Thr Gln Tyr
               500                 505                 510
   gcc ttg ctc tca gct ggg agc tgc ttt ggt gag att agt atc ctt aac     1584
   Ala Leu Leu Ser Ala Gly Ser Cys Phe Gly Glu Ile Ser Ile Leu Asn
           515                 520                 525
   att aag ggt agc aaa atg ggc aat cga cgt act gct aat atc cgt agc      1632
   Ile Lys Gly Ser Lys Met Gly Asn Arg Arg Thr Ala Asn Ile Arg Ser
       530                 535                 540
   ctg ggc tac tca gat ctc ttc tgc ttg tcc aag gac gat ctt atg gaa      1680
   Leu Gly Tyr Ser Asp Leu Phe Cys Leu Ser Lys Asp Asp Leu Met Glu
   545                 550                 555                 560
   gct gta act gag tat cct gat gcc aag aag gtc ctg gag gaa cgg ggt      1728
   Ala Val Thr Glu Tyr Pro Asp Ala Lys Lys Val Leu Glu Glu Arg Gly
                   565                 570                 575
   agg gag atc ctg atg aag atg ggt cta ctg gat gag aat gaa gtg gca      1776
   Arg Glu Ile Leu Met Lys Met Gly Leu Leu Asp Glu Asn Glu Val Ala
               580                 585                 590
   gct agt atg gag gta gat gtt cag gag aag ctg gaa cag ttg gag aca      1824
   Ala Ser Met Glu Val Asp Val Gln Glu Lys Leu Glu Gln Leu Glu Thr
           595                 600                 605
   aac atg gat acc ttg tac act cgc ttt gcc cgc ctg ctg gct gag tac      1872
   Asn Met Asp Thr Leu Tyr Thr Arg Phe Ala Arg Leu Leu Ala Glu Tyr
       610                 615                 620
   act ggg gcc cag cag aag ctc aag caa cgc atc aca gtg cta gag acc      1920
   Thr Gly Ala Gln Gln Lys Leu Lys Gln Arg Ile Thr Val Leu Glu Thr
   625                 630                 635                 640
   aag atg aaa cag aac cat gag gat gat tat cta tca gat ggg ata aac      1968
   Lys Met Lys Gln Asn His Glu Asp Asp Tyr Leu Ser Asp Gly Ile Asn
                   645                 650                 655
   act cct gag cca act gct gct gaa taa                                  1995
   Thr Pro Glu Pro Thr Ala Ala Glu
               660
<210> 6
   <211> 664
   <212> PRT
   <213> Rattus norvegicus
<400> 6
   Met Met Thr Glu Lys Ser Asn Gly Val Lys Ser Ser Pro Ala Asn Asn
   1               5                   10                  15
   His Asn His His Pro Pro Pro Ser Ile Lys Ala Asn Gly Lys Asp Asp
               20                  25                  30
   His Arg Ala Gly Ser Arg Pro Gln Ser Val Ala Ala Asp Asp Asp Thr
           35                  40                  45
   Ser Pro Glu Leu Gln Arg Leu Ala Glu Met Asp Thr Pro Arg Arg Gly
       50                  55                  60
   Arg Gly Gly Phe Gln Arg Ile Val Arg Leu Val Gly Val Ile Arg Asp
   65                  70                  75                  80
   Trp Ala Asn Lys Asn Phe Arg Glu Glu Glu Pro Arg Pro Asp Ser Phe
                   85                  90                  95
   Leu Glu Arg Phe Arg Gly Pro Glu Leu Gln Thr Val Thr Thr His Gln
               100                 105                 110
   Gly Asp Asp Lys Gly Gly Lys Asp Gly Glu Gly Lys Gly Thr Lys Lys
           115                 120                 125
   Lys Phe Glu Leu Phe Val Leu Asp Pro Ala Gly Asp Trp Tyr Tyr Arg
       130                 135                 140
   Trp Leu Phe Val Ile Ala Met Pro Val Leu Tyr Asn Trp Cys Leu Leu
   145                 150                   155                 160
   Val Ala Arg Ala Cys Phe Ser Asp Leu Gln Arg Asn Tyr Phe Val Val
                   165                 170                 175
   Trp Leu Val Leu Asp Tyr Phe Ser Asp Thr Val Tyr Ile Ala Asp Leu
               180                 185                 190
   Ile Ile Arg Leu Arg Thr Gly Phe Leu Glu Gln Gly Leu Leu Val Lys
           195                 200                 205
   Asp Pro Lys Lys Leu Arg Asp Asn Tyr Ile His Thr Leu Gln Phe Lys
       210                 215                 220
   Leu Asp Val Ala Ser Ile Ile Pro Thr Asp Leu Ile Tyr Phe Ala Val
   225                 230                 235                 240
   Gly Ile His Ser Pro Glu Val Arg Phe Asn Arg Leu Leu His Phe Ala
                   245                 250                 255
   Arg Met Phe Glu Phe Phe Asp Arg Thr Glu Thr Arg Thr Ser Tyr Pro
               260                 265                 270
   Asn Ile Phe Arg Ile Ser Asn Leu Val Leu Tyr Ile Leu Val Ile Ile
           275                 280                 285
   His Trp Asn Ala Cys Ile Tyr Tyr Val Ile Ser Lys Ser Ile Gly Phe
       290                 295                 300
   Gly Val Asp Thr Trp Val Tyr Pro Asn Ile Thr Asp Pro Glu Tyr Gly
   305                 310                 315                 320
   Tyr Leu Ala Arg Glu Tyr Ile Tyr Cys Leu Tyr Trp Ser Thr Leu Thr
                   325                 330                 335
   Leu Thr Thr Ile Gly Glu Thr Pro Pro Pro Val Lys Asp Glu Glu Tyr
               340                 345                 350
   Leu Phe Val Ile Phe Asp Phe Leu Ile Gly Val Leu Ile Phe Ala Thr
           355                 360                 365
   Ile Val Gly Asn Val Gly Ser Met Ile Ser Asn Met Asn Ala Thr Arg
       370                 375                 380
   Ala Glu Phe Gln Ala Lys Ile Asp Ala Val Lys His Tyr Met Gln Phe
   385                 390                 395                 400
   Arg Lys Val Ser Lys Asp Met Glu Ala Lys Val Ile Lys Trp Phe Asp
                   405                 410                 415
   Tyr Leu Trp Thr Asn Lys Lys Thr Val Asp Glu Arg Glu Val Leu Lys
   420 425 430
   Asn Leu Pro Ala Lys Leu Arg Ala Glu Ile Ala Ile Asn Val His Leu
   435 440 445
   Ser Thr Leu Lys Lys Val Arg Ile Phe Gln Asp Trp Glu Ala Gly Leu
   450 455 460
   Leu Val Glu Leu Val Leu Lys Leu Arg Pro Gln Val Phe Ser Pro Gly
   465 470 475 480
   Asp Tyr Ile Cys Arg Lys Gly Asp Ile Gly Lys Glu Met Tyr Ile Ile
   485 490 495
   Lys Glu Gly Lys Leu Ala Val Val Ala Asp Asp Gly Val Thr Gln Tyr
   500 505 510
   Ala Leu Leu Ser Ala Gly Ser Cys Phe Gly Glu Ile Ser Ile Leu Asn
   515 520 525
   Ile Lys Gly Ser Lys Met Gly Asn Arg Arg Thr Ala Asn Ile Arg Ser
   530 535 540
   Leu Gly Tyr Ser Asp Leu Phe Cys Leu Ser Lys Asp Asp Leu Met Glu
   545 550 555 560
   Ala Val Thr Glu Tyr Pro Asp Ala Lys Lys Val Leu Glu Glu Arg Gly
   565 570 575
   Arg Glu Ile Leu Met Lys Met Gly Leu Leu Asp Glu Asn Glu Val Ala
   580 585 590
   Ala Ser Met Glu Val Asp Val Gln Glu Lys Leu Glu Gln Leu Glu Thr
   595 600 605
   Asn Met Asp Thr Leu Tyr Thr Arg Phe Ala Arg Leu Leu Ala Glu Tyr
   610 615 620
   Thr Gly Ala Gln Gln Lys Leu Lys Gln Arg Ile Thr Val Leu Glu Thr
   625 630 635 640
   Lys Met Lys Gln Asn His Glu Asp Asp Tyr Leu Ser Asp Gly Ile Asn
   645 650 655
   Thr Pro Glu Pro Thr Ala Ala Glu
   660
<210> 7
   <211> 1995
   <212> DNA
   <213> Rattus norvegicus
<220>
   <221> CDS
   <222> (1) .. (1992)
   <223>
<400> 7
   atg atg acc gaa aaa tcc aat ggt gtg aaa agc tct cca gct aat aac        48
   Met Met Thr Glu Lys Ser Asn Gly Val Lys Ser Ser Pro Ala Asn Asn
   1               5                   10                  15
   cat aac cat cat cct cct cct tct atc aag gcc aat ggc aaa gat gac        96
   His Asn His His Pro Pro Pro Ser Ile Lys Ala Asn Gly Lys Asp Asp
               20                  25                  30
   cac agg gca gga agc aga cca cag tct gtg gca gct gat gat gac act       144
   His Arg Ala Gly Ser Arg Pro Gln Ser Val Ala Ala Asp Asp Asp Thr
           35                  40                  45
   tct cca gaa cta caa agg ctg gca gag atg gat acc cct cgg agg ggg       192
   Ser Pro Glu Leu Gln Arg Leu Ala Glu Met Asp Thr Pro Arg Arg Gly
       50                  55                  60
   agg ggt ggc ttc caa agg att gtt cgc ctg gtg ggg gtc atc agg gac       240
   Arg Gly Gly Phe Gln Arg Ile Val Arg Leu Val Gly Val Ile Arg Asp
   65                  70                  75                  80
   tgg gcc aac aag aat ttc cgt gaa gag gaa cca agg cct gac tcc ttc       288
   Trp Ala Asn Lys Asn Phe Arg Glu Glu Glu Pro Arg Pro Asp Ser Phe
                   85                  90                  95
   cta gag cgt ttc cgt ggg cca gaa ctc cag act gtg aca acc cat cag       336
   Leu Glu Arg Phe Arg Gly Pro Glu Leu Gln Thr Val Thr Thr His Gln
               100                 105                 110
   ggg gat gac aaa ggc ggc aag gac ggc gag gga aag ggc acc aaa aag       384
   Gly Asp Asp Lys Gly Gly Lys Asp Gly Glu Gly Lys Gly Thr Lys Lys
           115                 120                 125
   aaa ttt gaa ctg ttt gtt ttg gac cca gcc gga gac tgg tat tac cgt       432
   Lys Phe Glu Leu Phe Val Leu Asp Pro Ala Gly Asp Trp Tyr Tyr Arg
       130                 135                 140
   tgg ttg ttt gtc att gcc atg cct gtt ctt tac aac tgg tgc ctg ttg       480
   Trp Leu Phe Val Ile Ala Met Pro Val Leu Tyr Asn Trp Cys Leu Leu
   145                 150                 155                 160
   gtg gcc aga gcc tgc ttc agt gat cta cag aga aac tat ttt gtg gta       528
   Val Ala Arg Ala Cys Phe Ser Asp Leu Gln Arg Asn Tyr Phe Val Val
                   165                 170                 175
   tgg ctg gtg ctg gac tac ttc tca gac act gtc tat atc gca gac ctc       576
   Trp Leu Val Leu Asp Tyr Phe Ser Asp Thr Val Tyr Ile Ala Asp Leu
               180                 185                 190
   atc att cgg ctg cgc aca ggc ttc cta gaa cag ggg ctc ttg gtc aaa       624
   Ile Ile Arg Leu Arg Thr Gly Phe Leu Glu Gln Gly Leu Leu Val Lys
           195                 200                 205
   gat ccc aag aaa ttg cga gac aac tat att cac act ttg cag ttc aaa       672
   Asp Pro Lys Lys Leu Arg Asp Asn Tyr Ile His Thr Leu Gln Phe Lys
       210                 215                 220
   ttg gat gtg gct tct atc att ccc act gac ctt atc tat ttt gct gtg       720
   Leu Asp Val Ala Ser Ile Ile Pro Thr Asp Leu Ile Tyr Phe Ala Val
   225                 230                 235                 240
   ggt atc cac agc cct gag gta cgc ttc aac cgt cta tta cac ttt gcc       768
   Gly Ile His Ser Pro Glu Val Arg Phe Asn Arg Leu Leu His Phe Ala
                   245                 250                 255
   cgt atg ttt gag ttc ttt gac cgc act gag aca cgc acc agc tac ccc        816
   Arg Met Phe Glu Phe Phe Asp Arg Thr Glu Thr Arg Thr Ser Tyr Pro
               260                 265                 270
   aac atc ttc cga atc agc aat ctg gtc ctt tac atc ttg gtc atc atc        864
   Asn Ile Phe Arg Ile Ser Asn Leu Val Leu Tyr Ile Leu Val Ile Ile
           275                 280                 285
   cac tgg aat gct tgt att tat tat gtt att tct aag tcc att ggc ttt        912
   His Trp Asn Ala Cys Ile Tyr Tyr Val Ile Ser Lys Ser Ile Gly Phe
       290                 295                 300
   gga gtt gac acc tgg gtt tac ccc aac att act gac cct gaa tat ggc        960
   Gly Val Asp Thr Trp Val Tyr Pro Asn Ile Thr Asp Pro Glu Tyr Gly
   305                 310                 315                 320
   tac ctg gct aga gag tac att tac tgt ctt tac tgg tcc aca ctg acc       1008
   Tyr Leu Ala Arg Glu Tyr Ile Tyr Cys Leu Tyr Trp Ser Thr Leu Thr
                   325                 330                 335
   ctc acc acc att gga gag aca cca ccc cct gta aag gat gag gag tac       1056
   Leu Thr Thr Ile Gly Glu Thr Pro Pro Pro Val Lys Asp Glu Glu Tyr
               340                 345                 350
   cta ttt gtc atc ttt gac ttc ttg att ggt gtc ctc atc ttt gcc act       1104
   Leu Phe Val Ile Phe Asp Phe Leu Ile Gly Val Leu Ile Phe Ala Thr
           355                 360                 365
   att gtg gga aat gtg ggc tcc atg atc tcc aac atg aat gcc aca cga       1152
   Ile Val Gly Asn Val Gly Ser Met Ile Ser Asn Met Asn Ala Thr Arg
       370                 375                 380
   gca gag ttc cag gcc aag att gat gct gtc aaa cac tac atg cag ttc       1200
   Ala Glu Phe Gln Ala Lys Ile Asp Ala Val Lys His Tyr Met Gln Phe
   385                 390                 395                 400
   cga aag gtc agc aaa gac atg gaa gcc aag gtc atc aaa tgg ttt gac       1248
   Arg Lys Val Ser Lys Asp Met Glu Ala Lys Val Ile Lys Trp Phe Asp
                   405                 410                 415
   tac ttg tgg acc aat aag aag aca gta gat gaa cga gaa gtc ctc aag       1296
   Tyr Leu Trp Thr Asn Lys Lys Thr Val Asp Glu Arg Glu Val Leu Lys
               420                 425                 430
   aac ctg cca gca aag ctc agg gct gag ata gcc att aat gtt cac ttg       1344
   Asn Leu Pro Ala Lys Leu Arg Ala Glu Ile Ala Ile Asn Val His Leu
           435                 440                 445
   tcc act ctg aag aaa gtg cgc ata ttc cag gat tgg gaa gct ggc cta       1392
   Ser Thr Leu Lys Lys Val Arg Ile Phe Gln Asp Trp Glu Ala Gly Leu
       450                 455                 460
   ctg gtg gaa ctg gta ctg aag ctt cgt cct cag gtc ttt agt cct gga       1440
   Leu Val Glu Leu Val Leu Lys Leu Arg Pro Gln Val Phe Ser Pro Gly
   465                 470                 475                 480
   gat tat att tgc cgt aag ggg gac att ggc aag gaa atg tac atc atc       1488
   Asp Tyr Ile Cys Arg Lys Gly Asp Ile Gly Lys Glu Met Tyr Ile Ile
                   485                 490                 495
   aag gag ggc aag ttg gca gtg gta gct gat gat ggc gtg act cag tat      1536
   Lys Glu Gly Lys Leu Ala Val Val Ala Asp Asp Gly Val Thr Gln Tyr
               500                 505                 510
   gcc ttg ctc tca gct ggg agc tgc ttt ggt gag att agt atc ctt aac      1584
   Ala Leu Leu Ser Ala Gly Ser Cys Phe Gly Glu Ile Ser Ile Leu Asn
           515                 520                 525
   att aag ggt agc aaa atg ggc aat cga cgt act gct aat atc cgt agc      1632
   Ile Lys Gly Ser Lys Met Gly Asn Arg Arg Thr Ala Asn Ile Arg Ser
       530                 535                 540
   ctg ggc tac tca gat ctc ttc tgc ttg tcc aag gac gat ctt atg gaa      1680
   Leu Gly Tyr Ser Asp Leu Phe Cys Leu Ser Lys Asp Asp Leu Met Glu
   545                 550                 555                 560
   gct gta act gag gct cct gat gcc aag aag gtc ctg gag gaa cgg ggt      1728
   Ala Val Thr Glu Ala Pro Asp Ala Lys Lys Val Leu Glu Glu Arg Gly
                   565                 570                 575
   agg gag atc ctg atg aag atg ggt cta ctg gat gag aat gaa gtg gca      1776
   Arg Glu Ile Leu Met Lys Met Gly Leu Leu Asp Glu Asn Glu Val Ala
               580                 585                 590
   gct agt atg gag gta gat gtt cag gag aag ctg gaa cag ttg gag aca      1824
   Ala Ser Met Glu Val Asp Val Gln Glu Lys Leu Glu Gln Leu Glu Thr
           595                 600                 605
   aac atg gat acc ttg tac act cgc ttt gcc cgc ctg ctg gct gag tac      1872
   Asn Met Asp Thr Leu Tyr Thr Arg Phe Ala Arg Leu Leu Ala Glu Tyr
       610                 615                 620
   act ggg gcc cag cag aag ctc aag caa cgc atc aca gtg cta gag acc      1920
   Thr Gly Ala Gln Gln Lys Leu Lys Gln Arg Ile Thr Val Leu Glu Thr
   625                 630                 635                 640
   aag atg aaa cag aac cat gag gat gat tat cta tca gat ggg ata aac      1968
   Lys Met Lys Gln Asn His Glu Asp Asp Tyr Leu Ser Asp Gly Ile Asn
                   645                 650                 655
   act cct gag cca act gct gct gaa taa                                  1995
   Thr Pro Glu Pro Thr Ala Ala Glu
               660
<210> 8
   <211> 664
   <212> PRT
   <213> Rattus norvegicus
<400> 8
   Met Met Thr Glu Lys Ser Asn Gly Val Lys Ser Ser Pro Ala Asn Asn
   1               5                   10                  15
   His Asn His His Pro Pro Pro Ser Ile Lys Ala Asn Gly Lys Asp Asp
               20                  25                  30
   His Arg Ala Gly Ser Arg Pro Gln Ser Val Ala Ala Asp Asp Asp Thr
           35                  40                  45
   Ser Pro Glu Leu Gln Arg Leu Ala Glu Met Asp Thr Pro Arg Arg Gly
   50 55 60
   Arg Gly Gly Phe Gln Arg Ile Val Arg Leu Val Gly Val Ile Arg Asp
   65 70 75 80
   Trp Ala Asn Lys Asn Phe Arg Glu Glu Glu Pro Arg Pro Asp Ser Phe
   85 90 95
   Leu Glu Arg Phe Arg Gly Pro Glu Leu Gln Thr Val Thr Thr His Gln
   100 105 110
   Gly Asp Asp Lys Gly Gly Lys Asp Gly Glu Gly Lys Gly Thr Lys Lys
   115 120 125
   Lys Phe Glu Leu Phe Val Leu Asp Pro Ala Gly Asp Trp Tyr Tyr Arg
   130 135 140
   Trp Leu Phe Val Ile Ala Met Pro Val Leu Tyr Asn Trp Cys Leu Leu
   145 150 155 160
   Val Ala Arg Ala Cys Phe Ser Asp Leu Gln Arg Asn Tyr Phe Val Val
   165 170 175
   Trp Leu Val Leu Asp Tyr Phe Ser Asp Thr Val Tyr Ile Ala Asp Leu
   180 185 190
   Ile Ile Arg Leu Arg Thr Gly Phe Leu Glu Gln Gly Leu Leu Val Lys
   195 200 205
   Asp Pro Lys Lys Leu Arg Asp Asn Tyr Ile His Thr Leu Gln Phe Lys
   210 215 220
   Leu Asp Val Ala Ser Ile Ile Pro Thr Asp Leu Ile Tyr Phe Ala Val
   225 230 235 240
   Gly Ile His Ser Pro Glu Val Arg Phe Asn Arg Leu Leu His Phe Ala
   245 250 255
   Arg Met Phe Glu Phe Phe Asp Arg Thr Glu Thr Arg Thr Ser Tyr Pro
   260 265 270
   Asn Ile Phe Arg Ile Ser Asn Leu Val Leu Tyr Ile Leu Val Ile Ile
   275 280 285
   His Trp Asn Ala Cys Ile Tyr Tyr Val Ile Ser Lys Ser Ile Gly Phe
   290 295 300
   Gly Val Asp Thr Trp Val Tyr Pro Asn Ile Thr Asp Pro Glu Tyr Gly
   305 310 315 320
   Tyr Leu Ala Arg Glu Tyr Ile Tyr Cys Leu Tyr Trp Ser Thr Leu Thr
   325 330 335
   Leu Thr Thr Ile Gly Glu Thr Pro Pro Pro Val Lys Asp Glu Glu Tyr
   340 345 350
   Leu Phe Val Ile Phe Asp Phe Leu Ile Gly Val Leu Ile Phe Ala Thr
   355 360 365
   Ile Val Gly Asn Val Gly Ser Met Ile Ser Asn Met Asn Ala Thr Arg
   370 375 380
   Ala Glu Phe Gln Ala Lys Ile Asp Ala Val Lys His Tyr Met Gln Phe
   385                 390                 395                 400
   Arg Lys Val Ser Lys Asp Met Glu Ala Lys Val Ile Lys Trp Phe Asp
                   405                 410                 415
   Tyr Leu Trp Thr Asn Lys Lys Thr Val Asp Glu Arg Glu Val Leu Lys
               420                 425                 430
   Asn Leu Pro Ala Lys Leu Arg Ala Glu Ile Ala Ile Asn Val His Leu
           435                 440                 445
   Ser Thr Leu Lys Lys Val Arg Ile Phe Gln Asp Trp Glu Ala Gly Leu
       450                 455                 460
   Leu Val Glu Leu Val Leu Lys Leu Arg Pro Gln Val Phe Ser Pro Gly
   465                 470                 475                 480
   Asp Tyr Ile Cys Arg Lys Gly Asp Ile Gly Lys Glu Met Tyr Ile Ile
                   485                 490                 495
   Lys Glu Gly Lys Leu Ala Val Val Ala Asp Asp Gly Val Thr Gln Tyr
               500                 505                 510
   Ala Leu Leu Ser Ala Gly Ser Cys Phe Gly Glu Ile Ser Ile Leu Asn
           515                 520                 525
   Ile Lys Gly Ser Lys Met Gly Asn Arg Arg Thr Ala Asn Ile Arg Ser
       530                 535                 540
   Leu Gly Tyr Ser Asp Leu Phe Cys Leu Ser Lys Asp Asp Leu Met Glu
   545                 550                 555                 560
   Ala Val Thr Glu Ala Pro Asp Ala Lys Lys Val Leu Glu Glu Arg Gly
                   565                 570                 575
   Arg Glu Ile Leu Met Lys Met Gly Leu Leu Asp Glu Asn Glu Val Ala
               580                 585                 590
   Ala Ser Met Glu Val Asp Val Gln Glu Lys Leu Glu Gln Leu Glu Thr
           595                 600                 605
   Asn Met Asp Thr Leu Tyr Thr Arg Phe Ala Arg Leu Leu Ala Glu Tyr
       610                 615                 620
   Thr Gly Ala Gln Gln Lys Leu Lys Gln Arg Ile Thr Val Leu Glu Thr
   625                 630                 635                 640
   Lys Met Lys Gln Asn His Glu Asp Asp Tyr Leu Ser Asp Gly Ile Asn
                   645                 650                 655
   Thr Pro Glu Pro Thr Ala Ala Glu
               660
<210> 9
   <211> 690
   <212> PRT
   <213> Homo sapiens
<220>
   <221> MISC_FEATURE
   <222> (1) .. (690)
   <223> CNGA1
<400> 9
   Met Lys Leu Ser Met Lys Asn Asn Ile Ile Asn Thr Gln Gln Ser Phe
   1               5                   10                  15
   Val Thr Met Pro Asn Val Ile Val Pro Asp Ile Glu Lys Glu Ile Arg
               20                  25                  30
   Arg Met Glu Asn Gly Ala Cys Ser Ser Phe Ser Glu Asp Asp Asp Ser
           35                  40                  45
   Ala Tyr Thr Ser Glu Glu Ser Glu Asn Glu Asn Pro His Ala Arg Gly
       50                  55                  60
   Ser Phe Ser Tyr Lys Ser Leu Arg Lys Gly Gly Pro Ser Gln Arg Glu
   65                  70                  75                  80
   Gln Tyr Leu Pro Gly Ala Ile Ala Ile Phe Asn Val Asn Asn Ser Ser
                   85                  90                  95
   Asn Lys Asp Gln Glu Pro Glu Glu Lys Lys Lys Lys Lys Lys Glu Lys
               100                 105                 110
   Lys Ser Lys Ser Asp Asp Lys Asn Glu Asn Lys Asn Asp Pro Glu Lys
           115                 120                 125
   Lys Lys Lys Lys Lys Asp Lys Glu Lys Lys Lys Lys Glu Glu Lys Ser
       130                 135                 140
   Lys Asp Lys Lys Glu His His Lys Lys Glu Val Val Val Ile Asp Pro
   145                 150                 155                 160
   Ser Gly Asn Thr Tyr Tyr Asn Trp Leu Phe Cys Ile Thr Leu Pro Val
                   165                 170                 175
   Met Tyr Asn Trp Thr Met Val Ile Ala Arg Ala Cys Phe Asp Glu Leu
               180                 185                 190
   Gln Ser Asp Tyr Leu Glu Tyr Trp Leu Ile Leu Asp Tyr Val Ser Asp
           195                 200                 205
   Ile Val Tyr Leu Ile Asp Met Phe Val Arg Thr Arg Thr Gly Tyr Leu
       210                 215                 220
   Glu Gln Gly Leu Leu Val Lys Glu Glu Leu Lys Leu Ile Asn Lys Tyr
   225                 230                 235                 240
   Lys Ser Asn Leu Gln Phe Lys Leu Asp Val Leu Ser Leu Ile Pro Thr
                   245                 250                 255
   Asp Leu Leu Tyr Phe Lys Leu Gly Trp Asn Tyr Pro Glu Ile Arg Leu
               260                 265                 270
   Asn Arg Leu Leu Arg Phe Ser Arg Met Phe Glu Phe Phe Gln Arg Thr
           275                 280                 285
   Glu Thr Arg Thr Asn Tyr Pro Asn Ile Phe Arg Ile Ser Asn Leu Val
   290 295 300
   Met Tyr Ile Val Ile Ile Ile His Trp Asn Ala Cys Val Phe Tyr Ser
   305 310 315 320
   Ile Ser Lys Ala Ile Gly Phe Gly Asn Asp Thr Trp Val Tyr Pro Asp
   325 330 335
   Ile Asn Asp Pro Glu Phe Gly Arg Leu Ala Arg Lys Tyr Val Tyr Ser
   340 345 350
   Leu Tyr Trp Ser Thr Leu Thr Leu Thr Thr Ile Gly Glu Thr Pro Pro
   355 360 365
   Pro Val Arg Asp Ser Glu Tyr Val Phe Val Val Val Asp Phe Leu Ile
   370 375 380
   Gly Val Leu Ile Phe Ala Thr Ile Val Gly Asn Ile Gly Ser Met Ile
   385 390 395 400
   Ser Asn Met Asn Ala Ala Arg Ala Glu Phe Gln Ala Arg Ile Asp Ala
   405 410 415
   Ile Lys Gln Tyr Met His Phe Arg Asn Val Ser Lys Asp Met Glu Lys
   420 425 430
   Arg Val Ile Lys Trp Phe Asp Tyr Leu Trp Thr Asn Lys Lys Thr Val
   435 440 445
   Asp Glu Lys Glu Val Leu Lys Tyr Leu Pro Asp Lys Leu Arg Ala Glu
   450 455 460
   Ile Ala Ile Asn Val His Leu Asp Thr Leu Lys Lys Val Arg Ile Phe
   465 470 475 480
   Ala Asp Cys Glu Ala Gly Leu Leu Val Glu Leu Val Leu Lys Leu Gln
   485 490 495
   Pro Gln Val Tyr Ser Pro Gly Asp Tyr Ile Cys Lys Lys Gly Asp Ile
   500 505 510
   Gly Arg Glu Met Tyr Ile Ile Lys Glu Gly Lys Leu Ala Val Val Ala
   515 520 525
   Asp Asp Gly Val Thr Gln Phe Val Val Leu Ser Asp Gly Ser Thr Phe
   530 535 540
   Gly Glu Ile Ser Ile Leu Asn Ile Lys Gly Ser Lys Ala Gly Asn Arg
   545 550 555 560
   Arg Thr Ala Asn Ile Lys Ser Ile Gly Tyr Ser Asp Leu Phe Cys Leu
   565 570 575
   Ser Lys Asp Asp Leu Met Glu Ala Leu Thr Glu Tyr Pro Asp Ala Lys
   580 585 590
   Thr Met Leu Glu Glu Lys Gly Lys Gln Ile Leu Met Lys Asp Gly Leu
   595 600 605
   Leu Asp Leu Asn Ile Ala Asn Ala Gly Ser Asp Pro Lys Asp Leu Glu
   610 615 620
   Glu Lys Val Thr Arg Met Glu Gly Ser Val Asp Leu Leu Gln Thr Arg
   625                 630                 635                 640
   Phe Ala Arg Ile Leu Ala Glu Tyr Glu Ser Met Gln Gln Lys Leu Lys
                   645                 650                 655
   Gln Arg Leu Thr Lys Val Glu Lys Phe Leu Lys Pro Leu Ile Asp Thr
               660                 665                 670
   Glu Phe Ser Ser Ile Glu Gly Pro Trp Ser Glu Ser Gly Pro Ile Asp
           675                 680                 685
   Ser Thr
       690
<210> 10
   <211> 664
   <212> PRT
   <213> Homo sapiens
<220>
   <221> MISC_FEATURE
   <222> (1) .. (664)
   <223> CNGA2
<400> 10
   Met Thr Glu Lys Thr Asn Gly Val Lys Ser Ser Pro Ala Asn Asn His
   1 5 10 15
   Asn His His Ala Pro Pro Ala Ile Lys Ala Asn Gly Lys Asp Asp His
   20 25 30
   Arg Thr Ser Ser Arg Pro His Ser Ala Ala Asp Asp Asp Thr Ser Ser
   35 40 45
   Glu Leu Gln Arg Leu Ala Asp Val Asp Ala Pro Gln Gln Gly Arg Ser
   50 55 60
   Gly Phe Arg Arg Ile Val Arg Leu Val Gly Ile Ile Arg Glu Trp Ala
   65 70 75 80
   Asn Lys Asn Phe Arg Glu Glu Glu Pro Arg Pro Asp Ser Phe Leu Glu
   85 90 95
   Arg Phe Arg Gly Pro Glu Leu Gln Thr Val Thr Thr Gln Glu Gly Asp
   100 105 110
   Gly Lys Gly Asp Lys Asp Gly Glu Asp Lys Gly Thr Lys Lys Lys Phe
   115 120 125
   Glu Leu Phe Val Leu Asp Pro Ala Gly Asp Trp Tyr Tyr Cys Trp Leu
   130 135 140
   Phe Val Ile Ala Met Pro Val Leu Tyr Asn Trp Cys Leu Leu Val Ala
   145 150 155 160
   Arg Ala Cys Phe Ser Asp Leu Gln Lys Gly Tyr Tyr Leu Val Trp Leu
   165 170 175
   Val Leu Asp Tyr Val Ser Asp Val Val Tyr Ile Ala Asp Leu Phe Ile
               180                 185                 190
   Arg Leu Arg Thr Gly Phe Leu Glu Gln Gly Leu Leu Val Lys Asp Thr
           195                 200                 205
   Lys Lys Leu Arg Asp Asn Tyr Ile His Thr Leu Gln Phe Lys Leu Asp
       210                 215                 220
   Val Ala Ser Ile Ile Pro Thr Asp Leu Ile Tyr Phe Ala Val Asp Ile
   225                 230                 235                 240
   His Ser Pro Glu Val Arg Phe Asn Arg Leu Leu His Phe Ala Arg Met
                   245                 250                 255
   Phe Glu Phe Phe Asp Arg Thr Glu Thr Arg Thr Asn Tyr Pro Asn Ile
               260                 265                 270
   Phe Arg Ile Ser Asn Leu Val Leu Tyr Ile Leu Val Ile Ile His Trp
           275                 280                 285
   Asn Ala Cys Ile Tyr Tyr Ala Ile Ser Lys Ser Ile Gly Phe Gly Val
       290                 295                 300
   Asp Thr Trp Val Tyr Pro Asn Ile Thr Asp Pro Glu Tyr Gly Tyr Leu
   305                 310                 315                 320
   Ala Arg Glu Tyr Ile Tyr Cys Leu Tyr Trp Ser Thr Leu Thr Leu Thr
                   325                 330                 335
   Thr Ile Gly Glu Thr Pro Pro Pro Val Lys Asp Glu Glu Tyr Leu Phe
               340                 345                 350
   Val Ile Phe Asp Phe Leu Ile Gly Val Leu Ile Phe Ala Thr Ile Val
           355                 360                 365
   Gly Asn Val Gly Ser Met Ile Ser Asn Met Asn Ala Thr Arg Ala Glu
       370                 375                 380
   Phe Gln Ala Lys Ile Asp Ala Val Lys His Tyr Met Gln Phe Arg Lys
   385                 390                 395                 400
   Val Ser Lys Gly Met Glu Ala Lys Val Ile Arg Trp Phe Asp Tyr Leu
                   405                 410                 415
   Trp Thr Asn Lys Lys Thr Val Asp Glu Arg Glu Ile Leu Lys Asn Leu
               420                 425                 430
   Pro Ala Lys Leu Arg Ala Glu Ile Ala Ile Asn Val His Leu Ser Thr
           435                 440                 445
   Leu Lys Lys Val Arg Ile Phe His Asp Cys Glu Ala Gly Leu Leu Val
       450                 455                 460
   Glu Leu Val Leu Lys Leu Arg Pro Gln Val Phe Ser Pro Gly Asp Tyr
   465                 470                 475                 480
   Ile Cys Arg Lys Gly Asp Ile Gly Lys Glu Met Tyr Ile Ile Lys Glu
                   485                 490                 495
   Gly Lys Leu Ala Val Val Ala Asp Asp Gly Val Thr Gln Tyr Ala Leu
   500 505 510
   Leu Ser Ala Gly Ser Cys Phe Gly Glu Ile Ser Ile Leu Asn Ile Lys
   515 520 525
   Gly Ser Lys Met Gly Asn Arg Arg Thr Ala Asn Ile Arg Ser Leu Gly
   530 535 540
   Tyr Ser Asp Leu Phe Cys Leu Ser Lys Asp Asp Leu Met Glu Ala Val
   545 550 555 560
   Thr Glu Tyr Pro Asp Ala Lys Lys Val Leu Glu Glu Arg Gly Arg Glu
   565 570 575
   Ile Leu Met Lys Glu Gly Leu Leu Asp Glu Asn Glu Val Ala Thr Ser
   580 585 590
   Met Glu Val Asp Val Gln Glu Lys Leu Gly Gln Leu Glu Thr Asn Met
   595 600 605
   Glu Thr Leu Tyr Thr Arg Phe Gly Arg Leu Leu Ala Glu Tyr Thr Gly
   610 615 620
   Ala Gln Gln Lys Leu Lys Gln Arg Ile Thr Val Leu Glu Thr Lys Met
   625 630 635 640
   Lys Gln Asn Asn Glu Asp Asp Tyr Leu Ser Asp Gly Met Asn Ser Pro
   645 650 655
   Glu Leu Ala Ala Ala Asp Glu Pro
   660
<210> 11
   <211> 694
   <212> PRT
   <213> Homo sapiens
<220>
   <221> MISC_FEATURE
   <222> (1) .. (694)
   <223> CNGA3
<400> 11
<210> 12
   <211> 1245
   <212> PRT
   <213> Homo sapiens
<220>
   <221> MISC_FEATURE
   <222> (1) .. (1245)
   <223> CNGB1
<210> 13
   <211> 575
   <212> PRT
   <213> Homo sapiens
<220>
   <221> MISC_FEATURE
   <222> (1) .. (575)
   <223> CNGB2
<400> 13
   Met Ser Gln Asp Thr Lys Val Lys Thr Thr Glu Ser Ser Pro Pro Ala
   1               5                   10                  15
   Pro Ser Lys Ala Arg Lys Leu Leu Pro Val Leu Asp Pro Ser Gly Asp
               20                  25                  30
   Tyr Tyr Tyr Trp Trp Leu Asn Thr Met Val Phe Pro Val Met Tyr Asn
           35                  40                  45
   Leu Ile Ile Leu Val Cys Arg Ala Cys Phe Pro Asp Leu Gln His Gly
       50                  55                  60
   Tyr Leu Val Ala Trp Leu Val Leu Asp Tyr Thr Ser Asp Leu Leu Tyr
   65                  70                  75                  80
   Leu Leu Asp Met Val Val Arg Phe His Thr Gly Phe Leu Glu Gln Gly
                   85                  90                  95
   Ile Leu Val Val Asp Lys Gly Arg Ile Ser Ser Arg Tyr Val Arg Thr
               100                 105                 110
   Trp Ser Phe Phe Leu Asp Leu Ala Ser Leu Met Pro Thr Asp Val Val
           115                 120                 125
   Tyr Val Arg Leu Gly Pro His Thr Pro Thr Leu Arg Leu Asn Arg Phe
       130                 135                 140
   Leu Arg Ala Pro Arg Leu Phe Glu Ala Phe Asp Arg Thr Glu Thr Arg
   145                 150                 155                 160
   Thr Ala Tyr Pro Asn Ala Phe Arg Ile Ala Lys Leu Met Leu Tyr Ile
                   165                 170                 175
   Phe Val Val Ile His Trp Asn Ser Cys Leu Tyr Phe Ala Leu Ser Arg
               180                 185                 190
   Tyr Leu Gly Phe Gly Arg Asp Ala Trp Val Tyr Pro Asp Pro Ala Gln
           195                 200                 205
   Pro Gly Phe Glu Arg Leu Arg Arg Gln Tyr Leu Tyr Ser Phe Tyr Phe
       210                 215                 220
   Ser Thr Leu Ile Leu Thr Thr Val Gly Asp Thr Pro Pro Pro Ala Arg
   225                 230                 235                 240
   Glu Glu Glu Tyr Leu Phe Met Val Gly Asp Phe Leu Leu Ala Val Met
                   245                 250                 255
   Gly Phe Ala Thr Ile Met Gly Ser Met Ser Ser Val Ile Tyr Asn Met
               260                 265                 270
   Asn Thr Ala Asp Ala Ala Phe Tyr Pro Asp His Ala Leu Val Lys Lys
           275                 280                 285
   Tyr Met Lys Leu Gln His Val Asn Arg Lys Leu Glu Arg Arg Val Ile
       290                 295                 300
   Asp Trp Tyr Gln His Leu Gln Ile Asn Lys Lys Met Thr Asn Glu Val
   305                 310                 315                 320
   Ala Ile Leu Gln His Leu Pro Glu Arg Leu Arg Ala Glu Val Ala Val
                   325                 330                 335
   Ser Val His Leu Ser Thr Leu Ser Arg Val Gln Ile Phe Gln Asn Cys
               340                 345                 350
   Glu Ala Ser Leu Leu Glu Glu Leu Val Leu Lys Leu Gln Pro Gln Thr
           355                 360                 365
   Tyr Ser Pro Gly Glu Tyr Val Cys Arg Lys Gly Asp Ile Gly Gln Glu
       370                 375                 380
   Met Tyr Ile Ile Arg Glu Gly Gln Leu Ala Val Val Ala Asp Asp Gly
   385                 390                 395                 400
   Ile Thr Gln Tyr Ala Val Leu Gly Ala Gly Leu Tyr Phe Gly Glu Ile
                   405                 410                 415
   Ser Ile Ile Asn Ile Lys Gly Asn Met Ser Gly Asn Arg Arg Thr Ala
               420                 425                 430
   Asn Ile Lys Ser Leu Gly Tyr Ser Asp Leu Phe Cys Leu Ser Lys Glu
           435                 440                 445
   Asp Leu Arg Glu Val Leu Ser Glu Tyr Pro Gln Ala Gln Thr Ile Met
       450                 455                 460
   Glu Glu Lys Gly Arg Glu Ile Leu Leu Lys Met Asn Lys Leu Asp Val
   465                 470                 475                 480
   Asn Ala Glu Ala Ala Glu Ile Ala Leu Gln Glu Ala Thr Glu Ser Arg
                   485                 490                 495
   Leu Arg Gly Leu Asp Gln Gln Leu Asp Asp Leu Gln Thr Lys Phe Ala
               500                 505                 510
   Arg Leu Leu Ala Glu Leu Glu Ser Ser Ala Leu Lys Ile Ala Tyr Arg
           515                 520                 525
   Ile Glu Arg Leu Glu Trp Gln Thr Arg Glu Trp Pro Met Pro Glu Asp
       530                 535                 540
   Leu Ala Glu Ala Asp Asp Glu Gly Glu Pro Glu Glu Gly Thr Ser Lys
   545                 550                 555                 560
   Asp Glu Glu Gly Arg Ala Ser Gln Glu Gly Pro Pro Gly Pro Glu
                   565                 570                 575
<210> 14
   <211> 615
   <212> PRT
   <213> Homo sapiens
<220>
   <221> MISC_FEATURE
   <222> (1)..(615)
   <223> CNGB3
<400> 14
   Met Pro Leu Thr Glu Tyr Leu Lys Arg Ile Lys Leu Pro Asn Ser Ile
   1 5 10 15
   Asp Ser Tyr Thr Asp Arg Leu Tyr Leu Leu Trp Leu Leu Leu Val Thr
   20 25 30
   Leu Ala Tyr Asn Trp Asn Cys Trp Phe Ile Pro Leu Arg Leu Val Phe
   35 40 45
   Pro Tyr Gln Thr Ala Asp Asn Ile His Tyr Trp Leu Ile Ala Asp Ile
   50 55 60
   Ile Cys Asp Ile Ile Tyr Leu Tyr Asp Met Leu Phe Ile Gln Pro Arg
   65 70 75 80
   Leu Gln Phe Val Arg Gly Gly Asp Ile Ile Val Asp Ser Asn Glu Leu
   85 90 95
   Arg Lys His Tyr Arg Thr Ser Thr Lys Phe Gln Leu Asp Val Ala Ser
   100 105 110
   Ile Ile Pro Phe Asp Ile Cys Tyr Leu Phe Phe Gly Phe Asn Pro Met
   115 120 125
   Phe Arg Ala Asn Arg Met Leu Lys Tyr Thr Ser Phe Phe Glu Phe Asn
   130 135 140
   His His Leu Glu Ser Ile Met Asp Lys Ala Tyr Ile Tyr Arg Val Ile
   145 150 155 160
   Arg Thr Thr Gly Tyr Leu Leu Phe Ile Leu His Ile Asn Ala Cys Val
   165 170 175
   Tyr Tyr Trp Ala Ser Asn Tyr Glu Gly Ile Gly Thr Thr Arg Trp Val
   180 185 190
   Tyr Asp Gly Glu Gly Asn Glu Tyr Leu Arg Cys Tyr Tyr Trp Ala Val
   195 200 205
   Arg Thr Leu Ile Thr Ile Gly Gly Leu Pro Glu Pro Gln Thr Leu Phe
   210 215 220
   Glu Ile Val Phe Gln Leu Leu Asn Phe Phe Ser Gly Val Phe Val Phe
   225 230 235 240
   Ser Ser Leu Ile Gly Gln Met Arg Asp Val Ile Gly Ala Ala Thr Ala
   245 250 255
   Asn Gln Asn Tyr Phe Arg Ala Cys Met Asp Asp Thr Ile Ala Tyr Met
   260 265 270
   Asn Asn Tyr Ser Ile Pro Lys Leu Val Gln Lys Arg Val Arg Thr Trp
   275 280 285
   Tyr Glu Tyr Thr Trp Asp Ser Gln Arg Met Leu Asp Glu Ser Asp Leu
   290 295 300
   Leu Lys Thr Leu Pro Thr Thr Val Gln Leu Ala Leu Ala Ile Asp Val
   305 310 315 320
   Asn Phe Ser Ile Ile Ser Lys Val Asp Leu Phe Lys Gly Cys Asp Thr
   325 330 335
   Gln Met Ile Tyr Asp Met Leu Leu Arg Leu Lys Ser Val Leu Tyr Leu
   340 345 350
   Pro Gly Asp Phe Val Cys Lys Lys Gly Glu Ile Gly Lys Glu Met Tyr
   355 360 365
   Ile Ile Lys His Gly Glu Val Gln Val Leu Gly Gly Pro Asp Gly Thr
   370 375 380
   Lys Val Leu Val Thr Leu Lys Ala Gly Ser Val Phe Gly Glu Ile Ser
   385 390 395 400
   Leu Leu Ala Ala Gly Gly Gly Asn Arg Arg Thr Ala Asn Val Val Ala
   405 410 415
   His Gly Phe Ala Asn Leu Leu Thr Leu Asp Lys Lys Thr Leu Gln Glu
   420 425 430
   Ile Leu Val His Tyr Pro Asp Ser Glu Arg Ile Leu Met Lys Lys Ala
   435 440 445
   Arg Val Leu Leu Lys Gln Lys Ala Lys Thr Ala Glu Ala Thr Pro Pro
   450 455 460
   Arg Lys Asp Leu Ala Leu Leu Phe Pro Pro Lys Glu Glu Thr Pro Lys
   465 470 475 480
   Leu Phe Lys Thr Leu Leu Gly Gly Thr Gly Lys Ala Ser Leu Ala Arg
   485 490 495
   Leu Leu Lys Leu Lys Arg Glu Gln Ala Ala Gln Lys Lys Glu Asn Ser
   500 505 510
   Glu Gly Gly Glu Glu Glu Gly Lys Glu Asn Glu Asp Lys Gln Lys Glu
   515 520 525
   Asn Glu Asp Lys Gln Lys Glu Asn Glu Asp Lys Gly Lys Glu Asn Glu
   530 535 540
   Asp Lys Asp Lys Gly Arg Glu Pro Glu Glu Lys Pro Leu Asp Arg Pro
   545 550 555 560
   Glu Cys Thr Ala Ser Pro Ile Ala Val Glu Glu Glu Pro His Ser Val
   565 570 575
   Arg Arg Thr Val Leu Pro Arg Gly Thr Ser Arg Gln Ser Leu Ile Ile
   580 585 590
   Ser Met Ala Pro Ser Ala Glu Gly Gly Glu Glu Val Leu Thr Ile Glu
   595 600 605
   Val Lys Glu Lys Ala Lys Gln
   610 615
<210>
   <211> 664
   <212> PRT
   <213> Mus musculus
<220>
   <221> MISC_FEATURE
   <222> (1) .. (664)
   <223> CNGA2
<400> 15
<210> 16
   <211> 732
   <212> PRT
   <213> Oryctolagus cuniculus
<220>
   <221> MISC_FEATURE
   <222> (1) .. (732)
   <223> CNGA2
<400> 16
   Met Ser Ser Trp Arg Ser Cys Ala Arg Ala Pro Leu Ser Gly Ser Ala
   1               5                   10                  15
   Trp Arg Arg Ser Ala Ala Thr Arg Arg Ser Arg Arg Cys Leu Lys Thr
               20                  25                  30
   Lys Arg Lys Arg Trp Ser Ser Gly Lys Gly Thr Pro Met Gln Ser Thr
           35                  40                  45
   Gln Cys Glu Thr Arg Arg Arg Ala Gln Thr Pro Cys Glu Ser Thr Gly
       50                  55                  60
   His Thr Trp Arg Met Thr Glu Lys Ser Asn Gly Val Lys Ser Ser Pro
   65                  70                  75                  80
   Ala Asn Asn His Asn Asn His Val Pro Ala Thr Ile Lys Ala Asn Gly
                   85                  90                  95
   Lys Asp Glu Ser Arg Thr Arg Ser Arg Pro Gln Ser Ala Ala Asp Asp
               100                 105                 110
   Asp Thr Ser Ser Glu Leu Gln Arg Leu Ala Glu Met Asp Ala Pro Gln
           115                 120                 125
   Gln Arg Arg Gly Gly Phe Arg Arg Ile Val Arg Leu Val Gly Val Ile
       130                 135                 140
   Arg Gln Trp Ala Asn Arg Asn Phe Arg Glu Glu Glu Ala Arg Pro Asp
   145                 150                 155                 160
   Ser Phe Leu Glu Arg Phe Arg Gly Pro Glu Leu Gln Thr Val Thr Thr
   165 170 175
   Gln Gln Gly Asp Gly Lys Gly Asp Lys Asp Gly Asp Gly Lys Gly Thr
   180 185 190
   Lys Lys Lys Phe Glu Leu Phe Val Leu Asp Pro Ala Gly Asp Trp Tyr
   195 200 205
   Tyr Arg Trp Leu Phe Val Ile Ala Met Pro Val Leu Tyr Asn Trp Cys
   210 215 220
   Leu Leu Val Ala Arg Ala Cys Phe Ser Asp Leu Gln Arg Gly Tyr Phe
   225 230 235 240
   Leu Val Trp Leu Val Leu Asp Tyr Phe Ser Asp Val Val Tyr Ile Ala
   245 250 255
   Asp Leu Phe Ile Arg Leu Arg Thr Gly Phe Leu Glu Gln Gly Leu Leu
   260 265 270
   Val Lys Asp Pro Lys Lys Leu Arg Asp Asn Tyr Ile His Thr Leu Gln
   275 280 285
   Phe Lys Leu Asp Val Ala Ser Ile Ile Pro Thr Asp Leu Ile Tyr Phe
   290 295 300
   Ala Val Gly Ile His Asn Pro Glu Leu Arg Phe Asn Arg Leu Leu His
   305 310 315 320
   Phe Ala Arg Met Phe Glu Phe Phe Asp Arg Thr Glu Thr Arg Thr Ser
   325 330 335
   Tyr Pro Asn Ile Phe Arg Ile Ser Asn Leu Val Leu Tyr Ile Leu Val
   340 345 350
   Ile Ile His Trp Asn Ala Cys Ile Tyr Tyr Ala Ile Ser Lys Ser Ile
   355 360 365
   Gly Phe Gly Val Asp Thr Trp Val Tyr Pro Asn Ile Thr Asp Pro Glu
   370 375 380
   Tyr Gly Tyr Leu Ala Arg Glu Tyr Ile Tyr Cys Leu Tyr Trp Ser Thr
   385 390 395 400
   Leu Thr Leu Thr Thr Ile Gly Glu Thr Pro Pro Pro Val Lys Asp Glu
   405 410 415
   Glu Tyr Leu Phe Val Ile Phe Asp Phe Leu Ile Gly Val Leu Ile Phe
   420 425 430
   Ala Thr Ile Val Gly Asn Val Gly Ser Met Ile Ser Asn Met Asn Ala
   435 440 445
   Thr Arg Ala Glu Phe Gln Ala Lys Ile Asp Ala Val Lys His Tyr Met
   450 455 460
   Gln Phe Arg Lys Val Ser Lys Glu Met Glu Ala Lys Val Ile Lys Trp
   465 470 475 480
   Phe Asp Tyr Leu Trp Thr Asn Lys Lys Thr Val Asp Glu Arg Glu Val
   485 490 495
   Leu Lys Asn Leu Pro Ala Lys Leu Arg Ala Glu Ile Ala Ile Asn Val
   500 505 510
   His Leu Ser Thr Leu Lys Lys Val Arg Ile Phe Gln Asp Cys Glu Ala
   515 520 525
   Gly Leu Leu Val Glu Leu Val Leu Lys Leu Arg Pro Gln Val Phe Ser
   530 535 540
   Pro Gly Asp Tyr Ile Cys Arg Lys Gly Asp Ile Gly Lys Glu Met Tyr
   545 550 555 560
   Ile Ile Lys Glu Gly Lys Leu Ala Val Val Ala Asp Asp Gly Val Thr
   565 570 575
   Gln Tyr Ala Leu Leu Ser Ala Gly Ser Cys Phe Gly Glu Ile Ser Ile
   580 585 590
   Leu Asn Ile Lys Gly Ser Lys Met Gly Asn Arg Arg Thr Ala Asn Ile
   595 600 605
   Arg Ser Leu Gly Tyr Ser Asp Leu Phe Cys Leu Ser Lys Asp Asp Leu
   610 615 620
   Met Glu Ala Val Thr Glu Tyr Pro Asp Ala Lys Lys Val Leu Glu Glu
   625 630 635 640
   Arg Gly Arg Glu Ile Leu Met Lys Glu Gly Leu Leu Asp Glu Asn Glu
   645 650 655
   Val Ala Ala Ser Met Glu Val Asp Val Gln Glu Lys Leu Lys Gln Leu
   660 665 670
   Glu Thr Asn Met Glu Thr Leu Tyr Thr Arg Phe Gly Arg Leu Leu Ala
   675 680 685
   Glu Tyr Thr Gly Ala Gln Gln Lys Leu Lys Gln Arg Ile Thr Val Leu
   690 695 700
   Glu Val Lys Met Lys Gln Asn Thr Glu Asp Asp Tyr Leu Ser Asp Gly
   705 710 715 720
   Met Asn Ser Pro Glu Pro Ala Ala Ala Glu Gln Pro
   725 730
<210> 17
   <211> 663
   <212> PRT
   <213> Bos taurus
<220>
   <221> MISC_FEATURE
   <222> (1) .. (663)
   <223> CNGA2
<400> 17
   Met Thr Glu Lys Ala Asn Gly Val Lys Ser Ser Pro Ala Asn Asn His
   1               5                   10                  15
   Asn His His Ala Pro Pro Ala Ile Lys Ala Ser Gly Lys Asp Asp His
               20                  25                  30
   Arg Ala Ser Ser Arg Pro Gln Ser Ala Ala Ala Asp Asp Thr Ser Ser
           35                  40                  45
   Glu Leu Gln Gln Leu Ala Glu Met Asp Ala Pro Gln Gln Arg Arg Gly
       50                  55                  60
   Gly Phe Arg Arg Ile Ala Arg Leu Val Gly Val Leu Arg Glu Trp Ala
   65                  70                  75                  80
   Tyr Arg Asn Phe Arg Glu Glu Glu Pro Arg Pro Asp Ser Phe Leu Glu
                   85                  90                  95
   Arg Phe Arg Gly Pro Glu Leu His Thr Val Thr Thr Gln Gln Gly Asp
               100                 105                 110
   Gly Lys Gly Asp Lys Asp Gly Glu Gly Lys Gly Thr Lys Lys Lys Phe
           115                 120                 125
   Glu Leu Phe Val Leu Asp Pro Ala Gly Asp Trp Tyr Tyr Arg Trp Leu
       130                 135                 140
   Phe Leu Ile Ala Leu Pro Val Leu Tyr Asn Trp Cys Leu Leu Val Ala
   145                 150                 155                 160
   Arg Ala Cys Phe Ser Asp Leu Gln Lys Gly Tyr Tyr Ile Val Trp Leu
                   165                 170                 175
   Val Leu Asp Tyr Val Ser Asp Val Val Tyr Ile Ala Asp Leu Phe Ile
               180                 185                 190
   Arg Leu Arg Thr Gly Phe Leu Glu Gln Gly Leu Leu Val Lys Asp Thr
           195                 200                 205
   Lys Lys Leu Arg Asp Asn Tyr Ile His Thr Met Gln Phe Lys Leu Asp
       210                 215                 220
   Val Ala Ser Ile Ile Pro Thr Asp Leu Ile Tyr Phe Ala Val Gly Ile
   225                 230                 235                 240
   His Asn Pro Glu Val Arg Phe Asn Arg Leu Leu His Phe Ala Arg Met
                   245                 250                 255
   Phe Glu Phe Phe Asp Arg Thr Glu Thr Arg Thr Ser Tyr Pro Asn Ile
               260                 265                 270
   Phe Arg Ile Ser Asn Leu Ile Leu Tyr Ile Leu Ile Ile Ile His Trp
           275                 280                 285
   Asn Ala Cys Ile Tyr Tyr Ala Ile Ser Lys Ser Ile Gly Phe Gly Val
       290                 295                 300
   Asp Thr Trp Val Tyr Pro Asn Ile Thr Asp Pro Glu Tyr Gly Tyr Leu
   305                 310                 315                 320
   Ser Arg Glu Tyr Ile Tyr Cys Leu Tyr Trp Ser Thr Leu Thr Leu Thr
                   325                 330                 335
   Thr Ile Gly Glu Thr Pro Pro Pro Val Lys Asp Glu Glu Tyr Leu Phe
   340 345 350
   Val Ile Phe Asp Phe Leu Ile Gly Val Leu Ile Phe Ala Thr Ile Val
   355 360 365
   Gly Asn Val Gly Ser Met Ile Ser Asn Met Asn Ala Thr Arg Ala Glu
   370 375 380
   Phe Gln Ala Lys Ile Asp Ala Val Lys His Tyr Met Gln Phe Arg Lys
   385 390 395 400
   Val Ser Lys Glu Met Glu Ala Lys Val Ile Arg Trp Phe Asp Tyr Leu
   405 410 415
   Trp Thr Asn Lys Lys Ser Val Asp Glu Arg Glu Val Leu Lys Asn Leu
   420 425 430
   Pro Ala Lys Leu Arg Ala Glu Ile Ala Ile Asn Val His Leu Ser Thr
   435 440 445
   Leu Lys Lys Val Arg Ile Phe Gln Asp Cys Glu Ala Gly Leu Leu Val
   450 455 460
   Glu Leu Val Leu Lys Leu Arg Pro Gln Val Phe Ser Pro Gly Asp Tyr
   465 470 475 480
   Ile Cys Arg Lys Gly Asp Ile Gly Lys Glu Met Tyr Ile Ile Lys Glu
   485 490 495
   Gly Lys Leu Ala Val Val Ala Asp Asp Gly Val Thr Gln Tyr Ala Leu
   500 505 510
   Leu Ser Ala Gly Ser Cys Phe Gly Glu Ile Ser Ile Leu Asn Ile Lys
   515 520 525
   Gly Ser Lys Met Gly Asn Arg Arg Thr Ala Asn Ile Arg Ser Leu Gly
   530 535 540
   Tyr Ser Asp Leu Phe Cys Leu Ser Lys Asp Asp Leu Met Glu Ala Val
   545 550 555 560
   Thr Glu Tyr Pro Asp Ala Lys Arg Val Leu Glu Glu Arg Gly Arg Glu
   565 570 575
   Ile Leu Met Lys Glu Gly Leu Leu Asp Glu Asn Glu Val Ala Ala Ser
   580 585 590
   Met Glu Val Asp Val Gln Glu Lys Leu Glu Gln Leu Glu Thr Asn Met
   595 600 605
   Asp Thr Leu Tyr Thr Arg Phe Ala Arg Leu Leu Ala Glu Tyr Thr Gly
   610 615 620
   Ala Gln Gln Lys Leu Lys Gln Arg Ile Thr Val Leu Glu Thr Lys Met
   625 630 635 640
   Lys Gln Asn Asn Glu Asp Asp Ser Leu Ser Asp Gly Met Asn Ser Pro
   645 650 655
   Glu Pro Pro Ala Glu Lys Pro
   660
<210> 18
   <211> 664
   <212> PRT
   <213> Artificial Sequence
<220>
   <223> mammalian CNGA2 consensus sequence
<220>
   <221> MISC_FEATURE
   <222> (1) .. (664)
   <223> Xaa = any naturally occurring amino acid
<400> 18
   Met Met Thr Glu Lys Ser Asn Gly Val Lys Ser Ser Pro Ala Asn Asn
   1               5                   10                  15
   His Asn His His Ala Pro Pro Ala Ile Lys Ala Asn Gly Lys Asp Asp
               20                  25                  30
   His Arg Ala Gly Ser Arg Pro Gln Ser Xaa Ala Ala Asp Asp Asp Thr
           35                  40                  45
   Ser Ser Glu Leu Gln Arg Leu Ala Glu Met Asp Ala Pro Gln Gln Gly
       50                  55                  60
   Arg Gly Gly Phe Arg Arg Ile Val Arg Leu Val Gly Val Ile Arg Asp
   65                  70                  75                  80
   Trp Ala Asn Lys Asn Phe Arg Glu Glu Glu Pro Arg Pro Asp Ser Phe
                   85                  90                  95
   Leu Glu Arg Phe Arg Gly Pro Glu Leu Gln Thr Val Thr Thr Gln Gln
               100                 105                 110
   Gly Asp Gly Lys Gly Asp Lys Asp Gly Glu Gly Lys Gly Thr Lys Lys
           115                 120                 125
   Lys Phe Glu Leu Phe Val Leu Asp Pro Ala Gly Asp Trp Tyr Tyr Arg
       130                 135                 140
   Trp Leu Phe Val Ile Ala Met Pro Val Leu Tyr Asn Trp Cys Leu Leu
   145                 150                 155                 160
   Val Ala Arg Ala Cys Phe Ser Asp Leu Gln Arg Gly Tyr Phe Val Val
                   165                 170                 175
   Trp Leu Val Leu Asp Tyr Phe Ser Asp Val Val Tyr Ile Ala Asp Leu
               180                 185                 190
   Phe Ile Arg Leu Arg Thr Gly Phe Leu Glu Gln Gly Leu Leu Val Lys
           195                 200                 205
   Asp Pro Lys Lys Leu Arg Asp Asn Tyr Ile His Thr Leu Gln Phe Lys
       210                 215                 220
   Leu Asp Val Ala Ser Ile Ile Pro Thr Asp Leu Ile Tyr Phe Ala Val
   225                 230                 235                 240
   Gly Ile His Ser Pro Glu Val Arg Phe Asn Arg Leu Leu His Phe Ala
   245 250 255
   Arg Met Phe Glu Phe Phe Asp Arg Thr Glu Thr Arg Thr Ser Tyr Pro
   260 265 270
   Asn Ile Phe Arg Ile Ser Asn Leu Val Leu Tyr Ile Leu Val Ile Ile
   275 280 285
   His Trp Asn Ala Cys Ile Tyr Tyr Ala Ile Ser Lys Ser Ile Gly Phe
   290 295 300
   Gly Val Asp Thr Trp Val Tyr Pro Asn Ile Thr Asp Pro Glu Tyr Gly
   305 310 315 320
   Tyr Leu Ala Arg Glu Tyr Ile Tyr Cys Leu Tyr Trp Ser Thr Leu Thr
   325 330 335
   Leu Thr Thr Ile Gly Glu Thr Pro Pro Pro Val Lys Asp Glu Glu Tyr
   340 345 350
   Leu Phe Val Ile Phe Asp Phe Leu Ile Gly Val Leu Ile Phe Ala Thr
   355 360 365
   Ile Val Gly Asn Val Gly Ser Met Ile Ser Asn Met Asn Ala Thr Arg
   370 375 380
   Ala Glu Phe Gln Ala Lys Ile Asp Ala Val Lys His Tyr Met Gln Phe
   385 390 395 400
   Arg Lys Val Ser Lys Asp Met Glu Ala Lys Val Ile Lys Trp Phe Asp
   405 410 415
   Tyr Leu Trp Thr Asn Lys Lys Thr Val Asp Glu Arg Glu Val Leu Lys
   420 425 430
   Asn Leu Pro Ala Lys Leu Arg Ala Glu Ile Ala Ile Asn Val His Leu
   435 440 445
   Ser Thr Leu Lys Lys Val Arg Ile Phe Gln Asp Cys Glu Ala Gly Leu
   450 455 460
   Leu Val Glu Leu Val Leu Lys Leu Arg Pro Gln Val Phe Ser Pro Gly
   465 470 475 480
   Asp Tyr Ile Cys Arg Lys Gly Asp Ile Gly Lys Glu Met Tyr Ile Ile
   485 490 495
   Lys Glu Gly Lys Leu Ala Val Val Ala Asp Asp Gly Val Thr Gln Tyr
   500 505 510
   Ala Leu Leu Ser Ala Gly Ser Cys Phe Gly Glu Ile Ser Ile Leu Asn
   515 520 525
   Ile Lys Gly Ser Lys Met Gly Asn Arg Arg Thr Ala Asn Ile Arg Ser
   530 535 540
   Leu Gly Tyr Ser Asp Leu Phe Cys Leu Ser Lys Asp Asp Leu Met Glu
   545 550 555 560
   Ala Val Thr Glu Tyr Pro Asp Ala Lys Lys Val Leu Glu Glu Arg Gly
   565 570 575
   Arg Glu Ile Leu Met Lys Glu Gly Leu Leu Asp Glu Asn Glu Val Ala
   580 585 590
   Ala Ser Met Glu Val Asp Val Gln Glu Lys Leu Glu Gln Leu Glu Thr
   595 600 605
   Asn Met Glu Thr Leu Tyr Thr Arg Phe Ala Arg Leu Leu Ala Glu Tyr
   610 615 620
   Thr Gly Ala Gln Gln Lys Leu Lys Gln Arg Ile Thr Val Leu Glu Thr
   625 630 635 640
   Lys Met Lys Gln Asn Asn Glu Asp Asp Tyr Leu Ser Asp Gly Met Asn
   645 650 655
   Ser Pro Phe Pro Ala Ala Ala Glu
   660
<210> 19
   <211> 821
   <212> PRT
   <213> Artificial Sequence
<220>
   <223> human CNGA and CNGB consensus sequence
<220>
   <221> MISC FEATURE
   <222> (1) .. (821)
   <223> Xaa = any naturally occurring amino acid
<400> 19
   Met Xaa Xaa Xaa Xaa Ile Gly Thr Gln Xaa Ser Xaa Xaa Ser Xaa Xaa
   1               5                   10                  15
   Asn Leu Xaa Val Pro Xaa Xaa Glu Lys Ala Xaa Xaa Arg Ala Glu Asn
               20                  25                  30
   Xaa Gly Xaa Ser Arg Ala His Ser Xaa Ala Asp Asp Xaa Ala Ser Xaa
           35                  40                  45
   Xaa Xaa Xaa Xaa Xaa Xaa Xaa Glu Xaa Glu Xaa Leu Ala Asp Xaa Ala
       50                  55                  60
   Xaa Gly Ser Phe Ser Gly Xaa Gly Xaa Arg Lys Xaa Xaa Xaa Xaa Xaa
   65                  70                  75                  80
   Xaa Xaa Xaa Xaa Xaa Xaa Xaa Xaa Xaa Xaa Xaa Xaa Xaa Xaa Xaa Xaa
                   85                  90                  95
   Xaa Xaa Xaa Xaa Xaa Xaa Xaa Xaa Xaa Xaa Xaa Xaa Xaa Xaa Arg Leu
               100                 105                 110
   Xaa Xaa Leu Xaa Arg Xaa Trp Ala Xaa Xaa Asn Val Arg Xaa Xaa Xaa
           115                 120                 125
   Pro Xaa Pro Asp Ser Glu Pro Glu Glu Phe Lys Xaa Xaa Xaa Xaa Xaa
       130                 135                 140
   Xaa Xaa Xaa Xaa Xaa Xaa Xaa Xaa Xaa Xaa Xaa Xaa Xaa Xaa Xaa Xaa
   145 150 155 160
   Xaa Xaa Xaa Xaa Xaa Xaa Xaa Xaa Xaa Xaa Xaa Xaa Xaa Xaa Xaa Xaa
   165 170 175
   Xaa Xaa Xaa Xaa Xaa Xaa Xaa Xaa Xaa Xaa Xaa Xaa Xaa Xaa Xaa Xaa
   180 185 190
   Glu Leu Lys Glu Val Lys Ser Gln Asp Xaa Asp Val Lys Xaa Asp Glu
   195 200 205
   Xaa Xaa Xaa Xaa Xaa Xaa Xaa Xaa Xaa Xaa Xaa Xaa Xaa Xaa Xaa Xaa
   210 215 220
   Xaa Xaa Xaa Xaa Xaa Xaa Xaa Xaa Xaa Xaa Xaa Xaa Xaa Xaa Xaa Xaa
   225 230 235 240
   Xaa Ser Glu Asp Lys Lys Xaa Lys Lys Lys Xaa Xaa Xaa Xaa Xaa Lys
   245 250 255
   Leu Leu Xaa Val Ile Asp Pro Ser Gly Asp Xaa Leu Tyr Tyr Leu Trp
   260 265 270
   Leu Phe Xaa Ile Ala Leu Pro Val Asn Tyr Asn Trp Cys Leu Leu Val
   275 280 285
   Ala Arg Ala Cys Phe Pro Asp Leu Gln Ser Asp Tyr Leu His Tyr Trp
   290 295 300
   Leu Val Leu Asp Tyr Val Ser Asp Val Val Tyr Leu Leu Asp Met Xaa
   305 310 315 320
   Phe Val Arg Thr Arg Thr Gly Phe Leu Glu Gln Gly Leu Leu Val Val
   325 330 335
   Asp Thr Asn Lys Leu Arg Asn Asn Tyr Lys Thr Thr Leu Gln Phe Lys
   340 345 350
   Leu Asp Val Ala Ser Leu Ile Pro Thr Asp Leu Leu Tyr Leu Lys Val
   355 360 365
   Gly Xaa Asn Tyr Pro Glu Val Arg Leu Asn Arg Leu Leu Lys Phe Ser
   370 375 380
   Arg Leu Phe Glu Phe Phe Asp Arg Thr Glu Thr Arg Thr Asn Tyr Pro
   385 390 395 400
   Asn Ile Phe Arg Ile Ile Asn Leu Val Leu Tyr Ile Leu Val Ile Ile
   405 410 415
   His Trp Asn Ala Cys Val Tyr Tyr Ala Ile Ser Lys Tyr Ile Gly Phe
   420 425 430
   Gly Thr Asp Thr Trp Val Tyr Pro Gly Ile Gly Asp Pro Glu Phe Gly
   435 440 445
   Arg Leu Ala Arg Glu Tyr Ile Tyr Ser Leu Tyr Trp Ser Thr Leu Thr
   450 455 460
   Leu Thr Thr Ile Gly Glu Thr Pro Pro Pro Val Thr Asp Glu Glu Tyr
   465 470 475 480
   Val Phe Val Val Val Asp Phe Leu Ile Gly Val Leu Ile Phe Ala Thr
   485 490 495
   Ile Val Gly Asn Met Gly Ser Val Ile Ser Asn Met Asn Ala Ala Arg
   500 505 510
   Ala Glu Phe Gln Ala Lys Ile Asp Ala Val Lys Gln Tyr Met Asn Phe
   515 520 525
   Arg Lys Val Ser Lys Asp Val Glu Lys Arg Val Ile Thr Trp Phe Asp
   530 535 540
   Tyr Leu Trp Thr Asn Lys Lys Thr Val Asp Glu Ser Glu Val Leu Lys
   545 550 555 560
   Xaa Leu Pro Asp Lys Leu Arg Ala Glu Ile Ala Ile Asn Val His Leu
   565 570 575
   Ser Thr Leu Ser Lys Val Arg Ile Phe Gln Asp Cys Glu Ala Gly Leu
   580 585 590
   Leu Val Glu Leu Val Leu Lys Leu Arg Pro Gln Val Tyr Ser Pro Gly
   595 600 605
   Asp Tyr Val Cys Lys Lys Gly Asp Ile Gly Lys Glu Met Tyr Ile Ile
   610 615 620
   Lys Glu Gly Lys Leu Ala Val Val Ala Asp Xaa Asp Gly Val Thr Gln
   625 630 635 640
   Leu Val Val Leu Ser Ala Gly Ser Val Phe Gly Glu Ile Ser Ile Leu
   645 650 655
   Asn Ile Lys Gly Ser Lys Ser Gly Asn Arg Arg Thr Ala Asn Ile Val
   660 665 670
   Ser Leu Gly Tyr Ser Asp Leu Phe Cys Leu Ser Lys Asp Asp Leu Met
   675 680 685
   Glu Ala Leu Thr Glu Tyr Pro Asp Ala Lys Lys Ile Leu Glu Glu Lys
   690 695 700
   Gly Arg Glu Ile Leu Met Lys Asp Asn Leu Leu Asp Glu Asn Xaa Ala
   705 710 715 720
   Xaa Xaa Xaa Xaa Xaa Xaa Xaa Xaa Xaa Xaa Ala Gly Xaa Ala Thr Pro
   725 730 735
   Lys Asp Leu Glu Glu Lys Leu Gly Gly Leu Gly Lys Ser Xaa Xaa Xaa
   740 745 750
   Xaa Xaa Xaa Xaa Xaa Xaa Leu Asp Thr Leu Gln Thr Arg Phe Ala Arg
   755 760 765
   Leu Leu Ala Glu Tyr Glu Ala Ala Gln Gln Lys Xaa Xaa Leu Lys Gln
   770 775 780
   Arg Leu Ser Xaa Leu Glu Lys Gln Xaa Lys Glu Gly Xaa Xaa Xaa Xaa
   785 790 795 800
   Asp Xaa Glu Xaa Ala Asp Asp Glu Gly Glu Pro Asp Glu Xaa Ala Pro
   805 810 815
   Xaa Asp Glu Pro Glu
   820
<210> 20
   <211> 5
   <212> PRT
   <213> Artificial Sequence
<220>
   <223> mammalian Gi protein
<400> 20
   Asp Cys Gly Leu Phe
   1 5
<210> 21
   <211> 5
   <212> PRT
   <213> Artificial Sequence
<220>
   <223> mammalian Gi protein
<400> 21
   Glu Cys Gly Leu Tyr
   1 5
<210> 22
   <211> 5
   <212> PRT
   <213> Artificial Sequence
<220>
   <223> mammalian Gi protein
<400> 22
   Gly Cys Gly Leu Tyr
   1 5
<210> 23
   <211> 5
   <212> PRT
   <213> Artificial Sequence
<220>
   <223> mammalian Gi protein
<400> 23
   Tyr Ile Gly Leu Cys
   1               5

## Description

### FIELD OF THE INVENTION

The invention relates generally to cellular physiology. In particular, the invention relates to novel tools for use in cell-based assays that employ calcium and voltage-based dyes and other indicators, particularly novel CNG mutants for use in improved cyclic nucleotide assays. Such tools are useful for measuring the activity of G protein coupled receptors, and in other assays involving changes in intracellular levels of cyclic nucleotides.

### BACKGROUND OF THE INVENTION

G-protein-coupled receptors (GPCRs) comprise a large super-family of integral membrane proteins characterized by having 7 hydrophobic alpha helical transmembrane (TM) domains with three intracellular and three extracellular loops (Ji, et al., J Biol Chem 273:17299-17302, 1998). In addition all GPCRs contain N-terminal extracellular and C-terminal intracellular domains. Binding of extracellular ligand may be mediated by the transmembrane domains, the N-terminus, or extracellular loops, either in alone or in combination. For example binding of biogenic amines such as epinephrine, norepinephrine, dopamine, and histamine is thought to occur primarily at the TM3 site while TM5 and TM6 provide the sites for generating an intracellular signal. Agonist binding to GPCRs results in activation of one or more intracellular heterotrimeric GTP-binding proteins (G proteins) which, in turn, transduce and amplify the signal by subsequent modulation of down-stream effector molecules (such as enzymes, ion channels and transporters). This in turn results in rapid production of second messengers (such as cAMP, cGMP, inositol phosphates, diacylglycerol, cytosolic ions).

GPCRs mediate signal transduction across a cell membrane upon the binding of a ligand to a GPCR. The intracellular portion of the GPCR interacts with a G protein to modulate signal transduction from outside to inside a cell. A GPCR is thus coupled to a G protein. There are three polypeptide subunits in a G-protein complex: an alpha subunit-which binds and hydrolyzes GTP-and a dimeric beta-gamma subunit. In the inactive state, the G protein exists as a heterotrimer of the alpha and beta-gamma subunits. When the G protein is inactive, guanosine diphosphate (GDP) is associated with the alpha subunit of the G protein. When a GPCR is bound and activated by a ligand, the GPCR binds to the G-protein heterotrimer and decreases the affinity of the G alpha subunit for GDP. In its active state, the G subunit exchanges GDP for guanine triphosphate (GTP) and active G alpha subunit disassociates from both the GPCR and the dimeric beta-gamma subunit. The disassociated, active G alpha subunit transduces signals to effectors that are "downstream" in the G-protein signaling pathway within the cell. Eventually, the G protein's endogenous GTPase activity returns active G subunit to its inactive state, in which it is associated with GDP and the dimeric beta-gamma subunit.

The transduction of the signal results in the production of second messenger molecules. Once produced, the second messengers have a wide variety of effects on cellular activities. One such activity is the activation of cyclic nucleotide-gated (CNG) channels by the cyclic nucleotides cAMP and cGMP. CNG channels are membrane spanning molecules that control the flux of cations through the cellular membrane. The channels are activated-opened-by increased intracellular concentrations of cyclic nucleotide. Once opened the channels conduct mixed cation currents, including ions of Na⁺, K⁺, Mg²⁺ and Ca²⁺, for example. The activity of the CNG channels couples electrical excitation and Ca²⁺ signaling to changes in the intracellular concentration of cyclic nucleotides (Figure 1). US 2003/0157571 discloses a rat CNG mutant delta61-90/C460W/E583M which has increased sensitivity towards cAMP and a decreased sensitivity against Ca²⁺-calmodulin.

Receptor function is regulated by the G protein itself (GTP-bound form is required for coupling), by phosphorylation (by G-protein-coupled receptor kinases or GRKs) and by binding to inhibitory proteins known as β-arrestins (Lefkowitz, J Biol Chem, 273:18677-18680, 1998). It has long been established that many medically significant biological processes are mediated by proteins participating in signal transduction pathways that involve G proteins and/or second messengers (Lefkowitz, Nature, 351:353-354, 1991). In fact, nearly one-third of all prescription drugs are GPCR ligands (Kallal et al., Trends Pharmacol Sci, 21:175-180, 2000).

GPCRs fall into three major classes (and multiple subclasses) based on their known (or predicted) structural and functional properties (Rana et al., Ann Rev Pharmacol Toxicol, 41:593-624, 2001; Marchese et al., Trends Pharmacol Sci, 20:370-375, 1999). Most of these receptors fall into class A, including receptors for odorants, light, and biogenic amines, for chemokines and small peptides, and for several glycopeptide/glycoprotein hormones. Class B receptors bind higher molecular weight hormones while class C includes GABA_{B} receptors, taste receptors, and Ca²⁺-sensing receptors. GPCRs are found in all tissues. However, expression of any individual receptor may be limited and tissue-specific. As such some GPCRs may be used as markers for specific tissue types.

As might be expected from the wide range of GPCRs and GPCR ligands, aberrant function of these molecules has been implicated in a large number of human disease states (Rana *et al.* and Ji *et al.,* supra). GPCR agonists and antagonists have been developed to treat many of these diseases. For example the important group of receptors for biogenic amines has been the target of a large number of successful drugs. Among the receptors in this group are those for epinephrine and norepinephrine (α- and β-adrenergic receptors), dopamine, histamine, and serotonin. Examples of diseases in which GPCR function has been implicated include, but are by no means limited to: heart disease (*e.g*. tachycardia, congestive heart failure, etc.), asthma, hypertension, allergic reactions (including anaphylactic shock), gastrointestinal disorders, and a wide range of neurological disorders (*e.g*. Parkinson's disease, depression, schizophrenia, etc.). Finally, many receptors for drugs of abuse are GPCRs.

In many animals, GPCRs are found throughout the organism and are responsible for the maintenance of normal function as well as for pathological conditions. In other instances, the expression of specific GPCRs or families of GPCRs is very tightly controlled, *e.g*., being expressed only during early developmental stages, etc. Consequently, it is important to find compounds that can stimulate or activate GPCRs, or inhibit or deactivate GPCRs as needed. Agonists-compounds that stimulate the normal function of the GPCRs-have been used to treat asthma, Parkinson's disease, acute heart failure, osteoporosis, hypotension, etc. Antagonists, compounds that interfere with or block normal function have been used to treat, hypertension, myocardial infarction, ulcers, asthma, allergies, psychiatric and neurological disorders, anorexia and bulimia.

In addition to well-characterized receptors, many "orphan" receptors have been cloned (Marchese *et al.,* supra) which are known from sequence similarities to be part of these families, but for which no function or ligand(s) have been discerned. Given the central role of GPCRs in control of diverse cellular activities, there remains a need in the art for methods to identify the agonists and antagonists of these "orphan" receptors as well as to identify additional antagonists for those receptors whose agonists-ligands-are known.

As the first recognized second messenger, cAMP is synthesized by adenylate cyclase in response to activation of many receptors coupled to G proteins Gₛ and G_{olf} and cyclase activity is inhibited by activation of receptors coupled to G protein Gᵢ. cAMP activates cAMP-dependent protein kinase A (PKA) resulting in profound cellular responses. Physiologically, cAMP mediates such hormonal responses as mobilization of stored energy (*e.g*., the breakdown of carbohydrates in liver or triglycerides in fat cells, conservation of water by kidney, and Ca²⁺ homeostasis), control of the rate and contraction force of the heart muscle, relaxation of smooth muscle, production of sex hormones, and many other endocrine and neural processes.

There are a number of cAMP assays currently available. They include transcription reporter assay where a luciferase reporter is driven with a cAMP response promoter element CRE, cAMP immunoassay (Applied Biosystems Forster City, CA), an *in vitro* enzymatic assay for adenylyl cyclase (Molecular Devices, Sunnyvale, CA) and cAMP fluorescence polarization assay (PerkinElmer Life Sciences, Boston, MA). However, all these assays are end point assays where the cells are lysed and extracts are used for the tests. R.Y. Tsien and his colleagues have also developed fluorescent probes that report cAMP levels in single cells. However, the methods of application of these probes to cells makes them not suitable for high throughput screening formats (Adams et al., 1991, Nature 349:694-697; Zoccolo et al., 2000, Nat. Cell Biol. 2:25-29). There is a need in the art to be able to detect the activation of individual living cells for their cAMP production, particularly in a heterogeneous cell or tissue environment. Such detection capability would further allow the examination of receptor activation and cellular response to complex stimuli, as in the case of induced long-term memory. There also exists in the art a need for the ability to directly examine the cAMP in live cells in order to identify ligands for orphan GPCRs based on the concurrent examination of both Ca²⁺ and cAMP activation in a given cell as well as to identify agents that modulate GPCR-mediated activity. These and other needs are met by the present invention.

### SUMMARY OF THE INVENTION

The assays and methods of the present invention provide novel tools for use in cAMP assays. In particular, the invention provides novel CNG mutants for use in CNG-based assays, particularly assays for monitoring the activity of GPCRs and GPCR signaling cascades. The novel CNG mutants comprise
(a) at least one mutation that decreases divalent cation-mediated blockage of cation flux; and
(b) at least one mutation that makes the channel more sensitive to cAMP than a channel that does not comprise the mutation,
wherein said mutant CNG channel is a mutant of rat CNGA2 comprising SEQ ID NO:2, wherein said at least one mutation that decreases divalent cation-mediated blockage of cation flux comprises a mutation at position E342, and wherein said at least one mutation that makes the channel more sensitive to cAMP than a channel that does not comprise the mutation comprises a mutation at position C460 and/or E583.

Also described are quencher molecules for use in CNG-based assays and other assays employing calcium and voltage-based dyes. In such assays, membrane-impermeable quenchers have often been used in combination with fluorescence indicators to reduce background extracellular fluorescence while maintaining intracellular fluorescence intact. However, assays may be negatively effected when such quenchers exhibit pharmacological interference with the biological interaction being measured. The quencher molecules described herein are pharmacologically inert and therefore compatible with assaying of peptide, protein, nucleoside lipid and small organic molecules. Therefore, these molecules may be employed in a wide variety of assays employing fluorescent voltage dyes, including the GPCR/CNG assays exemplified herein.

### BRIEF DESCRIPTION OF THE DRAWINGS

Figure 1 is a schematic of the signaling pathway from a GPCR to a CNG channel.

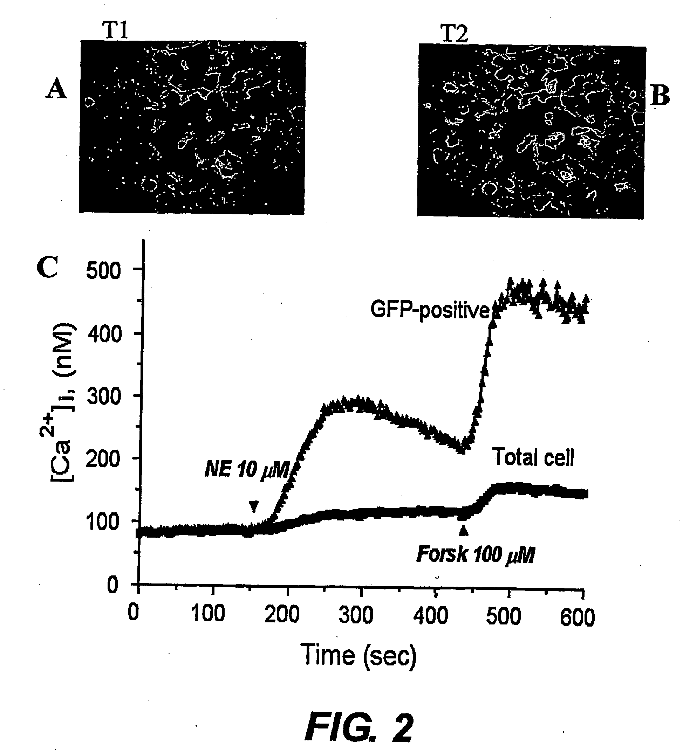

Figure 2A is a photograph of HEK239 cells transfected with a mutated rat CNG channel gene (SEQ ID NO: 3) prior to their being stimulated with a ligand for an endogenous GPCR. Figure 2B shows the cells after stimulation with norepinephrine (NE) that activates endogenous β2-adrenoceptor, a Gs coupled receptor. The signal is detected with a calcium sensitive fluorescence dye, Fura-2 (Molecular Probes, Eugene, OR) by using a fluorescence microscope. Figure 2C illustrated an integrated result on intracellular Ca²⁺ concentration of 100 individual cells by using ATTO Graph (ATTO, Rockville, MD) as a function of time. A plasmid expressing a green fluorescence protein (GFP) was co-transfected with the CNG gene in the host cells.

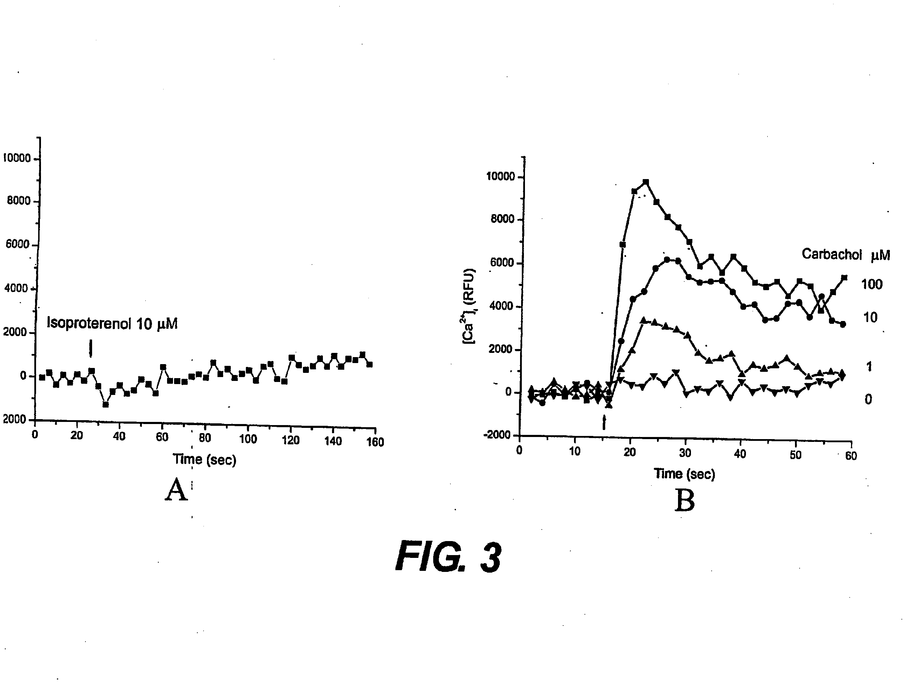

Figure 3A is a graph of intracellular Ca²⁺ concentration as a function of time in cells transfected with a mutated CNG channel gene (SEQ ID NO: 5) and stimulated with a ligand to Gs coupled endogenous β2-adrenoceptor that results in the accumulation of cAMP and the activation of the CNG channel. The determination of concentration was made by fluorescence of an intracellular dye (Fura-2) as measured by a commercially available microplate reader. Figure 3B is a graph of intracellular Ca²⁺ concentration as a function of time in cells transfected with a CNG channel and stimulated with a ligand activating a Gq coupled GPCR, resulting in the mobilization of intracellular Ca²⁺ stores.
Figure 4 is a graph of membrane potential as a function of time in cells transfected with a mutated CNG channel gene (SEQ ID NO: 7) and stimulated with forskolin, a direct adenylyl cyclase activator, resulting in the activation of the CNG. The determination of membrane potential was made using a commercially available voltage sensitive dye kit in a multiwell plate reader.
Figure 5 is a graph of membrane potential as a function of time in cells transfected with a mutated CNG channel gene (SEQ ID NO: 5) and stimulated with a ligand for Gs coupled endogenous β2-adrenoceptor that results in the production of cAMP and activation of the CNG channel. The determination of membrane potential was made using a commercially available voltage sensitive dye kit in a multiwell plate reader.
Figure 6 is a graph of membrane potential as a function of time in cells transfected with a triple mutant CNG channel gene (SEQ ID NO: 7) and stimulated with the indicated concentrations of a ligand to an endogenous Gs coupled β2-adrenoceptor that results in activation of the CNG channel. The determination of membrane potential was made using a commercially available voltage sensitive dye kit in a multiwell plate reader.
Figure 7 is a graph of membrane potential as a function of time in cells transfected with both a mutated CNG channel gene (SEQ ID NO: 7) and an heterologous Gs coupled GPCR, dopamine receptor D1, and stimulated with a dopamine. The determination of membrane potential was made using a commercially available voltage sensitive dye kit in a multiwell plate reader.
Figures 8A-C provide a sequence alignment of human CNG channels CNGA1 (SEQ ID NO: 9), CNGA2 (SEQ ID NO: 10), CNGA3 (SEQ ID NO: 11), CNGB1 (SEQ ID NO: 12), CNGB2 (SEQ ID NO: 13) and CNGB3 (SEQ ID NO: 14). A consensus sequence of this multiple alignment is also shown (SEQ ID NO: 19).
Figures 9A-B provide a sequence alignment of CNGA2 channels from the following mammals: human (SEQ ID NO: 10), rat (SEQ ID NO: 2), mouse (SEQ ID NO: 15), rabbit (SEQ ID NO: 16) and bull (SEQ ID NO: 17). A consensus sequence of this multiple alignment is also shown (SEQ ID NO: 18).
Figures 10A-D. Figure 10A depicts the response of HEK293H-CNG cells to 1 µM isoproterenol. Figure 10B depicts the response of non-transformed parental HEK293H cells to 1 µM isoproterenol. Figure 10C depicts dose-response curves of HEK293H-CNG cells to 0 (control), 1 nM, 3 nM, 10 nM, 30 nM, 0.1 µM, 0.3 µM and 1 µM isoproterenol. Figure 10D depicts the well-to-well consistency of the readings for measuring CNG activation.
Figure 11 depicts calcium uptake by HEK293H-CNG cells in a dose-dependent manner to 0 (control), 0.3, 1, 3, 10, 30 and 300 nM isoproterenol.
Figures 12A-B. Figure 12A shows voltage sensitive fluorescence of the same living cells immediately before the addition of 1 µM isoproterenol and 15, 30 and 45 seconds after addition. Figure 12B depicts the background corrected average fluorescence of 71 imaged cells.
Figure 13 depicts the sensitivity of CNG channels which are composed of wildtype heteromeric (α+β) subunits, wildtype homomeric (α) subunits, or mutant homomeric CNG channels comprising one (Y565A), two (C460R/E583M and C460H/E583M), or three (C460W/Y565A/E583M) substitution mutations.
Figure 14 depicts a comparison between the CNG channel assay and conventional CRE and ELISA assays. Each assay format is depicted as a dose-response to forskolin stimulation.
Figure 15 depicts the sensitivity of the CNG channel assay for activation of GPCRs for various ligands. Representative dose-response curves are depicted for Parathyroid Hormone Receptor 1 (PTHR1), Histamine Receptor H2 (HRH2), and 5-Hydroxytryptamine 4 Receptor (5-HT4) response to the agonists PTHrP (peptide), Histamine (monoamine), and 5-HT (monoamine), respectively.
Figure 16 depicts a dose-response comparison of the CNG channel assay using membrane potential dye to calcium assays using promiscuous G protein (Gα₁₆) or chimeric G protein (Gα_{qs}) for detecting activation of the tyramine receptor.
Figure 17A depicts the response of HEK293H-CNG cells to a panel adrenergic compounds as depicted in Figure 17B.
Figure 18 is a graph showing concentration-dependent quenching of extracellular fluorescence by a membrane impermeable absorbance dye, evans blue. Concentration-dependent fluorescence quenching is fitted to the Stern-Volmer equation. The smooth curve in figure is the best non-linear fit to the total fluorescence, as the most fluorescence readings are contributed by extracellular dye fluorescence. The half-quenching concentration is 0.38 g/L, corresponding to 0.34 mM evans blue.
Figure 19 is a graph showing how dye quenchers containing positive charges may interfere with cell responses. A stable 293H cell line expressing CNG channel and glucagon receptor, ASC0089, was loaded with a voltage dye, HLB30602 of 5 µM in the presence of a quencher dye for 1 h at room temperature. Cell response to glucagon was abolished when using dye quenchers, acid violet 17 (AV17), diazine black (DB), and HLB30818, which contain positive charges. Trypan blue (TB) is a negative-charged dye.
Figure 20 is a graph showing that most pre-selected negative-charged dye quenchers are suitable for assay of adenosine receptor A2B responses to an agonist, NECA, including HLB30701, HLB30702, HLB30703, and trypan blue. However, a negative-charged dye, bromophenol blue (BpB) reduced NECA response without affecting NECA potency.
Figure 21 is a graph showing that response to glucagon stimulation was interfered by dye quenchers, HLB30702, trypan blue (TB), and bromophenol blue (BpB). Trypan blue did not inhibit maximal responses but reduced glucagon potency by about one order in comparison with quenchers, HLB30701, HLB30703, and a commercial dye kit from Molecular Devices, Corp. (MDC, Cat # R-8034).
Figure 22 is a graph showing glucagon dose-responses in the presence of quencher dyes. Cell line: ASC0089 (GCGR). Glucagon potency increased using nitrazine yellow as a quencher.
Figure 23 is a graph showing VIP dose-responses in the presence of quencher dyes. Cell line: ASC0145 (VIP2R). VIP potency increased using nitrazine yellow as a quencher by more than one order comparing using a commercial dye kit from Molecular Devices, Corp. (MDC, Cat # R-8034).
Figure 24 is a graph showing GIP dose-responses in the presence of quencher dyes. Cell line: ASC0133 (GIPR). GIP potency increased using nitrazine yellow as a quencher by one order comparing using a commercial dye kit from Molecular Devices, Corp. (MDC, Cat # R-8034).
Figure 25 shows the formula of Nitrazine Yellow.
Figure 26 shows the formula of Nitro Red.
Figure 27 shows assay results using a stable HEK293 cell line expressing CNGA2 mutant (E342G, C460W, E583M). In assay using voltage dyes with CNG channel containing the pore mutation E342G, EGTA is no more required to be added to remove calcium from extracellular space. Assay using voltage dyes was performed in the presence of extracellular calcium of 0.9-2 mM, compound was dissolved in DPBS (A, B). In assay using calcium dyes with CNG channel containing the pore mutation E342G, compound was dissolved in media containing 30 mM CaCl2 to yield a final CaCl2 concentration around 8 mM (C, D).

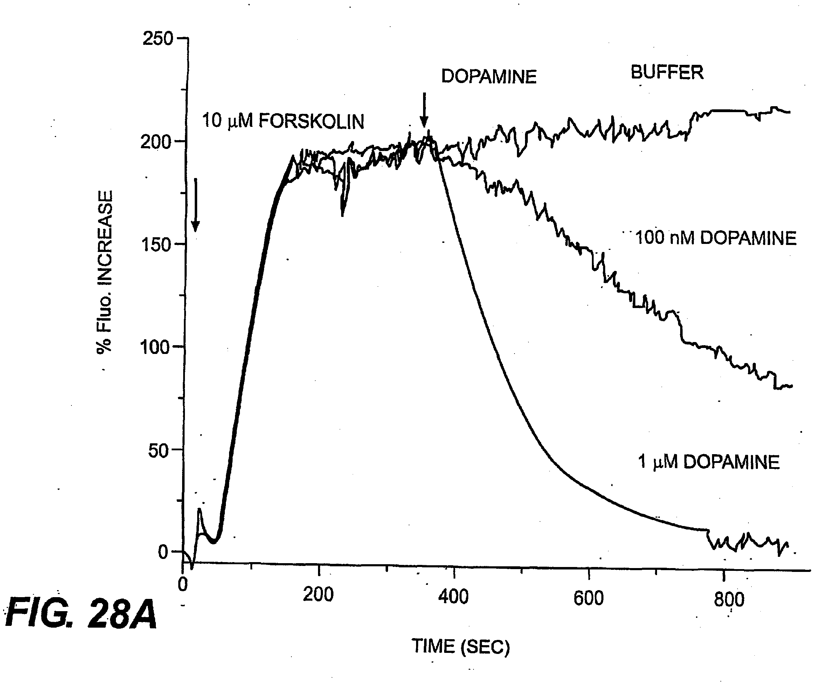

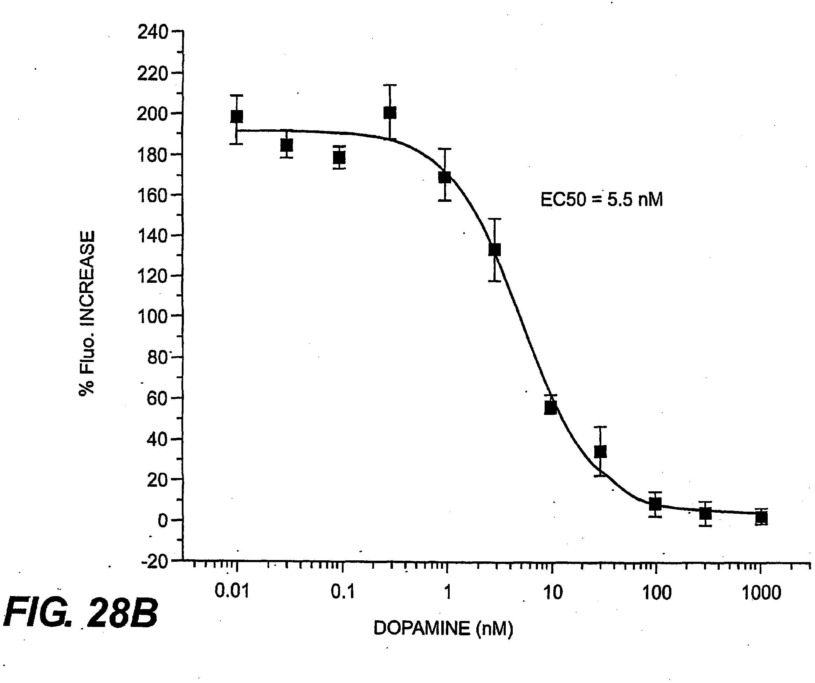

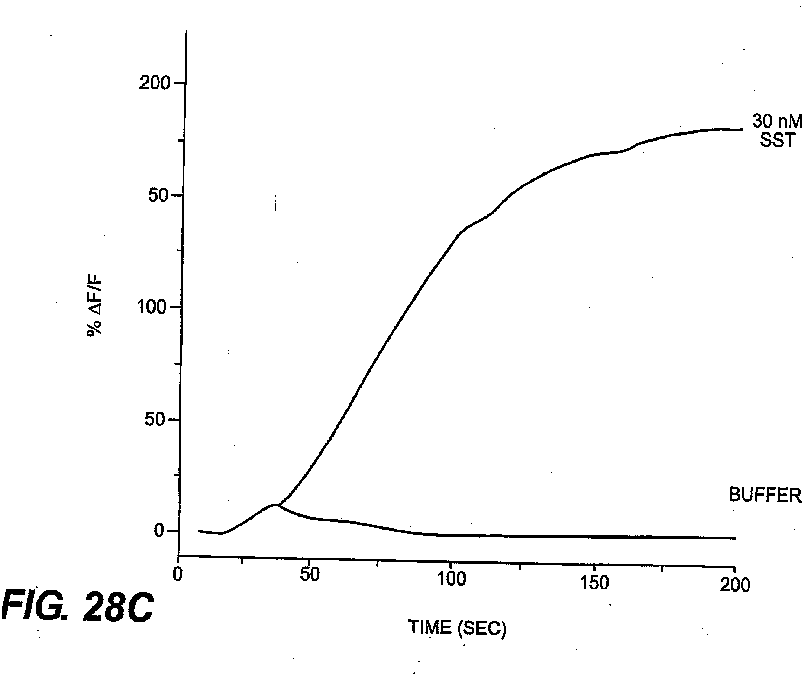

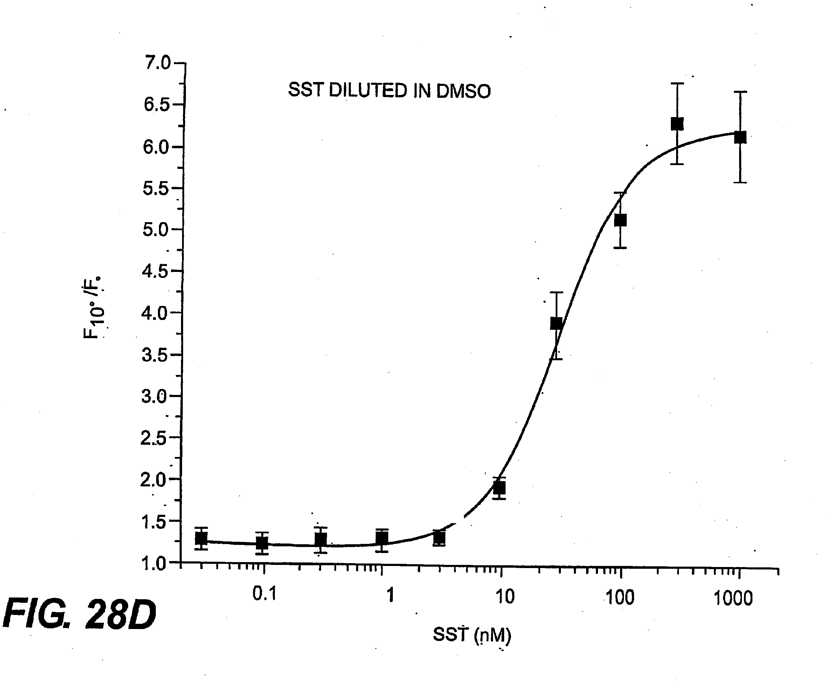

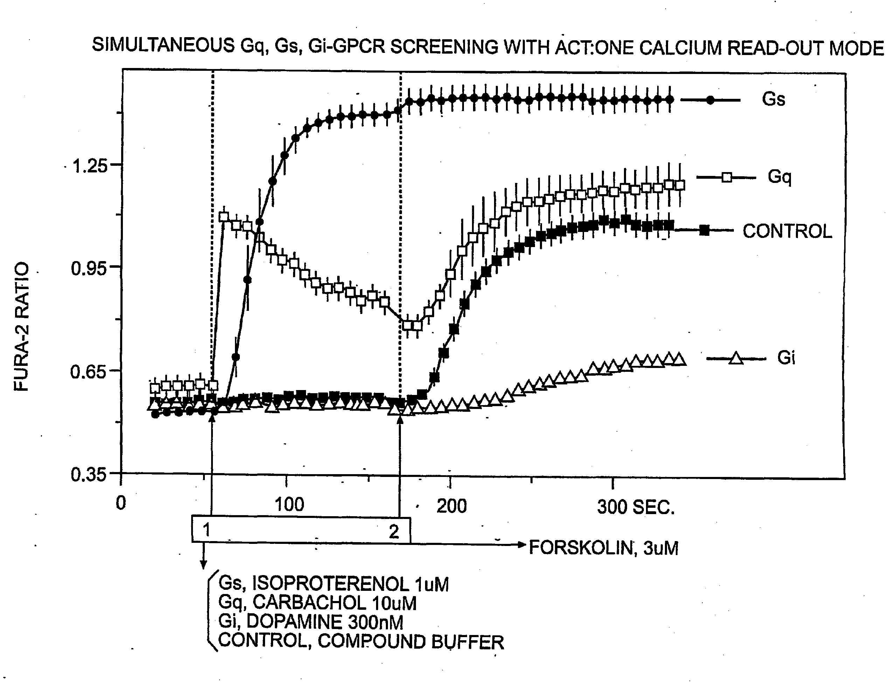

Figure 28A is a graph showing the kinetic response to dopamine in a CNG-DRD2 cell line measured in 96-well plates with FLEXstation plate reader. Figure 28B is a graph showing the results of an Endpoint assay of CNG-DRD2 cells measured in 96-well plates with FLEXstation plate reader. Figure 28C is a graph showing the kinetic response to somatostatin (SST) in HEK293H-CNG cell line transiently co-transfected with a Gi-coupled SSTRS receptor and Gs-i3 chimeric G-protein. Recording was made in a 96-well plate with FLEXstation. Figure 28D is a graph showing the results of an endpoint assay of responses to somatostatin (SST) in a cell line HEK293H-CNG:SSTR5::Gs-i3 stably expressing CNG, SSTR5 and a chimeric Gs-i3 protein. Recording was made in a 384-well plate with FLEXstation.
Figure 29 is a graph comparing calcium responses following Gq, Gs and Gi-GPCR activation.

### DETAILED DESCRIPTION OF THE PREFERRED EMBODIMENTS

### I. General Description

The novel dye quencher formulations described herein have broad applicability in any cell-based assay that employs a fluorescent detector dye. The CNG mutants of the present invention have broad applicability to any cell based assay for measuring intracellular cAMP levels. These utilities are exemplified by the GPCR/CNG assays described herein, and in application Serial No. 10/087,217.

### Novel Quencher/Dye Formulations

Membrane impermeable fluorescence quenchers are often used in combination with fluorescence indicators to reduce background fluorescence noise and simplify assay procedures. For example, trypan blue was used to reduce extracellular fluorescence background while maintaining intracellular fluorescence intact in assays monitoring internalization of fluorescein conjugates in macrophage cells (Sahlin *et al*., 1983; Hed *et al*., 1987; Wan *et al.,* 1993). Metal ion quencher and absorbance dye quencher were used in combination with fluorescent calcium dyes to measure intracellular calcium (Etter *et al*., 1996; Davies *et al*., 1997; Mehlin et al., Biotechniques, 2003). Collisional quencher I⁻ and an absorbance dye quencher were used in combination with fluorescent voltage dyes to monitor membrane potential (Melikyan *et al.,* 1996; Leenhouts & Kruijff, 1995; US Patent application publication No.: US2003/0087332 A1). Absorbance dye quenchers were used in combination with a fluorescent dye to measure gene transcription (US Patent 6,221,612).

Many dyes used as quenchers were developed for the textile industry. Consequently, the pharmacological effects on living cells typically remains unknown. Assay performance can be negatively impacted when quenchers have pharmacological actions on cell signaling pathways, receptors, or interact with ligands resulting, for example, from covalent, electrostatic, hydrogen and Van der Waals interactions. These interactions are particularly problematic when screening ligands comprised of protein, peptide, nucleoside, lipid or small organic molecules. This application describes quencher dyes that are compatible with assaying of peptide, protein, nucleoside, lipid and small organic molecules, and methods of identifying the same.

Accordingly, described herein is a method of identifying a biologically inert fluorescence quencher comprising
(a) providing a collection of candidate quencher molecules;
(b) providing a panel of at least five receptors, said receptors selected from at least three types and distinguished by pharmacophores, said pharmacophores comprising biogenic amines, adenosines, lipids, alkaloids, amino acids, peptides, and proteins,
(c) assaying activity of said receptors in the presence of an activating pharmacophore and in the presence and absence of members of said collection of candidate quencher molecules;
wherein a biologically inert fluorescence quencher has no effect on the activation of said panel of receptors. In such assays, activation of the receptor is typically quantitated in a manner that does not involve the functional use of the quencher.

In particular, it has been found that trypan blue, a commonly used quencher, has tendency to reduce the potency of a peptide ligands such as glucagon. Nitrazine yellow and a structurally similar molecule, nitro red, were identified as useful quenchers for fluorescent voltage dyes, showing little effect on responses of peptides and other small organic molecules. These quencher dyes have broad applicability in formulations for cell-based assays that use a variety of fluorescent detector dyes. In particular; the quencher dyes described herein may be used in combination with a voltage dye to monitor activity of CNG channels in a live-cell assay for agonists, antagonists or allosteric modulators. The dye/quencher formulation can also be used for deorphanization of GPCRs and other events that alter intracellular cAMP, calcium, and gene expression levels. Formulations of the quencher dyes with a variety of fluorescent detector dyes are provided for drug discovery, toxicology and other cell-based studies.

The formulations are particularly useful for homogeneous assays employing at least one of a fluorescent plate reader, a single-cell imaging platform a facs sorter, and an electrophysiology platform. The formulations may be used in any cell-based assay where cellular uptake is mediated by at least one of endocytosis, receptor internalization, viral infection, peptide-mediated uptake and lipid-mediated uptake. Further, it should be understood that the level of fluorescence may increase or decrease in response to the particular activity being measured.

One advantage of the current dye/quencher/buffer formulation is the ability to omit wash steps normally required to remove unbound dye in solution. The newly developed quencher(s) can be used in combination with other fluorescent biomarkers whenever a no-wash format is desirable. Examples include but are not limited to use of cells sensitive to mechanical stress, cells or assays wherein the biological response is dampened or perturbed as a result of medium/buffer changes, assays where the presence of at least one dye or assay component is required throughout the duration of the assay, such as occurs during steadily up-take of specific probes, *e.g*., vital tracers, or in long-term studies of cell differentiation, motility or division, and in assays in which probes leak out of the cells and washing is not possible/desirable.

Yet another advantage of the present quencher formulations is the ability to use a variety of calcium dyes in combination with the quencher. These include Fluo 3, Fluo 4 and Fura-2. In particular, Fura-2 is a ratio dye, providing higher accuracy measurements by minimizing effects of uneven dye loading between cells, uneven dye loading within single cells, uneven sample illumination, photobleaching, and artifacts, such as those generated if a ligand is inherently fluorescent.

The quenchers described herein have utility in certain FRET or BRET-based assays. Non-fluorescent quenchers such as Dabcyl and QSY dyes (Molecular Probes) are used in FRET combinations where a fluorescent (or luminescent) donor is quenched by a non-fluorescent acceptor (efficient quenchers such as Dabcyl reduce fluorescence emission of most fluorophores by 99%). When FRET is disrupted, the quencher no longer absorbs the donor emission which is then detected as an increase in the specific fluorescence emission of the fluorochrome present in the donor.

Non-fluorescent quenchers have the advantage of eliminating the potential problem of background fluorescence resulting from direct (i.e. nonsensitized) acceptor excitation (rather than excitation via the FRET donor molecule). Examples of commercially available probes containing fluorescent donor-non fluorescent acceptor pairs include probes aimed at detecting proteolysis (EDANS fluorophore/Dabcyl pair for HIV protease and renin substrates; FRET is disrupted upon cleavage of the substrate by specific proteases) (Molecular Probes) and nucleic acid hybridization (molecular beacons: hairpin oligonucleotides with a fluorochrome attached to one end and a non-fluorescent quencher attached to the other end; upon hybridization to a complementary sequence, the hairpin unfolds and FRET is disrupted). Because the quenchers described herein are relatively inert to peptide or proteins, their use in such assays decreases nonspecific binding of cellular materials or peptide agonists/antagonists.

### Novel CNG Mutants for Cell-Based cAMP Assays

Calcium ions present in extracellular media must be chelated in assays applying voltage dyes when using wild-type CNG channels. Otherwise, calcium binds to a glutamate residue at channel pore, thereby blocking ionic currents. The novel CNG mutants of the present invention contain one or more mutations in the channel pore domain, thereby removing calcium blockage of the currents. In addition, these mutations make CNG channels more permeable to calcium. Several combinations of mutations were generated and tested at three positions within the CNG that affect cAMP binding affinity and channel permeability to calcium. Mutations were identified that, alone or in combination, provide significant improvements in the assay that are particularly useful for high-throughput screening. The invention has broad applications, particularly in screening receptors, particularly 7-transmembrane receptors. For instance, the invention provides assays that can be used to detect the binding of agonists, antagonists, or allosteric modulators of 7-transmembrane receptors.

The novel mutants of the present invention may be expressed in combination with other CNG subunits to form heteromeric channels having several advantages. Native CNG channels in olfactory receptor neurons are composed of three subunits: CNGA2, CNGA4 (Bradley J, Li J, Davidson N, Lester HA, Zinn K. (1994). Heteromeric olfactory cyclic nucleotide-gated channels: a subunit that confers increased sensitivity to cAMP. Proc Natl Acad Sci USA. 1994 Sep 13;91(19):8890-4) and CNG4.3 (Sautter A, Zong X, Hofmann F, Biel M. (1998). An isoform of the rod photoreceptor cyclic nucleotide-gated channel beta subunit expressed in olfactory neurons. Proc Natl Acad Sci U S A. 1998 Apr 14;95(8):4696-701; Bonigk W, Bradley J, Muller F, Sesti F, Boekhoff I, Ronnett GV, Kaupp UB, Frings S. (1999). The native rat olfactory cyclic nucleotide-gated channel is composed of three distinct subunits. JNeurosci. 1999 Jul 1;19(13):5332-47; Bradley J, Frings S, Yau KW, Reed R. (2001). Nomenclature for ion channel subunits. Science. 2001 Dec 7;294(5549):2095-6). The CNGA2 protein can form homomeric channels, whereas CNGA4 and CNG4.3 can not. However, coexpression of CNGA2 and CNGA4 and/or CNG4.3 can form heteromeric channels comprised of either two or three subunits. In comparison with homomeric CNGA2 channels expressed in heterologous cells, the heteromeric channels have several advantages, including: (1) higher sensitivity to cAMP (increase detection sensitivity), (2) weakened outward current rectification (resulting in an increased membrane depolarization signal), and (3) better channel assembly with a consequence of higher expression and lower toxicity to cells.

Overall benefits of the new mutants include (1) increased signals when using voltage or calcium dyes; and (2) assays can now be conducted in physiological calcium-containing media. For instance, the novel calcium-insensitive CNG mutations of the invention enable the loading of the membrane potential/quencher cocktail in a more physiological buffer (such as HBSS, Hank's Balanced Salt Solution) rather than in DPBS (Phosphate Buffer Saline calcium/magnesium-free). Cell morphology is much better retained and cells adhere more tightly to the growth surface because the physiologically balanced divalent cation concentration is maintained during the loading period. Also, EGTA is not required in the compound addition buffer and cell shrinkage previously observed upon compound buffer addition is markedly reduced. Overall, such improvements are associated with greater cell responsiveness leading to enhanced overall sensitivity of the assay (both in cell imaging and whole-well reading formats).

The novel calcium-insensitive CNG mutations of the invention enable the simultaneous measurements of cAMP-dependent changes in calcium and membrane potential in the same cells providing the basis for multiplexed assays for ligands with unknown receptor specificity or for receptor de-orphanization (receptors with unknown signaling pathways) screening in a cell imaging platform. In CNG-bearing cells simultaneously labeled with both calcium and membrane potential dyes, activation of Gs-coupled GPCRs will trigger sustained signals in both calcium and membrane potential read-outs. In contrast, activation of Gq-coupled receptors will induce a rapid and transient calcium rise (due to release of intracellular calcium stores) with no effect on the membrane potential. Gi-coupled GPCRs will induce neither signal. Hence, Gs and Gq receptors can be easily distinguished.

Addition of a first compound known to increase cAMP levels, such as isoproterenol (a known Gs-coupled GPCR agonist) or forskolin (a known activator of adenylate cyclase, AC, the enzyme synthesizing cAMP) will result in a sustained signal measurable by at least one of a membrane potential dye, or an ion-sensitive dye (such as calcium a calcium sensitive dye). However, such cells in which a Gi-coupled GPCRs has also been activated will exhibit a reduced response (due to inhibition of AC by Gi) which is reflected in at least one of the membrane potential dye, or the ion-sensitive dye. Hence, the present invention provides for a single assay in which Gs, Gq or Gi receptors can be detected. Such an assay further allows for identification of the coupling specificity of a receptor.

Assays of the present invention also provide for the sequential screening of at least a second receptor. For example, a first compound may be found to induce a Gq-coupled receptor as indicated by transient increase in intracellular calcium. Subsequent addition of a second ligand may be seen to induce prolonged responses in both calcium and membrane potential, indicative of a Gs receptor. In yet a further addition, a third ligand could be seen to have no effect. However, simultaneous addition of forskolin and the third ligand also fails to induce an increase in at least one of membrane potential or calcium signals, whereas addition of forskolin alone does increase in at least one of membrane potential or calcium signals, indicative of a Gi receptor. Hence, the present invention enables multiple assays on single samples.

Further still, the present assay can be multiplexed with other assays to substantially increase the information content. This increases the throughput, reduces material requirements, and provides a basis for prioritizing lead compounds. For example, such assays might include, but are not limited to, the simultaneous assay of apoptosis (using the ratio dye JC1 for detection of changes in mitochondrial membrane potential), thereby providing initial information on the toxicity of particular compounds or classes of compounds. Further, the present invention can be performed simultaneously with redistribution assays where a protein of interest could be tagged with a fluorescent protein, luminescent protein, etc.

The components of multiplexed assays can also be performed sequentially in any order. For example, for Gq-GPCRs, the first transient calcium response will be followed by a sustained Gs-mediated calcium and membrane potential signal. For Gs-GPCRs, the first calcium and membrane potential response will be either not affected (in the case that the preceding ligand exhibited maximal potency/efficacy toward the CNG-mediated signaling) or further enhanced.

The novel calcium-insensitive CNG mutations of the invention exhibit high and homogenous expression in engineered cells (virtually 100% of the cells within newly generated clones bearing mutated CNG respond reproducibly to Gs-coupled agonists of endogenous receptors without showing adverse effects). The new mutations provide a long sought means for the measurement of real-time, live cell cAMP responses in experimental models wherein the biological substrate is represented by cells that are few in number and costly to produce, such as primary cell cultures. Primary cells are a highly valuable biological substrate for they are the closest model to *in vivo* physiological conditions and offer the best environment for the study of a number of critical pathways involved in physiologically relevant processes, such as cell differentiation, diseases, and cancer. However, due to their limited life span *in vitro*, they need to be originated frequently from animal organs. The limited time frame within which primary cells can be used together with the known resistance that these cells exhibit toward conventional transfection procedures, requires the transient transfection/infection of the gene of interest (in this case, CNG) to be maximally effective. The new mutations offer an advantage in this respect. Thus, relevant primary cells may be engineered to express CNG for the study of live cell cAMP responses mediated by endogenous receptors signaling through cAMP as well as for the study of real time, live cell crosstalk between second messenger pathways (defects in receptor signaling and transduction pathway interplays could be affected in a disease state only reproducible in primary cells).

The use of primary cells as biological substrates provides for the possibility to run cell imaging-based assays with a limited number of cells, without loss of sensitivity and statistical significance. Compared to whole-well plate reader-based assays where a high number of cells are needed to generate a detectable signal (*e.g*., >50,000 cells in a single well of a 96-well plate), cell imaging requires a much smaller number of cells (<5,000). This is because (a) cell imagers include more sensitive excitation/emission devices than those found in standard plate readers and thus single cell-generated signals are readily detectable, and (b) each and everyone of the cells within the imaging field is considered as a separate entity thus providing a statistically significant pool for data analysis (for example: up to 200-300 cells can be contained in a 20x microscopic field being measured).

With the improved sensitivity of the new CNG cell-line and assay reagents, it should be possible to work with fewer and fewer cells and still generate sufficient signal for visualization. Use of 1536 well plates using the new constructs will further enable this assay format. Microfluidic devices may be used that allow cells to be perfused with an agonist/antagonist/allosteric modulator in a format that generates fluorescence output that is, in turn, read by a fluorescence reader configured to the microfluidic format. Similarly, it should be possible to visualize localized activation of cells harboring specific GPCRs in a lawn of cells arrayed on a solid substrate that is compatible with fluorescence imaging.

The assays of the present invention also provide mutiplexing potential where other dyes are used to monitor different parameters. Optical compatibility among the probes is the only limitation in combining multiple fluorescent probes. Useful combinations could include probes for cytotoxicity detection and apoptosis induction. A variety of probes with various fluorescence properties are available for this purpose. Multiplexing CNG-based GPRC screens with assays that identify in the very early phase of drug discovery unwanted effects of the candidate drug has become a highly desirable approach to accelerate the finding and profiling of potentially successful drugs.

### GPCR/CNG Assays

In these assays the GPCRs may be endogenous to the cells or may be exogenously supplied. In addition, endogenous or exogenously supplied G proteins, including promiscuous G proteins, may be used in the assays and methods of the invention.

In some embodiments, the present invention provides a host cell that contains a first nucleic acid comprising a first promoter operably linked to a first polynucleotide wherein the polynucleotide comprises a sequence encoding a G protein-coupled receptor (GPCR) protein and a second nucleic acid comprising a promoter operably linked to a second polynucleotide wherein the second polynucleotide comprises a sequence encoding a cyclic nucleotide-gated (CNG) channel according to the present claims. In some embodiments, the GPCR and/or the CNG channel is not normally expressed in the cell. The nucleic acids may be part of one molecule or may be parts of different molecules. The nucleic acids may be provided to the cell in any formulation known to those skilled in the art, for example, one or both of the nucleic acids may be part of a virus and/or plasmid and/or may be expressed from the genome of the cell.

In some embodiments, it may be desirable to utilize or create a cell line that expresses one or more of the molecules from the genome of the cell. The creation of stable cell lines for the expression of proteins is within the capability of one ordinarily skilled in the art. Some embodiments of the present invention may include expressing one protein from the genome of the cell and the other from an exogenous nucleic acid, preferably a virus or a plasmid. Cells of the present invention may be any kind of cell but are preferably eukaryotic cells such as mammalian cells. Examples of cells suitable for the practice of the present invention include, but are not limited to, BHK cells, mouse L cells, Jurkat cells, 153DG44 cells, HEK cells, CHO cells, PC12 cells, human T-lymphocyte cells and Cos-7 cells.

The CNG channels used in the present invention are mutated to make them more responsive to cAMP. The channels comprise one or more mutations that make the channels more sensitive to cAMP than a channel that does not comprise the mutations. Channels that comprise two or more mutations that make the channel more sensitive to cAMP than a channel that does not comprise the mutations are also included in the present invention. Channels that comprise three or more mutations that make the channel more sensitive to cAMP than a channel that does not comprise the mutations are also included in the present invention.

In some embodiments, the CNG channels used in the present invention may be responsive to cGMP. In other embodiments, the CNG channels used in the present invention may be responsive to analog or derivative cyclic purine monophosphates (cPuMP) or cyclic nucleotide monophosphates (cNMP). In still other embodiments, a CNG channel used in the present invention may be responsive to only one of cAMP, cGMP, an analog or derivative cPuMP or a cNMP. In a preferred embodiment, a CNG channel used in the present invention may be responsive to at least one of cAMP, cGMP, an analog or derivative cPuMP, or a cNMP. In yet another preferred embodiment, a CNG channel used in the present invention may be responsive to two or more of cAMP, cGMP, an analog or derivative cPuMP, or a cNMP.

The nucleic acid molecules encoding GPCRs according to the present invention may encode a full length wildtype G protein-coupled receptor or may encode a mutant GPCR. Some preferred mutants include N- and C-terminal truncations and insertion and/or deletion mutants. Other preferred mutants may have at least one conservative or non-conservative amino acid base substitution. Still other preferred mutants may have a combination of mutations, comprising at least two selected from the group consisting of N-terminal truncations, C-terminal truncations, insertions, deletions, conservative amino acid base substitutions and non-conservative amino acid base substitutions. A mutant GPCR is suitable for use in the present invention if it is capable of inducing a GPCR-mediated activity when contacted with an agonist.

In some embodiments, cells of the present invention may contain a third nucleic acid comprising a third promoter operably linked to a third polynucleotide wherein the third polynucleotide comprises a sequence encoding a G protein. The G protein may be a promiscuous G protein. The G protein may be normally expressed in the cell but may be expressed at a higher level when the cell contains the third nucleic acid. Alternatively, the G protein may not be naturally expressed in the cell.

In some embodiments of the invention, the G protein-coupled receptor is substantially coupled to at least one G protein selected from the group consisting of Gαₛ, Gαᵢ, Gα₁₆ or Gα_{q}. and promiscuous G proteins. Alternatively, the G protein-coupled receptor may be substantially coupled to a hybrid G protein, such as Gα_{qs}, for example.

It has been shown that the C-terminal 4-5 amino acids of Gα proteins encodes the domain mediating interaction with the receptor (Conklin et al 1993. Nature 363:274-276). Chimera Gas proteins in which the C-terminus of Gαi proteins replaces that of a Gas (Gα s/i) have been shown to couple to Gi receptors, and stimulate the activity of adenylyl cyclase (Komatsuzaki et al., 1997. FEBS Letters 406:165-170). The use of certain chimeric Gα proteins in screening orphan G-protein coupled receptors is known in the art. In particular, assays are available that utilize chimeric Gαqs to direct the coupling of a Gs receptor through a Gq pathway, allowing a Gs receptor to be monitored by release of intracellular calcium. Similar assays exist for converting Gi receptors to Gq using chimeric Gαqi. A major limitation to the use of chimeric Gαq is that such assays provide only transient release of calcium. This restricts the throughput potential, and limits the extent of miniaturization possible. The use of Gαsi chimeras for screening GPCRs is also known. In radioimmunoassays, increases in cAMP levels can be detected, however these show relatively low signal to noise ratios. Both radioimmunoassays and transcription reporter assays often required prolonged incubation with ligand. (Drew et. al., 2002. BBA 1592:185-192).

In contrast, the current invention enables efficient use of Gαsi chimeras. In particular, the current invention provides a prolonged signal that allows for either kinetic or end point assays. In a preferred embodiment, the described invention is practiced in a multi-well plate. Standard formats include 96 well, 384 wells or 1536 wells. The disclosed invention, using intact live cells and examining cAMP levels at a single-cell level, is particularly suited for 1536 well formats. Said assays can be miniaturized to plates containing at least 1536 wells, thereby substantially reducing reagent cost, the number of cells necessary to perform the assay, and increases the throughput speed.

In standard cAMP assays, cells are lysed and total cAMP is measured by antibodies, or any of a number of other methods known well to those skilled in the art. The signal and dose-response characteristics of lysed-cell assays are dependent on cell number in each well. The assay sensitivity decreases with decreasing cell number. In contrast, because the current invention measures cAMP levels within single live cells, the signal (F/Fb, fluorescence fold-change) and dose-response characteristics vary little with decreasing cell number, providing detector settings are optimized. Intracellular concentration of free cAMP is independent of cell number in the CNG channel-based cAMP assay. This capability has substantial value, particularly when the invention is used in screening of libraries of pharmaceutical compounds, as it substantially increases the throughput, and decreases the amount of materials required for each assay (cost). This is particularly true when the cells being examined are difficult to obtain, such as in primary cells or tissue.

It will be apparent to those skilled in the art that it is of great utility and value that the current invention enables further reduction in the number of cells being examined, down to the single cell, and it is envisioned that screening formats with larger numbers of wells, including volumes permitting at least one cell per well, are possible. Further, the cells need not be confined to wells, rather arrays of at least one cell per feature, are envisioned. Consequently, screening formats are envisioned wherein arrays comprising hundreds, thousands, or tens of thousands of features, each feature comprising at least one cell, wherein the at least one cell expresses at least one endogeneous or heterologous receptor, and wherein the receptors expressed at each feature can be the same or different.

Moreover, the cells need not be arrayed. It is further envisioned that single cells positioned randomly, wherein the at least one cell expresses at least one endogeneous or heterologous receptor, and wherein the receptors expressed at each feature can be the same or different, are identified as having altered cAMP levels as a result of a perturbation. In certain instances, it is sufficient to know that at least one cell, in a mixture of cells, is responding to a particular perturbation. As used herein, the term "perturbation" refers to any environmental change that can alter the biological activity of a cell. Exemplary perturbations include, but are not limited to, any combination of one or more of chemical, biological, mechanical, thermal (e.g., heat shock, and the like), electromagnetic, gravitational, nuclear, or temporal factors, for example. It will be appreciated that the format in which the cells are assayed by the present invention is flexible regardless of the type of perturbation. In particular, for screening compounds in the pharmaceutical industry, any of the described formats can be used for agonists, antagonists, allosteric modulators, partial agonists, partial antagonists and the like.

In cases where the identity of a receptor in a responding cell within a population of nonresponding cells is of interest, the responding cells can be examined directly for the identity of the receptor using antibodies, PCR or other methods known to those in the art, or the cells can be isolated, using lasers for example, further propagated and characterized for the identity of the responding receptor. The assays of the invention are suitable for characterizing any unknown proteins that participate in the signaling pathway of interest, providing means for deorphanizing G-protein linkages and identifying cyclases that are activated or inhibited by particular G proteins, for example. For instance, using RNA interference and specifically small interfering RNAs (siRNAs) corresponding to candidate G proteins and cyclases, the different G proteins and cyclases may be selectively inhibited within cells during the assay, thereby permitting one to characterize the effects of up or down regulation of these and other players, on cAMP production.

Further still, the present invention allows for detection of either cation influx (e.g., calcium) or membrane potential changes, providing methods of monitoring that are compatible with existing pharmaceutical screens. Therefore, in a preferred embodiment, among others, the present invention enables the high-throughput screening of cAMP concentration changes in cells that also express a chimeric G alpha protein. Such chimeric alpha proteins can include, but are not limited to, Gαsi proteins. In another preferred embodiment, at least one of the chimeric alpha, and the CNG of the current invention is stably integrated into the chromosome of the host cell. Said host cell expressing at least one of an endogeneous and a heterologous GPCR. In yet another preferred embodiment, the chimeric alpha protein is covalently linked to the GPCR.

In another aspect, the present invention provides a method of detecting activity of a GPCR by expressing the GPCR in a cell-optionally from an exogenous or heterologous GPCR-encoding nucleic acid molecule-expressing a cyclic nucleotide-gated channel that comprises one or more mutations that make the channel more sensitive to cAMP according to the present claims; and measuring activity of the channel wherein activity of the channel indicates activity of the GPCR. The CNG channel may be expressed from an exogenous nucleic acid or from the genome of the cell.

In some embodiments, measuring may entail the use of a dye, for example, a fluorescent dye that can be detected by fluorescence imaging systems. Some preferred dyes include, but are not limited to, Ca²⁺ sensitive dyes and voltage sensitive dyes, in formats involving small organic molecules and genetic encoded protein molecules. In some embodiments, measuring may entail determination of activation of CNG channel activity in a single cell. This may be accomplished using any means known to persons skilled in the art such as by fluorescence detection using a microscope or FACS (Fluorescence Activated Cell Sorting). When a microscope is used it may be desirable to couple the microscope to a computer system. The computer system may be used to track individual cells and perform statistical analysis.

Non-dye indicators may also be employed in any of the assays described herein. For example, GFP-based indicators exist for measuring membrane potential. Aequroin proteins produce light as an indication of calcium influx. Other indicators are known and available for measuring other cations, such as sodium and potassium. Accordingly, the present invention may be performed using any appropriate indicator substance, including, for example, fluorescent and luminescent indicators.

In some embodiments, the method may be configured to be conducted in a multiwell plate-96 well, 384 well etc.-and measuring may be performed with a multiwell microplate reader. Examples of suitable readers include those that are fluorometric-based readers with a CCD camera and fluorometric-based scanning microplate readers.

In some embodiments, it may be desirable to attach the cells to a solid surface before, during or after performing the methods of the invention. Suitable solid surfaces include, but are not limited to, slides and multiwell plates.

In some instances it may be desirable to increase the sensitivity of the methods of the invention. This may be accomplished by, for example, pretreating the cells with a cAMP analogue before measuring. Suitable analogues include caged photoactivatable analogues.

The methods of the invention may be practiced with cells expressing a promiscuous G protein. The promiscuous G protein may be expressed from the genome of the cell and/or may be expressed from an exogenous nucleic acid.

In some embodiments of the invention, the GPCR-mediated activity to be measured may be ion flux. In these cases, ion flux may be measured by any method known to those skilled in the art including, but not limited to, by determining a change in spectral characteristic of a dye or by patch clamp.

In another aspect, the present invention provides a method of identifying a ligand for a receptor by contacting a cell with a compound wherein the cell expresses the receptor and at least one cyclic nucleotide-gated (CNG) channel according to the present claims and measuring activation of the CNG channel, wherein activation of the CNG channel indicates that the compound is a ligand for the receptor. In some embodiments, the receptor may not be endogenous to the cell. The CNG and/or the GPCR channel may be expressed from an exogenous nucleic acid and/or from the genome of the cell. Measuring of the CNG channel activation may be by any means known to those skilled in the art including , but not limited to, by the use of a dye. An example of a suitable dye is a fluorescent dye that can be detected by imaging systems. Preferably a dye may be a Ca²⁺ sensitive dye and/or a voltage sensitive dye.

The methods of identifying a ligand may be used on a single cell by measuring activation of CNG channel activity in a single cell. Methods of this type may employ the use of fluorescence detection using a microscope. When a microscope is used, it may be desirable to couple the microscope to a computer system. The computer system may be used to track individual cells and perform statistical analysis.

The methods of identifying a ligand may be used in a multiwell-96 well, 384 well, 1536 well, etc-format and measuring may be performed with a microplate reader. A suitable reader may be a fluorometric-based reader with a CCD camera and/or a fluorometric-based scanning microplate reader.

The methods of identifying a ligand may be used with cells attached to a solid surface. The cells may be attached before, during or after performing one or more of the method steps. Suitable solid surfaces include, but are not limited to, slides and multiwell plates.

The methods of the invention may also be performed on non-adherent cells, for instance using fluorescence activated cell sorting (FACS) analysis. Protocols using FACS to monitor [Ca²⁺]i and membrane potential of cells are well established and can be easily adopted to monitor CNG channel activity *(*Lazzar et al., 1986, J Biol Chem, 261:9710-9713; Shapiro, 2000, Methods, 21:271-279).

The methods of identifying a ligand may be used with cells that have been pretreated with a cAMP analogue before being contacted with the ligand, for example, with a caged, photoactivatable analogue.

The methods of identifying a ligand may be used with one or more cells that express a promiscuous G protein.

In some embodiments, the methods of identifying a ligand may include a measuring step that comprises determining ion flux. Ion flux may be determined by any means known to those skilled in the art such as by a change in spectral characteristic of a dye and/or by patch clamp.

In another aspect, the present invention provides a method of identifying an agent that modulates an activity mediated by a GPC receptor by contacting a cell with the agent and a ligand for the receptor wherein the cell expresses the receptor and at least one cyclic nucleotide-gated (CNG) channel according to the present claims, and measuring activation of the CNG channel. In some embodiments, it may be desirable to compare activation of the CNG channel in the presence of the agent to activation of the channel in the absence of the agent. Typically, a difference in activation of the CNG channel indicates the agent modulates the activity. The CNG channel may be expressed from an exogenous nucleic acid and/or from the genome of the cell. Measuring the activation of the CNG channel may entail the use of a dye. An example of a suitable dye is one that is a fluorescent dye that can be detected by UV-based imaging systems. Dyes may be Ca²⁺ sensitive dyes and/or voltage sensitive dyes. Dyes of the present invention may be added exogenously to the cells either before or during the assay. Alternatively, dyes of the present invention may be expressed exogenously by the cells as probes. Said probes may be introduced into said cells for transient expression or for stable expression.

The methods of identifying an agent that modulates an activity mediated by a GPC receptor may be practiced on a single cell by determination of activation of CNG channel activity in a single cell. Methods of making such a determination are known to those skilled in the art and include by UV-based fluorescence using a microscope. When a microscope is used it may be coupled to a computer system. The computer system may be one that tracks individual cells and performs statistical analysis.

The methods of identifying an agent that modulates an activity mediated by a GPC receptor may be configured to use a multiwell-96 well, 384 well etc-format. Configurations of this type may employ a multiwell microplate reader, for example, a fluorometric-based reader with a CCD camera and/or a fluorometric-based scanning microplate reader.

The methods of identifying an agent that modulates an activity mediated by a GPC receptor may be practiced on cells attached to a solid support. The cells may be attached before, during or after performing one or more method steps. Suitable solid supports include slides and multiwell plates.

The methods of identifying an agent that modulates an activity mediated by a GPC receptor may be performed using cells pretreated with a cyclic nucleotide analogue. Suitable analogues include caged, photoactivatable analogues.

Any of the methods of identifying an agent that modulates an activity mediated by a GPC receptor may be practiced using cells that express a promiscuous G protein.

The present invention further provides kits adapted to perform the methods of the invention. Such kits will typically include one or more cells of the invention in a suitable container. Kits may optionally comprise one or more reagents such as buffers and/or salts and/or dyes. When dyes are included, they will typically be voltage sensitive dyes and/or Ca²⁺ sensitive dyes.

### A. Definitions

In the description that follows, numerous terms and phrases known to those skilled in the art are used. In the interest of clarity and consistency of interpretation, the definitions of certain terms and phrases are provided.

As used herein, "fluorescence quenching" refers to any process which decreases the fluorescence intensity of a sample. General teaching can be found, *e.g*., in "Principles of Fluorescence Spectroscopy" (Joseph R. Lakowicz, 1983, 1st edition, Kluwer Academic/Plenum Publishers, New York). Several ways have been described to quench fluorescence as follows:
1. Collisional quenching involving collisions with other molecules that result in the loss of excitation energy as heat instead of as emitted light. This process is always present to some extent in solution samples; species that are particularly efficient in inducing the process are referred to as collisional quenchers (*e.g*. iodide ions, molecular oxygen, nitroxide radical).
2. Static quenching. Interaction of the fluorophore with the quencher forms a stable non-fluorescent complex. Since this complex typically has a different absorption spectrum from the fluorophore, the presence of an absorption change is diagnostic of this type of quenching (by comparison, collisional quenching is a transient excited state interaction and so does not affect the absorption spectrum). A special case of static quenching is self-quenching, where fluorophore and quencher are the same species. Self-quenching is particularly evident in concentrated solutions of tracer dyes.
3. Resonance energy transfer. Like collisional quenching, this is an excited state interaction but the two participating molecules do not have to collide - it can occur over distances of 100 angstroms or more. One dye (the donor) is excited by absorption of a photon, but instead of emitting a fluorescence photon, the excitation is transferred by electronic coupling to an acceptor molecule. The result of this exchange is an excited acceptor and a ground state donor. In signal terms the result is either fluorescence at longer wavelength (compared to the spectrum of the donor alone) or no fluorescence, depending on whether the acceptor is itself fluorescent or non-fluorescent.
4. Inner filter effect. This is a measurement artifact as opposed to a quenching process. It occurs in samples with very high absorbance (the absorbance can be due to the fluorophore itself or other absorbing components of the sample - it doesn't matter which). Under these conditions, all of the incident exciting light is absorbed at the front face of the sample. In a conventional 90 degree detection geometry, the excitation light cannot penetrate deeply enough to the point at which the detection optics are focused. The result is the detected fluorescence decreases as the sample absorbance increases. As implied, the magnitude of inner filter effect depends on the geometrical relationship between the excitation and emission detection paths and on the thickness of the sample. To avoid inner filter effects (and other artifacts), it is generally advisable for the sample absorbance measured at the excitation wavelength to not exceed 0.1.

As used herein, "substantially interacts" refers to the amount of an effect one molecule has on another, for example, the effect of a GPCR on a G protein. An interaction is substantial if it results in a detectable response of an amplitude capable of having a physiological effect.

As used herein, the "genome" of a cell refers to the genetic material contained on the chromosomes of the cell.

As used herein, "GPCR-mediated activity" refers to any cellular process that can be affected by signal transduction mediated by a GPCR. This phrase is seen to include, but is not limited to cyclic nucleotide production, Ca²⁺ influx, inositol triphosphate (IP₃) and diacylglycerol production and the like.

In some instances, putative GPCRs may be identified by homology to other known GPCRs. Homology or identity at the nucleotide or amino acid sequence level is determined by BLAST (Basic Local Alignment Search Tool) analysis using the algorithm employed by the programs **blastp, blastn, blastx, tblastn and tblastx** (Altschul et al., Nucleic Acids Res 25: 3389-3402, 1997 and Karlin et al., Proc Natl Acad Sci USA 87:2264-2268, 1990, both fully incorporated by reference) which are tailored for sequence similarity searching. The approach used by the **BLAST** program is first to consider similar segments, with and without gaps, between a query sequence and a database sequence, then to evaluate the statistical significance of all matches that are identified and finally to summarize only those matches which satisfy a preselected threshold of significance. For a discussion of basic issues in similarity searching of sequence databases, see Altschul et al. (Nature Genetics 6:119-129, 1994) which is fully incorporated by reference. The search parameters for **histogram, descriptions, alignments, expect** (*i.e*., the statistical significance threshold for reporting matches against database sequences), cutoff, **matrix** and **filter** (low complexity) are at the default settings. The default scoring matrix used by blastp, **blastx, tblastn,** and **tblastx** is the **BLOSUM62** matrix (Henikoff et al., Proc Natl Acad Sci USA 89:10915-10919, 1992, fully incorporated by reference), recommended for query sequences over 85 in length (nucleotide bases or amino acids).

For **blastn**, the scoring matrix is set by the ratios of **M** (*i.e.,* the reward score for a pair of matching residues) to **N** (*i.e*., the penalty score for mismatching residues), wherein the default values for **M** and **N** are +5 and -4, respectively. Four **blastn** parameters were adjusted as follows: Q=10 (gap creation penalty); R=10 (gap extension penalty); wink=1 (generates word hits at every wink^{th} position along the query); and gapw=16 (sets the window width within which gapped alignments are generated). The equivalent **Blastp** parameter settings were Q=9; R=2; wink=1; and gapw=32. A **Bestfit** comparison between sequences, available in the GCG package version 10.0, uses DNA parameters GAP=50 (gap creation penalty) and LEN=3 (gap extension penalty) and the equivalent settings in protein comparisons are GAP=8 and LEN=2.

As used herein, "stringent conditions" are those that (1) employ low ionic strength and high temperature for washing, for example, 0.015 M NaCl/0.0015 M sodium citrate/0.1% SDS at 50°C, or (2) employ during hybridization a denaturing agent such as formamide, for example, 50% (vol/vol) formamide with 0.1% bovine serum albumin/0.1% Ficoll/0.1% polyvinylpyrrolidone/50 mM sodium phosphate buffer at pH 6.5 with 750 mM NaCl, 75 mM sodium citrate at 42°C. Another example is hybridization in 50% formamide, 5× SSC (0.75 M NaCl, 0.075 M sodium citrate), 50 mM sodium phosphate (pH 6.8), 0.1% sodium pyrophosphate, 5× Denhardt's solution, sonicated salmon sperm DNA (50 µg/ml), 0.1% SDS, and 10% dextran sulfate at 42°C, with washes at 42°C in 0.2x SSC and 0.1% SDS. A skilled artisan can readily determine and vary the stringency conditions appropriately to obtain a clear and detectable hybridization signal. Preferred molecules are those that hybridize under the above conditions to the complement of SEQ ID NO: 1, 3, and which encode a functional protein. Even more preferred hybridizing molecules are those that hybridize under the above conditions to the complement strand of the open reading frame of SEQ ID NO: 1, 3.

As used herein, a nucleic acid molecule is said to be "isolated" when the nucleic acid molecule is substantially separated from contaminant nucleic acid molecules encoding other polypeptides.

The present invention further provides fragments of the encoding nucleic acid molecule. As used herein, a fragment of an encoding nucleic acid molecule refers to a small portion of the entire protein coding sequence. The size of the fragment will be determined by the intended use. For example, if the fragment is chosen so as to encode an active portion of the protein, the fragment will need to be large enough to encode the functional region(s) of the protein. For instance, fragments that encode peptides corresponding to predicted antigenic regions may be prepared. If the fragment is to be used as a nucleic acid probe or PCR primer, then the fragment length is chosen so as to obtain a relatively small number of false positives during probing/priming.

Fragments of the encoding nucleic acid molecules of the present invention (*i.e*., synthetic oligonucleotides) that are used as probes or specific primers for the polymerase chain reaction (PCR), or to synthesize gene sequences encoding proteins of the invention, can easily be synthesized by chemical techniques, for example, the phosphotriester method of Matteucci et al. (JAm Chem Soc 103, 3185-3191, 1981) or using automated synthesis methods. In addition, larger DNA segments can readily be prepared by well-known methods, such as synthesis of a group of oligonucleotides that define various modular segments of the gene, followed by ligation of oligonucleotides to build the complete modified gene.

Cyclic nucleotide-gated (activated) ion channels are most well known for mediating visual and olfactory signal transductions, but they are also expressed in other cell types and tissues. In native tissues, these channels are heteromultimers, with different heteromers showing distinct nucleotide sensitivity, ion conductance (selectivity), and Ca²⁺ modulation. Molecular cloning and genome sequencing efforts have revealed the presence of six genes coding for subunits of cyclic nucleotide-gated channels in human and mouse. The adopted nomenclature for these channel subunits recognizes two phylogenetically distinct subfamilies, CNGA and CNGB, defined by their sequence relationships. The CNGA subfamily comprises CNGA1 (CNG1/CNGα1/RCNC1); CNGA2 (CNG2/CNGα3/OCNC1); CNGA3 (CNG3/CNGα2/CCNC1); CNGA4 (CNG5/CNGα4/OCNC2/CNGB2). In the CNGB subfamily, the member expressed in rod photoreceptors, olfactory neurons and other tissues is designated CNGB1 (CNG4/CNGβ1/RCNC2 and a splice variant CNG4.3), whereas that found in cone photoreceptors and possibly other tissues is CNGB3 (CNG6/CNGβ2/CCNC2) (Bradley, J. et al., Nomenclature for ion channel subunits. Science. 2001 Dec 7;294:2095-6). It will be appreciated by those skilled in the art that channels derived from other organisms can be placed into the described subfamilies based on homology, and such channels are anticipated as applicable to the present invention.

As used herein, "mutant" or "mutated" CNG channels are those comprising subunits that have an altered amino acid sequence. Alterations of the amino acid sequence may include, but are not limited to, N-terminal truncations, C-terminal truncations, amino acid residue deletions or additions, conservative or non-conservative amino acid residue substitutions. A mutant CNG channel subunit may comprise one or more, two or more or three or more alterations to its amino acid sequence. A mutant CNG channel may be heteromeric, being composed of at least two different subunit types, such as α and β. A mutant CNG channel may be heteromeric in comprising subunits that have different mutations. A mutant CNG channel may be heteromeric in comprising at least one mutant subunit and at least one wildtype subunit. A mutant CNG channel may also be a heteromer comprising subunits corresponding to all of the wildtype subunits it is normally composed of when expressed in its native cell source, wherein at least one of those subunits comprises at least one alteration of its amino acid sequence. A mutant CNG channel may be composed of subunits derived from the same species as the recombinant cell transformed to express the CNG channel. A mutant CNG channel may be composed of subunits derived from a different species as the recombinant cell transformed to express the CNG channel. A mutant CNG channel may be composed of subunits derived different species. Mutant CNG channels may comprise subunits derived from any species, including, but not limited to, rat, murine, human, bovine, canine, feline, any other mammal or vertebrate, Drosophila and other insects, and C. *elegans*, for example.

As used herein, "wildtype" CNG channels are those composed of subunits that have not had mutations made to the amino acid sequence of those subunits as isolated from natural sources or subunits with mutations as compared to the subunit isolated from natural sources, wherein the mutations do not substantially alter channel function or activity. A wildtype CNG channel is preferably heteromeric, being composed of at least two different subunit types, such as α and β. A wildtype CNG channel may also include, in some preferred embodiments, a third different subunit. A wildtype CNG channel may also be a heteromer comprising all of the subunits it is normally composed of when expressed in its native cell source. A wildtype CNG channel may be composed of subunits derived from the same species as the recombinant cell transformed to express the CNG channel. A wildtype CNG channel may be composed of subunits derived from a different species as the recombinant cell transformed to express the CNG channel. A wildtype CNG channel may be composed of subunits derived different species. Wildtype CNG channels may also comprise subunits derived from any species, including, but not limited to, rat, murine, human, bovine, canine, feline, any other mammal or vertebrate, Drosophila and other insects, and *C*. *elegans*, for example. CNG channel subunits of the present invention may be present on a single or on multiple vectors for introduction into a host cell. For example, in the case of a wildtype α/β heteromeric CNG channel, the coding sequences for the α and β subunits may be contained on a single vector which is introduced into the host cell, or they may on separate vectors which are introduced into the host cell either separately or at the same time.

As used herein, "voltage sensitive dyes" or "membrane potential dyes" include those dyes that enter depolarized cells, bind to intracellular proteins or membranes and exhibit enhanced fluorescence. Voltage sensitive dyes include, but are not limited to, carbocyanine, rhodamine, oxonols, and merocyanine bis-barbituric acid oxonols. Voltage sensitive and membrane potential dyes also include probes which are encoded by nucleic acid sequences that can be incorporated into a vector for expression by a host cell.

As used herein, "calcium-sensitive dyes" include those dyes which exhibit enhanced fluorescence in response to increased levels of intracellular calcium. Calcium-sensitive dyes include, but are not limited to, Fura-2, Fluo-3, Fluo-4, and Calcium Green-1. Calcium-sensitive dyes also include probes which are encoded by nucleic acid sequences that can be incorporated into a vector for expression by a host cell and include, but are not limited to, Aeuorin (Euroscreen) and green fluorescent protein (GFP)-based calcium sensors such as Cameleon, for example.

### B. Techniques

The present invention further provides recombinant DNA molecules (rDNAs) that contain a coding sequence. Preferred coding sequences are those that encode mutant forms of CNG channels according to the claims. As used herein, a rDNA molecule is a DNA molecule that has been subjected to molecular manipulation *in situ.* Methods for generating rDNA molecules are well known in the art, for example, see Sambrook et al., (Molecular Cloning - A Laboratory Manual, Cold Spring Harbor Laboratory Press, Cold Spring Harbor, NY, 1989). In the preferred rDNA molecules, a coding DNA sequence is operably linked to expression control sequences and/or vector sequences.

The choice of vector and/or expression control sequences to which one of the protein encoding sequences of the present invention is operably linked depends directly, as is well known in the art, on the functional properties desired, *e.g*., protein expression, and the host cell to be transformed. A vector contemplated by the present invention is at least capable of directing the replication or insertion into the host chromosome, and preferably also expression, of the structural gene included in the rDNA molecule.

Expression control elements that are used for regulating the expression of an operably linked protein encoding sequence are known in the art and include, but are not limited to, inducible promoters, constitutive promoters, secretion signals, and other regulatory elements. Preferably, the inducible promoter is readily controlled, such as being responsive to a nutrient in the host cell's medium.

Expression vectors compatible with eukaryotic cells, preferably those compatible with mammalian cells, can be used to form rDNA molecules that contain a coding sequence. Eukaryotic cell expression vectors, including but not limited to viral vectors and plasmids, are well known in the art and are available from several commercial sources. Typically, such vectors are provided containing convenient restriction sites for insertion of the desired DNA segment. Typical of such vectors are pSVL and pKSV-10 (Pharmacia), pBPV-1/pML2d (International Biotechnologies, Inc.), pTDT1 (ATCC, #31255), and the like eukaryotic expression vectors.

Eukaryotic cell expression vectors used to construct the rDNA molecules used in the present invention may further include a selectable marker that is effective in an eukaryotic cell, preferably a drug resistance selection marker. An example of a drug resistance marker is the gene whose expression results in neomycin resistance, *i.e*., the neomycin phosphotransferase (neo) gene (Southern et al., J Mol Anal Genet 1:327-341, 1982). Alternatively, the selectable marker can be present on a separate plasmid, and the two vectors are introduced by co-transfection of the host cell, and selected by culturing in the appropriate drug for the selectable marker.

The nucleic acid molecule is then preferably placed in operable linkage with suitable control sequences, as described above, to form an expression unit containing the protein open reading frame. The expression unit is used to transform a suitable host and the transformed host is cultured under conditions that allow the production of the recombinant protein.

Various mammalian cell culture systems can also be employed to express recombinant protein. Examples of mammalian expression systems include the COS-7 lines of monkey kidney fibroblasts, described by Gluzman (Cell 23:175, 1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements. Each of the foregoing steps can be done in a variety of ways. For example, the desired coding sequences may be obtained from genomic fragments and used directly in appropriate hosts. The construction of expression vectors that are operable in a variety of hosts is accomplished using appropriate replicons and control sequences, as set forth above. The control sequences, expression vectors, and transformation methods are dependent on the type of host cell used to express the gene and were discussed in detail earlier. Suitable restriction sites can, if not normally available, be added to the ends of the coding sequence so as to provide an excisable gene to insert into these vectors.

Other variants on expression vectors include fusion proteins between the gene of interest and other polypeptides. Applications include but are not limited to means of visualization (such as green fluorescent protein, GFP, and variants) or for protein purification (such as polyhistidine, or glutathione-S-transferase, GST).

Specifically contemplated are genomic DNA, cDNA, mRNA and antisense molecules, as well as nucleic acids based on alternative backbones or including alternative bases whether derived from natural sources or synthesized. Such nucleic acids, however, are defined further as being novel and unobvious over any prior art nucleic acid including that which encodes, hybridizes under appropriate stringency conditions, or is complementary to a nucleic acid encoding a protein according to the present invention.

The encoding nucleic acid molecules of the present invention may further be modified so as to contain a detectable label for diagnostic and probe purposes. A variety of such labels are known in the art and can readily be employed with the encoding molecules herein described. Suitable labels include, but are not limited to, biotin, radiolabeled nucleotides and the like. A skilled artisan can readily employ any such label to obtain labeled variants of the nucleic acid molecules of the invention.

Modifications to the primary structure of the nucleic acid molecules by deletion, addition, or alteration of the nucleotide sequence can be made without destroying the activity of the encoded proteins. Such substitutions or other alterations result in proteins having an amino acid sequence falling within the contemplated scope of the present invention.

### II. Specific Embodiments

### A. G Protein Coupled Receptors

At present, there are about 400 GPCR genes that can be identified from genomic databases, excluding odorant and taste receptors. Ligands for about 200 of these have been identified, leaving the rest as "orphan receptors". The key to uncover the potential of therapeutic benefits of agonists and/or antagonists of these orphan GPCRs is in the ability to identify the natural biological ligands for them to elucidate their biological functions and disease associations. For GPCRs whose ligand is known, the identification of agents that modulate a GPCR-mediated activity allows the development of pharmaceuticals with high affinity and desirable functionality against these receptors for the evaluation of their clinical potential (for a review, see, Debouck and Metcalf, 2000, Annu. Rev. Pharmacol. Toxicol. 40:193-208; Howard et al., 2001, Trends Pharmacol. Sci. 22:132-140).

### B. Cyclic Nucleotide-Gated Channels

Cyclic nucleotide-gated (CNG) channels of vertebrates are cation channels controlled by the cytosolic concentration of cGMP and cAMP (for reviews, see Kaupp, 1995, Curr. Opin. Neurobiol. 5:434-442; Finn et al., 1996, Annu. Rev. Physio. 58:395-426; Zogotta and Siegelbaum, 1996, Annu. Rev. Neurosci. 19:235-263; Li et al., 1997, Q. Rev. Biophys. 30:177-193). These channels conduct cation currents, carried by mixed ions-Na+, K+ and Ca²⁺-and serve to couple both electrical excitation and Ca²⁺ signaling to changes of intracellular cyclic nucleotide concentration. In vertebrate photoreceptors and olfactory sensory receptors, CNG channels depolarize the membrane voltage and determine the activity of a number of Ca²⁺-regulated proteins involved in cell excitation and adaptation (for reviews, see Kaupp and Koch, 1992, Annu. Rev. Physiol. 54:153-175; Koch, 1995, Cell Calcium 18:314-321).

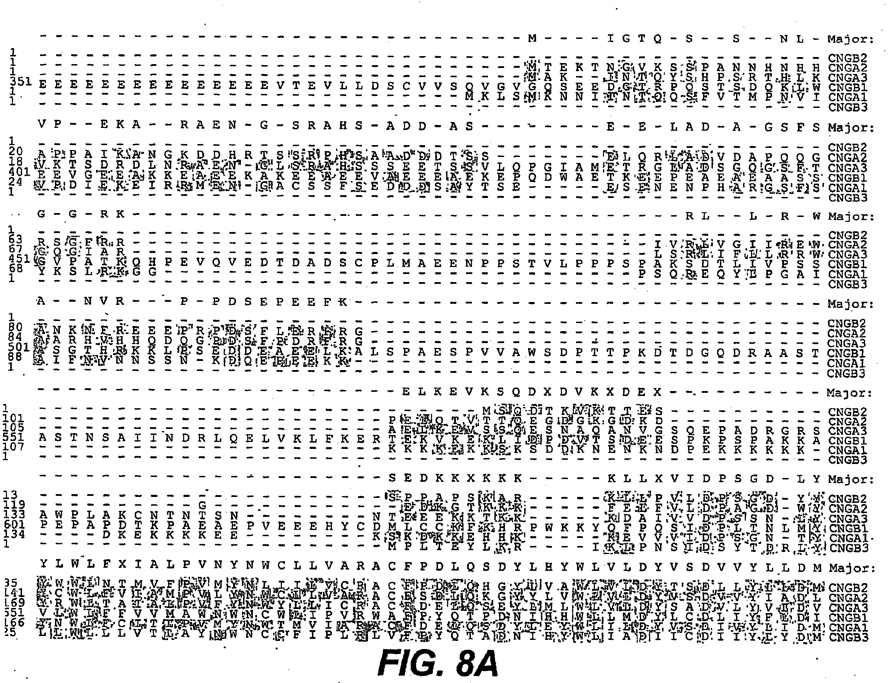

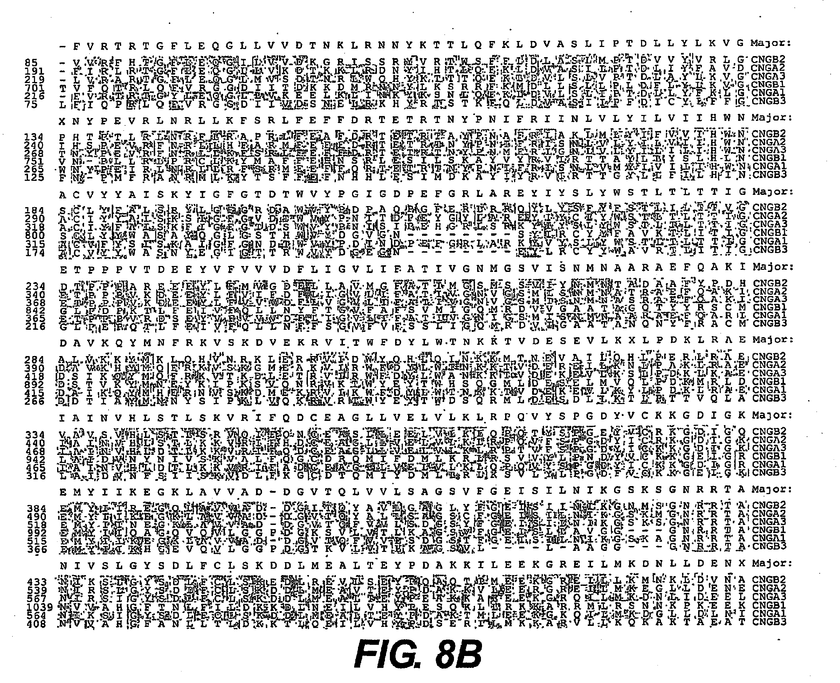

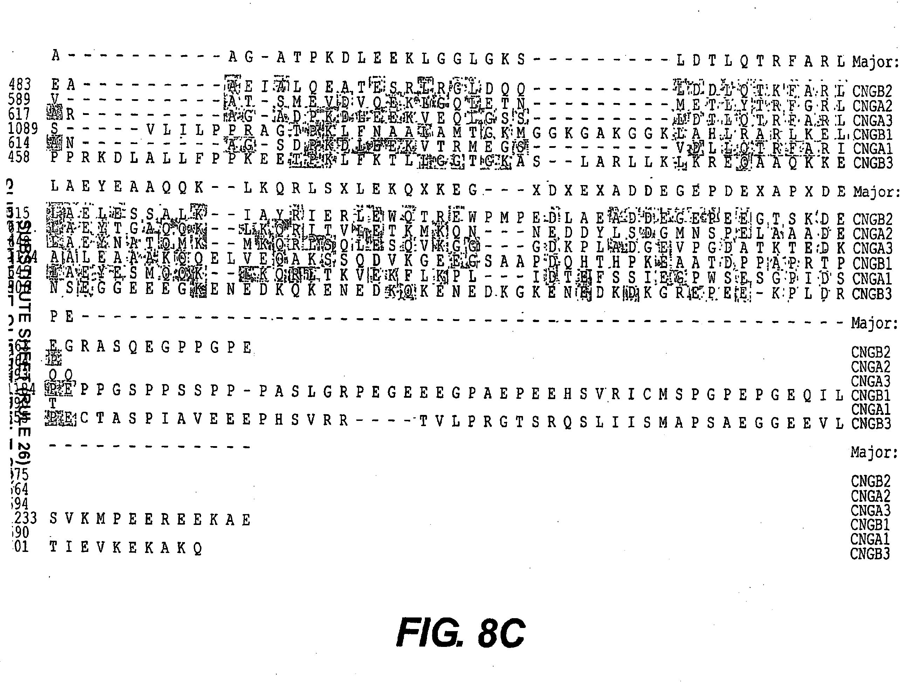

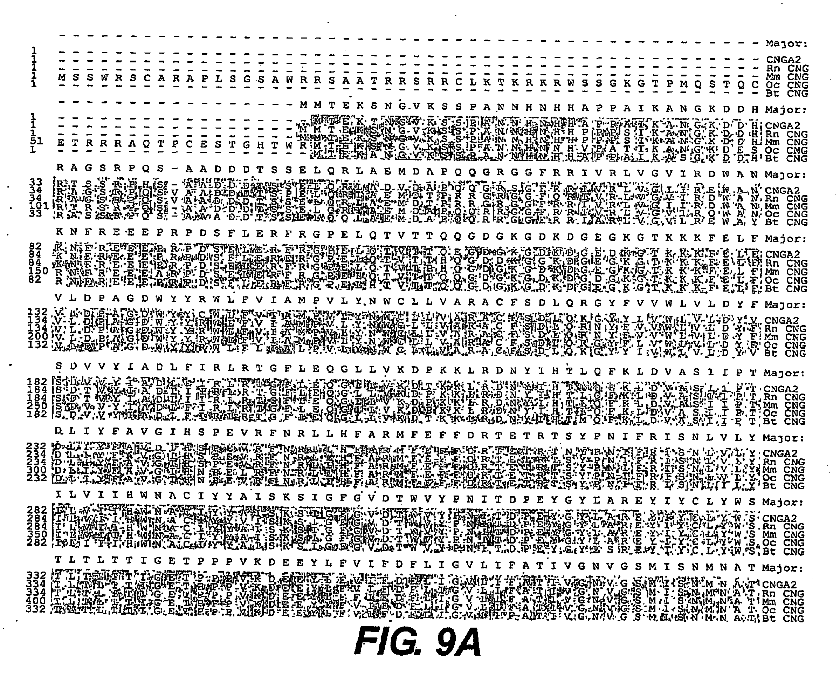

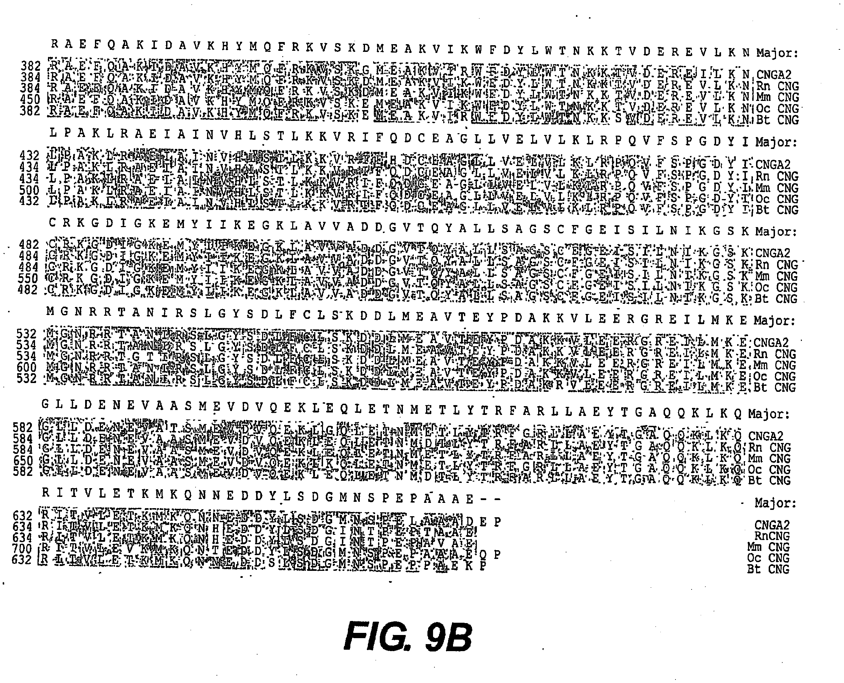

CNG channels are typically heteromultimers containing homologous α and β subunits. Some CNG channels also have a third subunit as well. For example, a third subunit has been described for the rat olfactory CNG channel (GenBank Acc. No. AF068572). Although they are members of the voltage gated channel superfamily, they are not voltage sensitive, instead responding to changes in cyclic nucleotide concentration. Presently, six human genes have been identified that encode CNG channel subunits. An alignment of the human CNG channels is provided in Figure 8 panels A-C. An alignment of several mammalian CNG channels is provided in Figure 9 panels A and B.

CNG channel subunits typically consist of a cytoplasmic N-terminus, six membrane spanning segments and a cytoplasmic C-terminus. Between the fifth and sixth membrane spanning segments, a domain critical for pore lining-the P domain-has been identified. Various amino acid residues have been implicated in ion specificity and activation characteristics (see Gavazzo, et al., 2000, J. Gen. Phys. 116:311-15, Vamum, et al. 1995, Neuron. 15:619-625).

The rat olfactory CNG channel (CNGA2) forms cAMP-activated channels when heterologously expressed in mammalian cells with a half-maximally effective concentration (EC50) for cAMP of 68 µM (Dhallan *et al* 1990). When co-expressed with CNGB2, or CNGB2 and CNCβ1b (GenBank accession number AF068572) EC50 for cAMP is reduced to 10.3 and 4 µM (Boenigk *et al* 1999).

In the present invention, mutants of the CNG channels are used to further increase detectability of GPCR activation.

CNG channel subunits of the present invention may be present on a single or on multiple vectors for introduction into a host cell.

### C. G Proteins

Many heterotrimeric G proteins have been cloned, including more than 20 genes encoding various G alpha subunits. The various G subunits have been categorized into six families, on the basis of amino acid sequences and functional homology. These six families are termed G_{s.} Gᵢ, G_{q}, G_{olf}, Gₒ, and G₁₂. With the exception of G_{q} that results in the release of cytoplasmic Ca²⁺, all other G proteins mediate their signals through cyclic nucleotides, primarily cAMP (Watson and Arkinstall, The G-Protein-Linked Receptor Facts Book, Academic Press, London, 1994).

Certain G proteins are considered "promiscuous" G proteins because their G subunits allow them to couple with GPCRs that normally couple with G proteins of other families. For example, two members of the Gα_{q} family, human Gα₁₆ and its murine homolog Gα₁₅ are promiscuous G proteins. Although G proteins having these G subunits interact with a variety of GPCRs, they still specifically activate their downstream effector. See US pat no. 6,004,808 issued to Negulescu et al*.*

### D. Adenylyl Cyclase

3',5' cyclic adenosine monophosphate (cAMP) is the archetypal second messenger in species from bacteria to man. cAMP is produced in mammals by a family of at least nine adenylyl cyclase (AC) isozymes. The mammalian ACs differ from one another in their activation or inhibition by Ca2+/calmodulin, phosphorylation by protein kinases A and C, the inhibitory G protein α subunit (Giα) and the G protein β and γ subunits (Gβγ). All mammalian ACs are activated by the GTP-bound stimulatory G protein α subunit (Gsα) and all but AC9 are activated by the hypotensive drug forskolin. The known mammalian ACs consist of 12 transmembrane helices and two cytoplasmic catalytic domains (Hurley, J.H., Curr Opin Struct Biol. 1998 Dec;8(6):770-7).

In addition to their regulation by Gsα and forskolin, mammalian adenylyl cyclases are subjected to complex regulation by other G proteins, Ca⁺² signals, and phosphorylation. It is known that members of the Gi family (Gi; Go, Gz) that can be activated by diverse hormones and neurotransmitters (i.e., adenosine, epinephrine, and cannabinoid). On activation, the Gi family of proteins releases two potential regulators, Gα and Gβγ. Inhibition of adenylyl cyclase can be mediated through the α subunit of Gi, Go, or Gz or through Gβγ. For certain subtypes of adenylyl cyclases, such as type V and VI enzymes (predominantly expressed in heart), enzyme activity is inhibited by Giα and not altered by Gβγ.

The amount of a particular class of AC will vary between cell types. For this reason, and the above described differences in activation or inhibition, it will be appreciated that the properties of the present invention can be modified by further altering expression of at least a first adenylyl cyclase. Such alterations can include, but are not limited to, introduction of one or more heterologous ACs (both transiently expressed or integrated stably into the host genome; utilizing plasmid or viral vectors), or the up or down regulation of one or more endogeneous ACs. Methods for achieving said up or down regulation are many and known to those skilled in the art. The choice of the specific modulation is dependent upon the cell type, the G-protein linkage, the regulatory effects of various inhibitors or activators, and the means in which the present invention is to be applied.

### E. Host Cells

The present invention further provides host cells that may be transformed with a nucleic acid molecule encoding one or more of a GPCR and/or G protein and/or CNG channel. Host cells can also include cells or cell lines which have an endogenous GPCR and/or G protein which can be used for methods of the present invention in combination with recombinant CNG channel expression. Preferred cells are eukaryotic cells. Eukaryotic cells useful for practicing the present invention include, but are not limited to, mammalian cells. Any cell may be used so long as the cell line is compatible with cell culture methods and compatible with the propagation of the expression vector and expression of the gene product. Preferred eukaryotic host cells include, but are not limited to, yeast, insect and mammalian cells, preferably vertebrate cells such as those from a mouse, rat, monkey or human cell line. Preferred eukaryotic host cells include Chinese hamster ovary (CHO) cells, for example those available from the ATCC as CCL61, NIH Swiss mouse embryo cells (NIH/3T3) available from the ATCC as CRL 1658, baby hamster kidney cells (BHK), mouse L cells, Jurkat cells, SF9, Xenopus oocytes, 153DG44 cells, HEK cells, PC12 cells, human T-lymphocyte cells and Cos-7 cells, and the like eukaryotic host cells.

Transfection of appropriate cell hosts with a rDNA molecule of the present invention is accomplished by well-known methods that typically depend on the type of vector used and host system employed. With regard to transformation of vertebrate cells with vectors containing rDNAs, electroporation, cationic lipid or salt treatment methods are typically employed, see, for example, Graham et al. (Virol 52:456, 1973) and Wigler et al., (Proc Natl Acad Sci USA 76: 1373-1376, 1979). Similarly, a number of options are commercially available including from Invitrogen/Life Technologies, Promega, Qiagen, etc.

Successfully transformed cells, *i.e.,* cells that contain a rDNA molecule of the present invention, can be identified by well known techniques including the selection for a selectable marker. For example, cells resulting from the introduction of an rDNA of the present invention can be cloned to produce single colonies. Cells from those colonies can be harvested, lysed and their DNA content examined for the presence of the rDNA using a method such as that described by Southern (J Mol Biol 98:503, 1975) or Berent et al. (Biotech 3:208, 1985) or the proteins produced from the cell assayed via an immunological method.

### F. Assay Formats

The present invention provides various methods to assay for the presence and/or modulation of GPCR-mediated activity. In preferred embodiments, this may entail the detection of cAMP production by the activation of a CNG channel. In some embodiments, host cells of the present invention are assayed for the influx of Ca²⁺ as a result of their activation by cAMP produced as the result of activation of a GPCR and transduction of the signal through the intermediacy of G proteins and adenyl cyclase to the production of cAMP.

In some embodiments, cells of the present invention may be loaded with a dye that responds to the influx of Ca²⁺ with a change in one or more spectral qualities of the dye. In some embodiments, the dye binds Ca²⁺ directly resulting in an observable change in spectral quality. One example of a dye of this type is fura-2.

In other embodiments, cells may be loaded with dyes that respond to the change in membrane potential that results from the ion flux produced by the activation of the CNG channel. Dyes of this type are known to those skilled in the art (see, Zochowski, et al., 2000, Biological Bulletin 198:1-21) and are commercially available, for example, from Molecular Devices, Inc.

CNG channels were proposed as sensors for cAMP in assays aiming to detect Ca²⁺ levels with the calcium sensitive dye Fura-2 (Rich et al, 2000, J. Gen. Physiol. 116:147-161). A large number of mutants of a CNG channel alpha subunit have been identified that include C460W (Gordon et al., 1997, Neuron 19:431-441), E583M (Varnum et al., Neuron 15, 619-925), and Y565A change (Li and Lester, 1998, Mol. Pharmacol. 55:873-882). While the mutants enhanced the CNG channel's sensitivity to cAMP, the improved sensitivities are still not sufficient for use in a multiwell format. In the best case so far reported, it required 3-4 X 10⁶ cells for the elevated Ca²⁺ level in response to cAMP induction to be detected by a spectrofluorimeter (Rich et al, 2001, J. Gen. Physiol. 118:63-77). In contrast, a typical multiwell assay will involve the use of about 20-50,000 cells per well which is about 100 fold fewer cells than required for Ca²⁺ sensitive fluorescence dyes.

Voltage sensitive dyes that may be used in the assays and methods of the invention have been long used to address cellular membrane potentials (for review, see Zochowski et al., Biol. Bull. 198:1-21). Several classes of fluorescent dyes were developed that include carbocyanine, rhodamine, oxonols and merocyanine that can be obtained from Molecular Probes (Eugene, OR). The three bis-barbituric acid oxonols, often referred to as DiBAC dyes, form a family of spectrally distinct potentiometric probes with excitation maxima at approximately 490 nm (DiBAC4(3)), 530 nm (DiSBAC2(3)) and 590 nm (DiBAC4(5)). The dyes enter depolarized cells where they bind to intracellular proteins or membranes and exhibit enhanced fluorescence and red spectral shifts (Epps et al., 1994, Chem. Phys. Lipids 69:137-150). Increased depolarization results in more influx of the anionic dye and thus an increase in fluorescence. DiBAC4(3) reportedly has the highest voltage sensitivity (Brauner et al., Biochim. Biophys. Acta. 771:208-216). Similar assays were developed for membrane potential assays in high throughput platforms such as FLIPR (Molecular Devices, Sunnyvale, CA). As cAMP also induces Na+ and K+ flux in addition to Ca²⁺ changes of membrane potential as the result of Na+ and K+ flux in the presence of CNG channels can be used as the indicators of intracellular cAMP accumulation.

Detection of the alteration in the spectral characteristics of the dye may be performed by any means known to those skilled in the art. In preferred embodiments, the assays of the present invention are performed either on single cells using microscopic imaging to detect changes in spectral-i.e., fluorescent- properties or are performed in a multiwell format and spectral characteristics are determined using a microplate reader.

One suitable configuration for single cell imaging involves the use of a microscope equipped with a computer system. ATTO's Attofluor^{®} RatioVision^{®} real-time digital fluorescence analyzer from Carl Zeiss is a completely integrated work station for the analysis of fluorescent probes in living cells and prepared specimens (ATTO, Rockville, MD). Calcium can be visualized in real time. The system can observe these ions either individually or simultaneously in combinations limited only by the optical properties of the probes in use. The standard imaging system is capable of performing multiple dye experiments such as Fura-2 (for calcium) combined with GFP (for transfection) in the same cells over the same period of time. Ratio images and graphical data from multiple dyes are displayed on line.

When the assays of the invention are performed in a multiwell format, a suitable device for detecting changes in spectral qualities of the dyes used is multiwell microplate reader. Suitable devices are commercially available, for example, from Molecular Devices (FLEXstation™ microplate reader and fluid transfer system or FLIPR® system). These systems can be used with commercially available dyes such as Fluo-3, Fluo-4, and Calcium Green-1. All of these indicators excite in the visible wavelength range.

The Molecular Devices' FLIPR Fluorometric Imaging Plate Reader (Molecular Devices, Sunnyvale, CA) has been used in a high throughput screening assay to detect transient calcium release from intracellular with a calcium sensitive fluorescent dye in response to the activation of the Gq coupled subclass of receptors that activate the phopholipase signaling pathway. Promiscuous G proteins were used for other GPCRs with mixed results. Until the present invention, there was no comparable assay for cAMP that produces real-time, kinetic information on GPCR receptor activation. Furthermore, there was no easy way to directly examine cAMP accumulation in single cell activated by GPCR ligand in live cells in an imaging platform.

In some embodiments of the present invention, the cells of the invention may be treated with compounds designed to increase the intracellular level of cAMP. For example, the cell may be treated with a "caged" cAMP analogue that can be released in response to photons of light. (see Corrie, et al. Bioorganic Photochemistry vol 2 pp 243-305, Wiley and Sons, Chichester, UK, and Hagen, et al, 1996, Biochemistry 35:7762-7771)

### G. Methods to Identify Agents that Modulate GPCR-mediated Activity

An additional embodiment of the present invention provides methods for identifying agents that modulate a GPCR-mediated activity. Agents that bind to the proteins involved in the activity or that affect the expression of these proteins may or may not affect the function of said proteins. Investigation of functional effects of agents includes but is not limited to: 1) effects on ligand binding, 2) effects on G protein coupled signaling pathways, 3) activation or inhibition of receptor down regulation/desensitization.

In one embodiment of the invention, the materials and methods described may be used to identify ligands for a GPCR. This embodiment will be useful to identify ligands for "orphan receptors" *i.e*., those receptors for which a ligand has yet to be identified. Ligands may be identified by contacting a cell of the invention with a compound that is a putative ligand. The cell may be transfected with a nucleic acid that expresses a GPCR of interest and a nucleic acid that expresses a CNG channel according to the present claims. Activation of the GPCR is assayed by measuring activation of the CNG channel. For example, a cell may be loaded with a calcium sensitive dye and/or a voltage sensitive dye and changes in the spectral characteristics of the dye in the presence and absence of the putative ligand may be determined. A compound is identified as a ligand if it induces opening of the CNG channel and a concomitant change in the spectral characteristics of the dye.

In another embodiment of the invention, the ability of an agent to modulate GPCR-mediated activity by, for example, altering ligand binding, may be determined. Alteration of ligand binding may be assessed by the ability of the agent being tested to modulate the binding of a known ligand for the target GPCR. This may be accomplished using the assays described above wherein the GPCR transfected into the cell has a previously identified ligand. Alternatively, an endogenous GPCR with an identified ligand may be used. The ability of the previously identified ligand to induce activity is assayed in the presence and absence of the agent. An agent modulates a GPCR-mediated activity when the activity in the presence of the agent differs-is greater, lesser or of differing kinetic characteristics-from the activity in the absence of the agent. Standard methods of data analysis such as inhibition curves are employed to analyze effects of the agents being tested.

Alteration of activation of G protein coupled signaling pathways requires the presence of an active receptor coupled to a G protein-dependent signaling system. As an example, this may be accomplished by preparing cell lines co-transfected with the GPCR along with a promiscuous G protein such as G α16. This G protein acts as a universal adapter and, when activated by a GPCR partner, results in calcium mobilization (Marchese et al., Trends Pharmacol Sci, 20:370-375, 1999). Calcium mobilization, in turn is easily assessed by use of the assays described above. For example, a number of fluorescent intracellular calcium probes are available from Molecular Probes, Inc. Changes in intracellular calcium concentration result in changes in fluorescence intensity and/or characteristics of the probe and may be detected using a fluorescence plate reader according to the manufacturer's instructions. Confirmation that an agent affects G protein-coupled signaling by the receptor is then obtained by incubating cells in the presence of the agent of interest at a suitable concentration-typically between about 10 pM and 1 mM-and determining the resultant changes in intracellular calcium concentration. Standard dose-response curves are generated and analyzed.

### H. Uses for Agents that Modulate GPCR-mediated Activity

Agents that modulate one or more GPCR-mediated activities, such as agonists or antagonists of a GPCR, may be used to modulate processes associated with GPCR function and activity. In some embodiments, agents that modulate a GPCR-mediated activity-increase, decrease, or change the kinetic characteristics of the activity-may be used to modulate biological and pathologic processes associated with one or more GPCR-mediated activity.

As used herein, a subject can be any vertebrate, preferably a mammal, so long as the vertebrate or mammal is in need of modulation of a pathological or biological process mediated by a GPCR protein of the invention. The term "mammal" is defined as an individual belonging to the class Mammalia. The invention is particularly useful in the treatment of human subjects.

Pathological processes refer to a category of biological processes that produce a deleterious effect. For example, a particular GPCR-mediated activity or level of activity may be associated with a disease or other pathological condition. As used herein, an agent is said to modulate a pathological process when the agent reduces the degree or severity of the process. For example, a GPCR-mediated activity may be associated with a G-protein signaling disorder, such as those associated with other receptors for biogenic amines (see Background section above for examples).

The agents of the present invention can be provided alone, or in combination with other agents that modulate a particular pathological process. For example, an agent of the present invention can be administered in combination with other known drugs. As used herein, two agents are said to be administered in combination when the two agents are administered simultaneously or are administered independently in a fashion such that the agents will act at the same time.

The agents of the present invention can be administered via parenteral, subcutaneous, intravenous, intramuscular, intraperitoneal, transdermal, or buccal routes. Alternatively, or concurrently, administration may be by the oral route. The dosage administered will be dependent upon the age, health, and weight of the recipient, kind of concurrent treatment, if any, frequency of treatment, and the nature of the effect desired.

### I. Agents that Modulate GPCR-mediated Activity

Potential agents can be screened to determine if application of the agent modulates a GPCR-mediated activity. This may be useful, for example, in determining whether a particular drug is effective in treating a particular patient with a disease characterized by an aberrant GPCR-mediated activity. In the case where the activity is affected by the potential agent such that the activity returns to normal or is altered to be more like normal, the agent may be indicated in the treatment of the disease. Similarly, an agent that induces an activity that is similar to that expressed in a disease state may be contraindicated.

According to the present invention, a GPCR with an identified ligand may be used as the basis of an assay to evaluate the effects of a candidate drug or agent on a cell, for example on a diseased cell. A candidate drug or agent can be screened for the ability to modulate an activity mediated by the GPCR, for example Ca²⁺ influx.

Assays to monitor the modulation of a GPCR-mediated activity may utilize any available means of monitoring for changes in CNG activity, but is preferably accomplished using one or more of the assay formats described above.

Agents that are assayed in the above methods can be randomly selected or rationally selected or designed. As used herein, an agent is said to be randomly selected when the agent is chosen randomly without considering the specific sequences involved in the association of the a protein of the invention alone or with its associated substrates, binding partners, etc. An example of randomly selected agents is the use a chemical library or a peptide combinatorial library, or a growth broth of an organism.

As used herein, an agent is said to be rationally selected or designed when the agent is chosen on a nonrandom basis which takes into account the sequence of the target site and/or its conformation in connection with the agent's action. Agents can be rationally selected or rationally designed by utilizing the peptide sequences that make up these sites. For example, a rationally selected peptide agent can be a peptide whose amino acid sequence is identical to or a derivative of any functional consensus site.

The agents can be, as examples, peptides, small molecules, vitamin derivatives, as well as carbohydrates, lipids, oligonucleotides and covalent and noncovalent combinations thereof. Dominant negative proteins, DNA encoding these proteins, antibodies to these proteins, peptide fragments of these proteins or mimics of these proteins may be introduced into cells to affect function. "Mimic" as used herein refers to the modification of a region or several regions of a peptide molecule to provide a structure chemically different from the parent peptide but topographically and functionally similar to the parent peptide (see Grant, (1995) in Molecular Biology and Biotechnology Meyers (editor) VCH Publishers). A skilled artisan can readily recognize that there is no limit as to the structural nature of the agents.

### J. Compositions Comprising Agents that Modulate GPCR-mediated Activity

Compositions comprising the agents identified in methods of the invention can be provided alone, or in combination with other compositions and/or agents that modulate a particular pathological process. For example, an agent can be administered in combination with other known drugs. As used herein, two agents are said to be administered in combination when the two compositions and/or agents are administered simultaneously or are administered independently in a fashion such that the agents will act at the same time.

Compositions comprising the agents identified in methods of the invention can be administered via parenteral, subcutaneous, intravenous, intramuscular, intraperitoneal, transdermal, or buccal routes. Alternatively, or concurrently, administration may be by the oral route. The dosage administered will be dependent upon the age, health, and weight of the recipient, kind of concurrent treatment, if any, frequency of treatment, and the nature of the effect desired.

The present application further describes compositions containing one or more agents that modulate a GPCR-mediated activity. While individual needs vary, determination of optimal ranges of effective amounts of each component is within the skill of the art. Typical dosages comprise 0.1 to 100 µg/kg body wt. The preferred dosages comprise 0.1 to 10 µg/kg body wt. The most preferred dosages comprise 0.1 to 1 µg/kg body wt.

In addition to the pharmacologically active agent, the compositions may contain suitable pharmaceutically acceptable carriers comprising excipients and auxiliaries that facilitate processing of the active compounds into preparations which can be used pharmaceutically for delivery to the site of action. Suitable formulations for parenteral administration include aqueous solutions of the active compounds in water-soluble form, for example, water-soluble salts. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspension include, for example, sodium carboxymethyl cellulose, sorbitol, and/or dextran. Optionally, the suspension may also contain stabilizers. Liposomes can also be used to encapsulate the agent for delivery into the cell.

The pharmaceutical formulation for systemic administration described herein may be formulated for enteral, parenteral or topical administration. Indeed, all three types of formulations may be used simultaneously to achieve systemic administration of the active ingredient.

Suitable formulations for oral administration include hard or soft gelatin capsules, pills, tablets, including coated tablets, elixirs, suspensions, syrups or inhalations and controlled release forms thereof.

The compositions described herein can be utilized *in vivo,* ordinarily in mammals, such as humans, sheep, horses, cattle, pigs, dogs, cats, rats and mice, or *in vitro.*

Without further description, it is believed that one of ordinary skill in the art can, using the preceding description and the following illustrative examples, make and utilize the compounds of the present invention and practice the claimed methods. The following working examples therefore, specifically point out the preferred embodiments of the present invention, and are not to be construed as limiting in any way the remainder of the disclosure.

### EXAMPLES

### Example 1

### Single Cell Imaging Assay

Figure 2 shows the results of a single cell imaging assay. In this example, HEK293 cells transiently transfected with a CNG channel (SEQ ID NO: 3) for two days were loaded with a calcium fluorescent dye prior to the recordings. Specifically, cells were cultured on a microscope Fisher brand cover glass #1 pre-coated with MATRIGEL (Becton Dickinson, San Jose, CA) incubated in culture medium (DMEM with 10% fetal bovine serum) containing 5 µM fura-2 AM (Molecular Probes, Sunnyvale, CA) for 0.5 hour at 37°C. Calcium fluorescence recordings were made on Attofluor^{®} RatioVision^{®} a real-time fluorescence imaging device (ATTO, Rockville, MD). This system is capable of performing experiments using multiple fluorescent probes such as Fura-2 (for calcium) combined with GFP (transfection marker) in the same cells over the same period of time. Ratio images and graphical data from multiple dyes are displayed on line. This example demonstrates that activation of Gs-coupled GPCR and adenylyl cyclase can be detected by monitoring a change in cytosolic free calcium concentration in a single cell expressing of CNG channels in real time. Upon addition of norepinephrine (NE) and forskolin (forsk) at the times indicated by arrows in Figure 2, cytosolic calcium concentration started to rise in GFP-positive-*i.e*., transfected-cells. Two representative images were taken before (Panel A) and after (Panel B) adding NE showing individual cell responses. Calcium fluorescence changes are averaged respectively in GFP-positive cell population and whole cell population and displayed graphically (Panel C).

### Example 2

### Comparison of Calcium Sensitive Dyes and Voltage Sensitive Dyes in a Multiwell Format

In this example, recordings were made using a microplate reader FLEXstation (Molecular Devices, Sunnyvale, CA) and the protocols provided with the assay kit were adopted for both the calcium assay and the membrane potential assay. Figure 3 shows the results of a multiwell assay using a mutated CNG channel (SEQ ID NO: 5), which is reported to have a lower value EC50 value for cAMP (Rich, et al. 2001, J. Gen. Phys. 118:63-77) than that of SEQ ID NO:3, and therefore expected to be more sensitive to cAMP change. Figure 3A shows the response to isoproterenol (agonist of β2 adrenergic receptor) determined using a Ca²⁺ sensitive dye, fluo-4 in cells transiently transfected with the CNG channel. There was no significant change in fluorescence of the dye after stimulating cells with a saturating dose of isoproterenol of 10 µM. Figure 3B shows the results of activation with carbachol, a muscarinic receptor agonist, as a positive control for mobilization of intracellular calcium stores via the Gq pathway. The changes in intracellular Ca²⁺ concentration as a result of this treatment were observable.

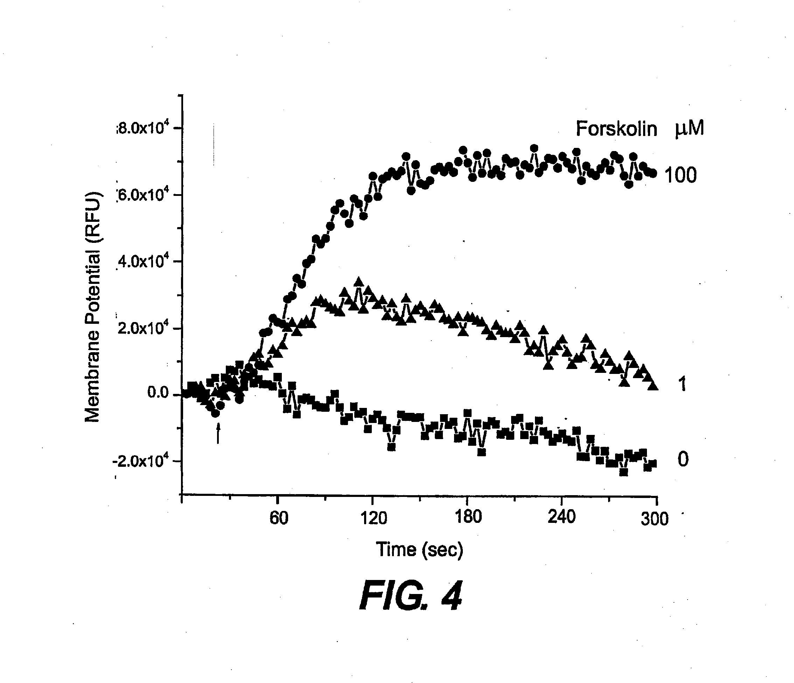

To establish the utility of CNG channels and membrane potential dyes in detecting intracellular cAMP, forskolin, an adenylyl cyclase activator, was used to generate intracellular cAMP. HEK293 cells transiently transfected with a CNG channel (SEQ ID NO: 5) were loaded with the voltage-sensitive dye at room temperature for about 0.5 hour. Figure 4 shows that upon the addition of forskolin, the intracellular cAMP can be readily detected in the presence of a voltage-sensitive dye by using a microplate reader.

### Example 3

### Assay for Intracellular cAMP in response to GPCR activation Using Mutant CNG Channel with a Membrane Potential Dye

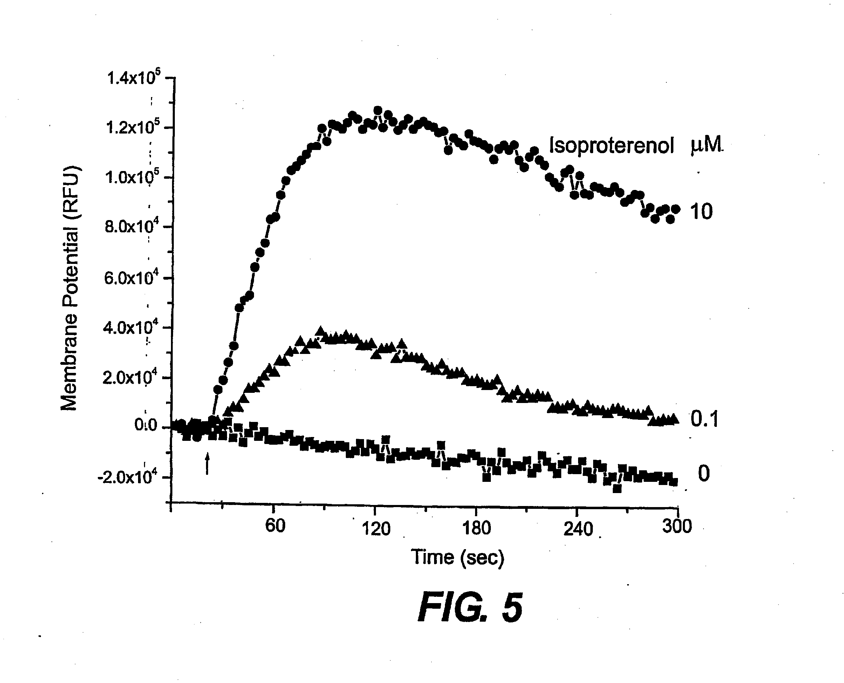

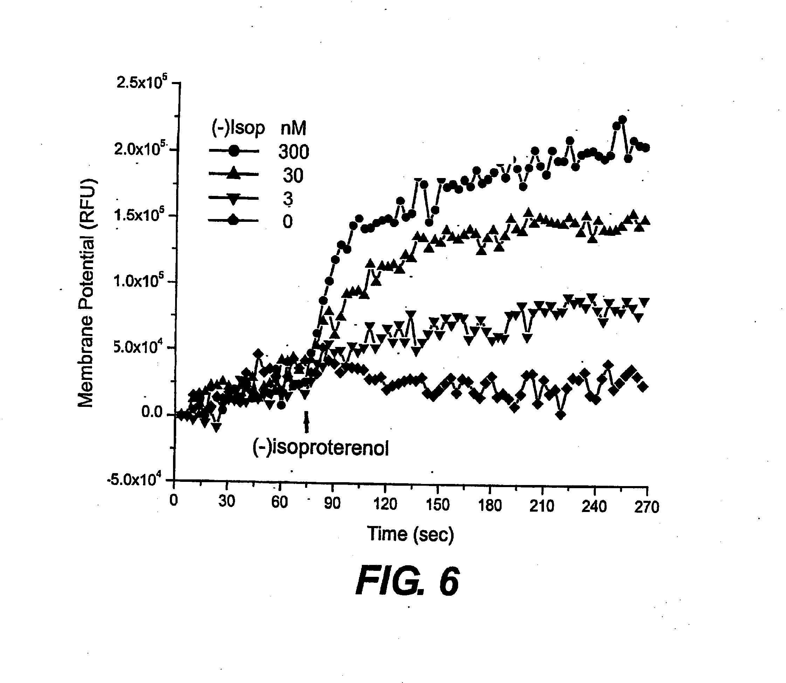

Figure 5 shows the results of a similar assay in which the voltage-sensitive dye of the membrane potential assay kit was used (Molecular Probes, Sunnyvale, CA). DPBS (divalent-free Dulbecco's Phosphate Buffer Salts) supplemented with 0.1 mM MgCl₂, 1 mM EGTA and titrated to pH 7.3 was used to reconstitute the voltage-sensitive dye instead of the buffer solution supplied in the commercial kit. HEK293 cells transiently transfected with a CNG channel (SEQ ID NO: 5) were loaded with the voltage-sensitive dye at room temperature for about 0.5 hour. A readily detectable change in fluorescence signal was seen at concentrations as low as 0.1 µM isoproterenol that activate β adrenoceptor, as compared to no detectable change with 10 µM isoproterenol using calcium-sensitive dye shown in Figure 3A. Similarly, Figure 6 shows the results with membrane potential dye with a different CNG channel mutant (SEQ ID NO: 7).

### Example 4

### Assay for Intracellular cAMP with Co-expressing GPCR and CNG Channel with a Membrane Potential Dye

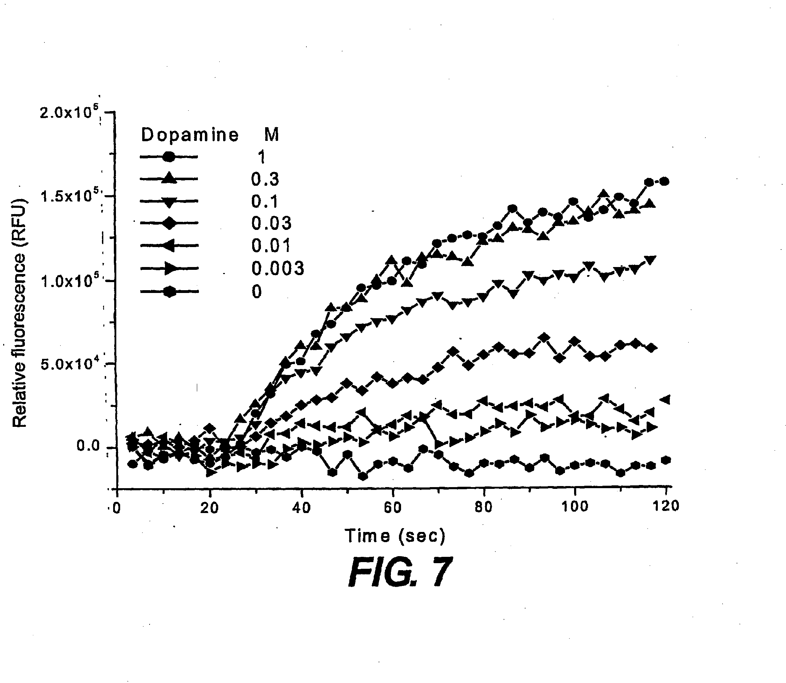

The assays of the present invention can be conducted using an exogenous introduced GPCR. DNA encoding a dopamine type I receptor was co-transfected into cells with a mutated CNG channel (SEQ ID NO: 7) into HEK273 cells. Figure 7 shows the results of activation of the receptor with its natural ligand, dopamine. Dosage dependent fluorescence signals are obtained immediately following the additional of dopamine in the presence of a membrane potential dye (Molecular Probes, Sunnyvale, CA).

### Example 5

### Identification of Ligands for an Orphan GPCR in Transiently Transfected Cells

Genes encoding for a wild type or mutated CNG channel protein and a GPCR of interest can be transfected into target cell using standard transfection techniques (Ausuebl et al., Current Protocols in Molecular Biology, (2001) John Wiley & Sons). Two days after transfection, approximately 50,000 cells/well for a 96-well plate and 10,000 cells/well for a 384-well plate may be used to create a confluent cell monolayer with a plating volume of 100 µL/well for 96-well plates or 25 µL/well for 384-well plates.

Cell plates may be removed from the incubator after overnight incubation. An equal volume of Loading Buffer with a membrane potential dye (Molecular Devices, Sunnyvale, CA) can be added to each well (100 µL per well for 96-well plates, 25 µL for 384-well plates) and the cell plates further incubated for 30 minutes at 37 °C. After incubation, the plates can be directly assayed using a FLIPR or FlexStation.

Candidate natural synthetic ligand collections can be obtained and diluted to concentrations ranging from 1 nM to 10 µM for testing. Membrane potential assays will be performed immediately following the addition of the compounds as described in the FLIPR system manual for membrane potential assay (Molecular Devices, Sunnyvale, CA). Such assays can also be performed in the presence of Ca⁺⁺ sensitive dye (Molecular Devices, Sunnyvale, CA).

### Example 6

### Identification of Agents that Modulate GPCR-mediated Activity

Compounds may be screened for their ability to function as agents for the modulation of one or more GPCR-mediated activities. A cell prepared according to the present invention may be contacted with a compound and one or more GPCR-mediated activities may be assayed. As an example, stable cell lines expressing a genes encoding for a CNG channel protein and a GPCR of interest can be obtained (Ausuebl et al., Current Protocols in Molecular Biology, (2001) John Wiley & Sons). The GPCR gene can be of either endogenous or exogenous sources. Approximately 50,000 cells/well for a 96-well plate and 10,000 cells/well for a 384-well plate can be used to create a confluent cell monolayer with a plating volume of 100 µL/well for 96-well plates or 25 µL/well for 384-well plates.

Cell plates can be removed from the incubator after overnight incubation. An equal volume of Loading Buffer with a membrane potential dye (Molecular Devices, Sunnyvale, CA) is then added to each well (100 µL per well for 96-well plates, 25 µL for 384-well plates) and the cell plates further incubated for 30 minutes at 37 °C. After incubation, the plates can be directly assayed using the FLIPR. Libraries of compounds can be obtained and diluted to concentrations ranging from 1 nM to 10 µM for testing. Membrane potential assays are performed immediately following the addition of the compounds as described in the FLIPR system manual for membrane potential assay (Molecular Devices, Sunnyvale, CA). Such assay can also be performed in the presence of both membrane potential and Ca²⁺ sensitive dyes (Molecular Devices, Sunnyvale, CA).

### Example 7

### A homogeneous, kinetic assay with HEK 293-CNG cells in 384-well plates with membrane potential dye

Populations of cells are stably transformed to express CNG channel and established under either adherent or suspension culture conditions. The cells are harvested and adjusted to 1 x 10⁶ cells/ml in DMEM (high glucose) comprising 10% FBS. 20 µl of the cell suspension is dispensed per well into 384-well microplates (Coming; 3712) and incubated 16-24 hours prior to assay. Immediately prior to assay, wells of the cell plates are observed microscopically to confirm the presence of confluent lawns of consistently spread cells.

Membrane potential dye stock (Membrane potential reagent kit, Component A, Molecular Devices, R-7056) stock solution is prepared by dissolving one bottle of dye in the kit in 10 ml Dulbecco's Phosphate Buffered Saline (DPBS) supplemented with 20 mM HEPES (pH 7.0), aliquoted into 1 ml portions and stored at -80°C. Dye Loading Buffer is prepared on the day of the assay by diluting 1 ml dye stock with 9 ml of the DPBS supplemented with HEPES at 20 mM, pH 7.0 per 384-well plate. 20 µl Dye Loading Buffer per well is added to the 384-well cell plates. Plates are incubated at room temperature, about 20-25°C, for 1-7 h. During incubation, dilutions of test compounds are prepared in Compound Buffer (10 mM EGTA in Dye Loading Buffer; pH 7.2).

Dye loaded cell plates are then loaded into a FLIPR384, FLEXstation, or other fluorescence microplate reader and assayed per fluorescence microplate reader instructions. For example, in a FLIPR384, 488 nm excitation and 540-590 nm emission filters are used; for FLEXstation and other fluorescence microplate readers, wavelengths close to the maxima of absorption and emission of the dye are used: for example, 540 nm excitation and 560 nm emission for the membrane potential dye of Molecular Devices, R-7056). Ten µl of test compound in Compound Buffer is added per well and the results are recorded.

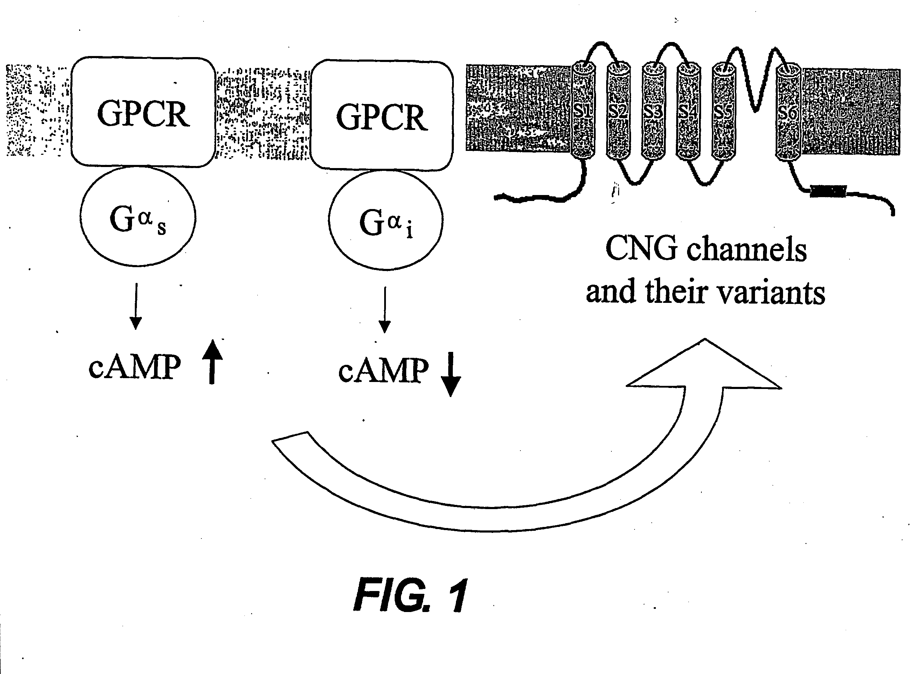

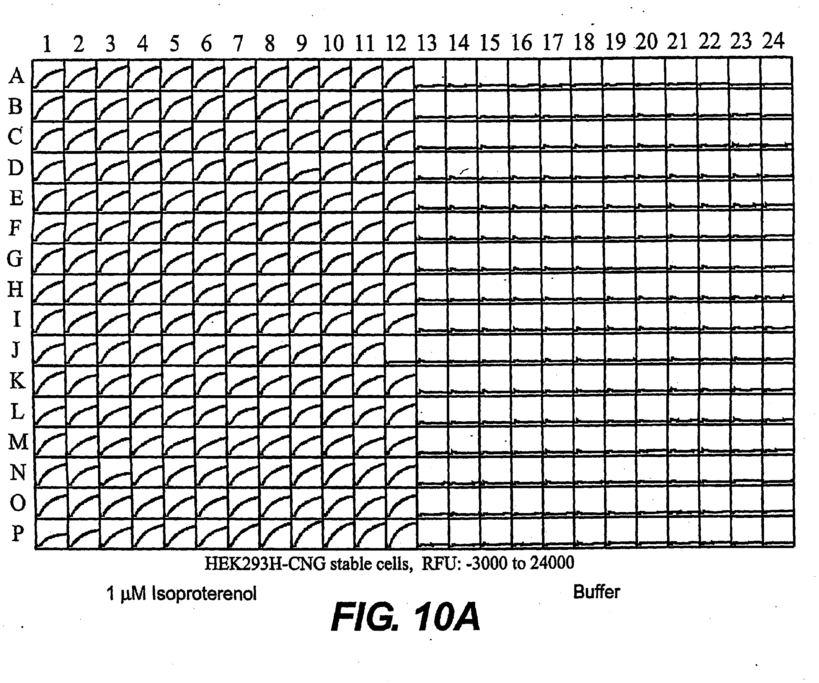

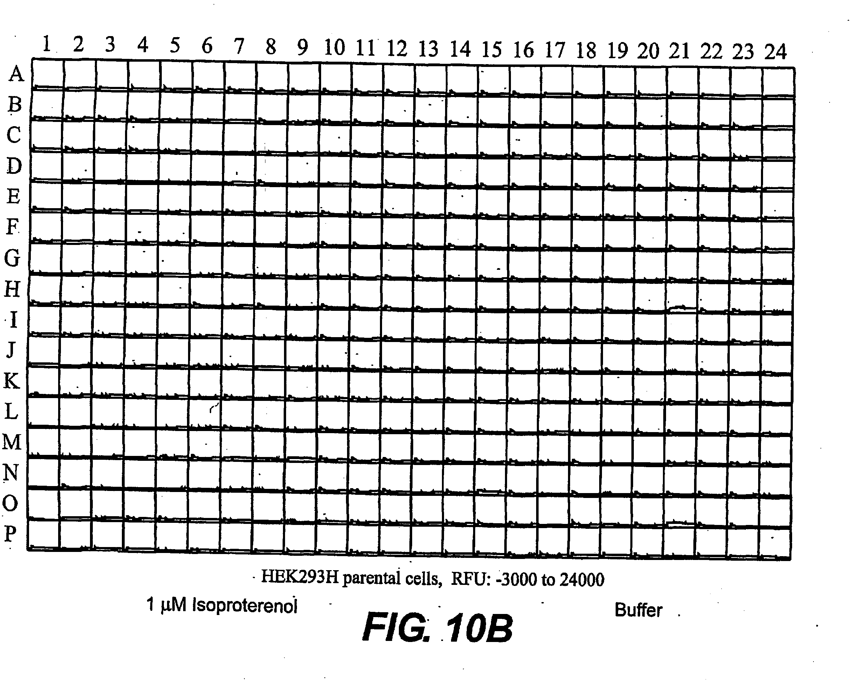

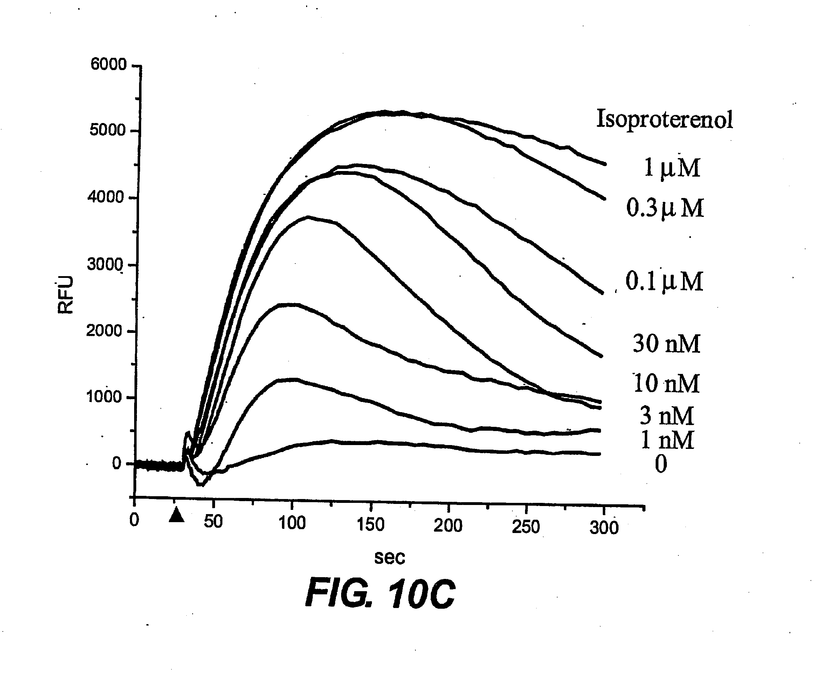

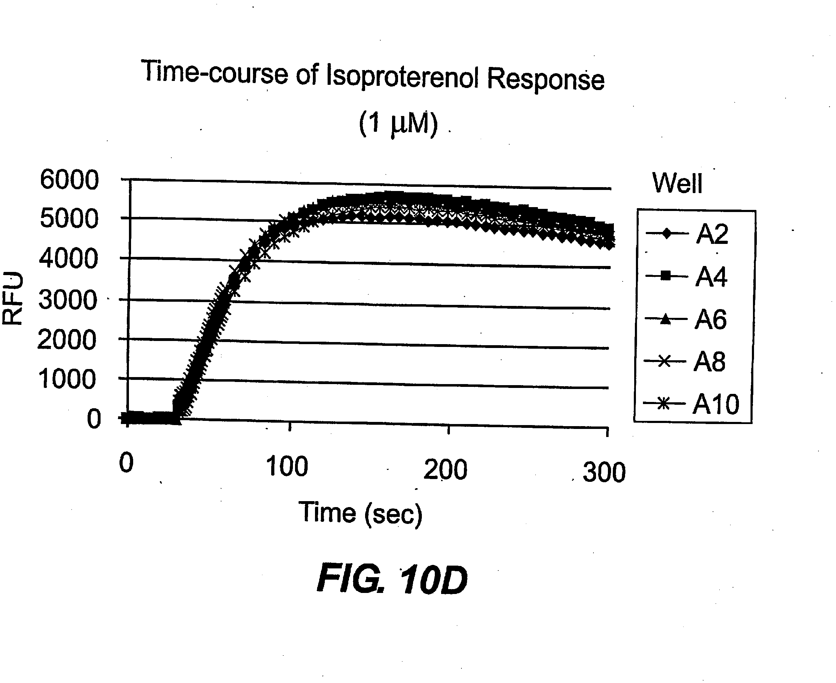

In the present example, the CNG channel assay was adapted to a HTS platform, FLIPR (Molecular Devices). Specifically, a stably transformed HEK 293H cell line expressing CNG channels (SEQ ID NO:: 7) was used for a cAMP assay. Cells were seeded into 384-well plate coated with MATRIGEL (Becton Dickinson, 354234) using a Multidrop 384 dispenser (Titertek Instruments, Inc.). Well-to-well variability of CNG channel assay for cAMP responses was assessed in the recordings of 4 min duration shown in Fig. 10. 30 seconds after beginning the recordings, Isoproterenol (1 µM final) was added to the wells of columns 1-12 while compound buffer was added as a control to the wells of columns 13-24 at time of (Fig. 10). HEK293H-CNG cell line and the parental cell line lacking CNG was used in Fig. 10A and 10B respectively. Dose-dependent responses to isoproterenol were obtained in separate recordings. Multiple fluorescence traces in response to various doses of isoproterenol were overlaid (Fig. 1OC). Data consistency is demonstrated in Fig. 10D by overlaying multiple responsive curves for 1 µM isoproterenol from the plate of Fig 10A.

### Example 8

### A kinetic assay with HEK 293H-CNG cells with calcium-sensitive dye

Populations of cells, *e.g.,* HEK 293 or HEK 293H, are stably transformed to express CNG channel, *e.g*., SEQ ID NO: 7, and established under either adherent or suspension culture conditions. The cells are harvested and adjusted to 1 x 10⁶ cells/ml in DMEM (high glucose) comprising 10% FBS. 20 µl of the cell suspension is dispensed per well into 384-well microplates, or 100 µl of the cell suspension is dispensed per well into 96-well microplates, and incubated 16-24 hours prior to assay. Immediately prior to assay, wells of the cell plates are observed microscopically to confirm the presence of confluent lawns of consistently spread cells.

Calcium-sensitive dye Fluo-4 AM (Molecular Probes, F-14202) is prepared as a 4 mM stock solution in DMSO and stored at -20°C. On the day of assay, stock solution is diluted to a final dye solution concentration of 4 µM cells in Hanks' Balanced Salt Solution (HBSS, pH 7.2). Cells are loaded with the dye by replacing the DMEM +FBS in the wells with the HBSS dye solution and incubation at room temperature for 1 hour. During incubation, prepare compound plates. Test compounds are dissolved in HBS or HBS supplemented with 10 mM CaCl₂.

Dye loaded cell plates are then loaded into a FLIPR384, FLEXstation, or other fluorescence microplate reader and assayed per fluorescence microplate reader instructions.

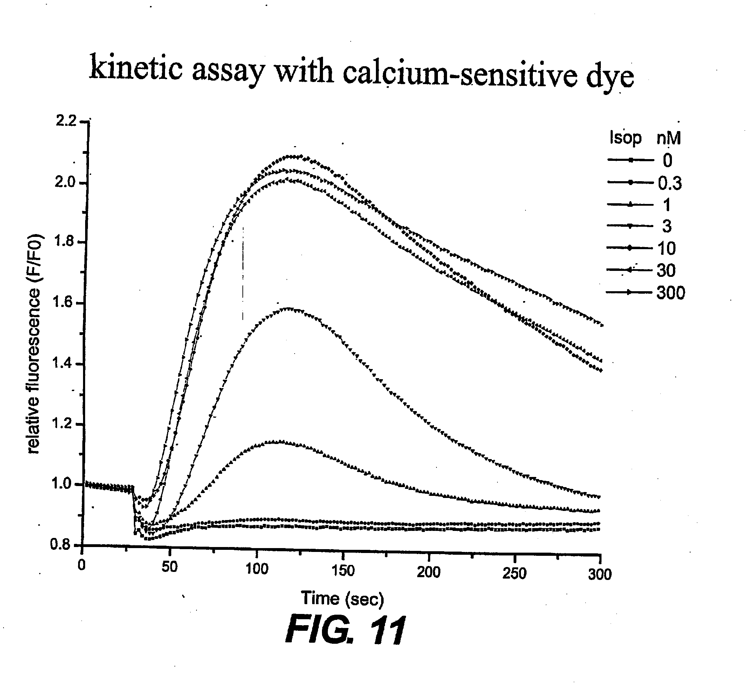

In the present example, a stably transformed HEK 293H cell line expressing CNG channels (SEQ ID NO: 7) was assayed for calcium uptake. 30 seconds after beginning the recordings, isoproterenol was added to wells at final concentrations of 0.3, 1.0, 1.0, 10.0, 30.0 and 300.0 nM. Buffer solution only was added to control wells. Dose-dependent responses to isoproterenol were recorded and multiple fluorescence traces in response to the various doses of isoproterenol were overlaid as shown in Fig. 11.

### Example 9

### Single Cell Imaging Assay:

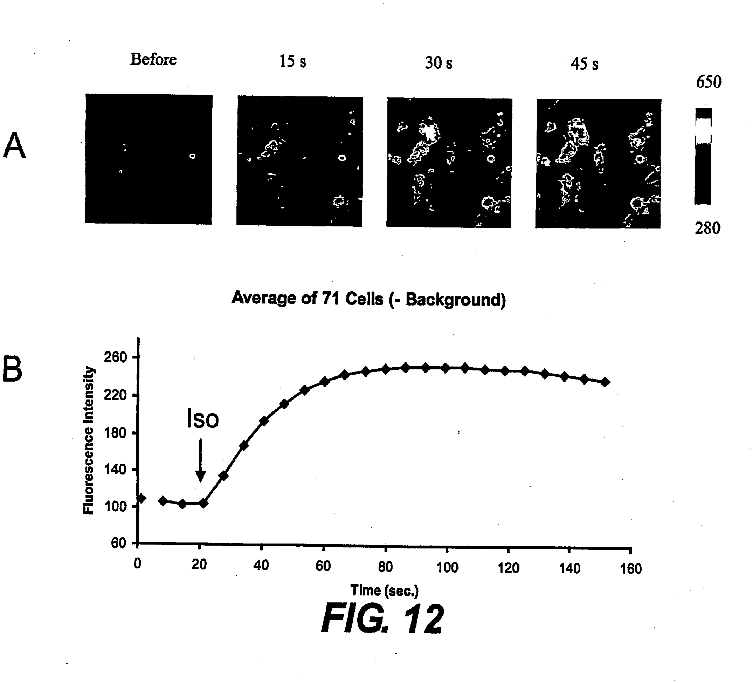

In this example, stably transformed HEK 293H cells expressing a CNG gene (SEQ ID NO: 7) were assayed using a voltage-sensitive dye (Molecular Devices, R-7056). Cells were seeded into a 96-well plate pre-coated with MATRIGEL. Membrane potential fluorescence recordings were made on Pathway HT imaging platform (ATTO, Rockville, MD) in the same cells over the same period of time. Confocal fluorescence intensity images were displayed in Fig. 12 with an arbitrary intensity range of 280-650. The images were obtained before and 15, 30 and 45 seconds after addition of isoproterenol to final 1 µM in a time sequence marked in the Fig 12A. One imaging area of 50x50 um was displayed in Fig 12A. Average of fluorescence traces obtained in 71 imaged cells was shown in Fig. 12B. The time of addition of isoproterenol was marked with an arrow.

### Example 10

### Assay for intracellular cAMP using CNG channels of wild type subunits and subunits containing mutations

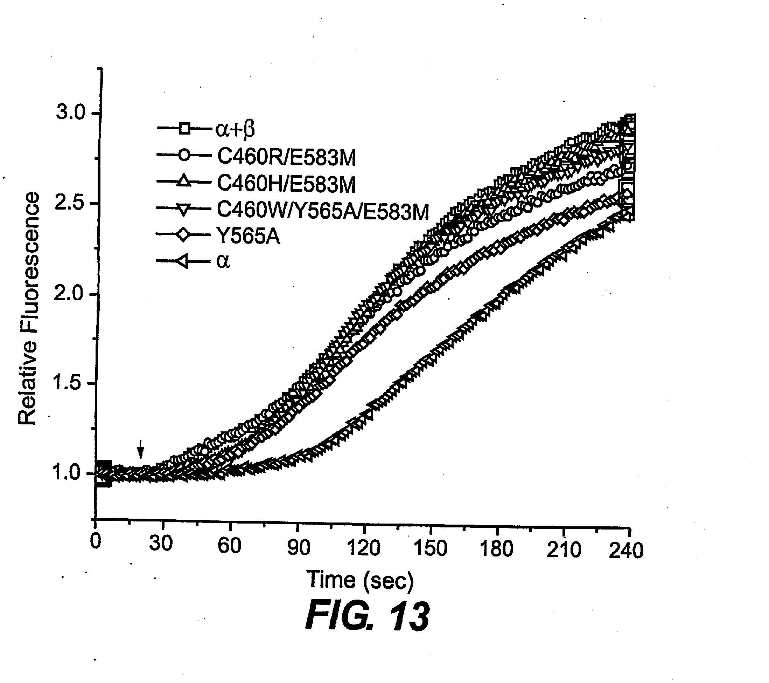

Relative fluorescence responses to cAMP rise of in cells transiently transfected with wild type α + β subunit heteromeric CNG channels, homomeric CNG channel α subunits comprising various mutations, or homomers of CNG channel wild-type α subunit alone was explored. In this example, HEK293 cells were transfected two days prior to the recordings with rat olfactory wild type CNG channel α subunit (NCBI LocusID 25411, SEQ ID NO: 1) plus β subunit (NCBI LocusID 85258), the rat olfactory CNG channel α subunit containing mutations C460R/E583M, C460H/E583M, C460W/Y565A /E583M (SEQ ID NO: 7), or Y565A (SEQ ID NO: 3), and α subunit alone (NCBI LocusID 25411, SEQ ID NO: 1). The cells were incubated in *Dye Loading Buffer* containing membrane potential dye (Molecular Devices, R-7056) at room temperature. Isoproterenol was dissolved in Compound Buffer as in Example 8 to a final concentration of 300 nM and added at the time marked with an arrow (Fig. 13). Figure 13 shows that isoproterenol responses in cells expressing heteromeric CNG channels composed of wild type α and β subunits are larger than or similar to those expressing CNG channels formed by homomeric wild type α subunit or α subunit homomers containing mutations of C460R/E583M, C460H/E583M, C460W/Y565A /E583M, or Y565A (Fig. 13).

### Example 11

### Sensitivity Comparison between CNG Channel Assay with a Conventional Transcription Assay and a cAMP ELISA Assay

A number of cAMP assay technologies have been developed based on the principles of competitive binding of cAMP antibody or transcription of genes regulated by cAMP-response elements (CRE). In assays using cAMP-specific antibodies, cell lysis is required to release cAMP to the assay media. As a result, assay sensitivity is compromised as cell number is reduced. In gene reporter assays, more false positive and negative recordings are expected as CRE transcription can be affected non-specifically by varieties of signaling pathways. In contrast, the CNG channel assay provides a direct physiological readout of cAMP intracellularly to avoid the problems associated with conventional indirect cAMP assay technologies. CNG channels are targeted to the plasma membrane and co-localized with adenylyl cyclase to permit a sensitive detection of a local cAMP rise. Because fluorescence readout in the CNG channel assay derives from activity of single live cells, assay sensitivity is not compromised by reducing cell numbers, as it may be in indirect assays.

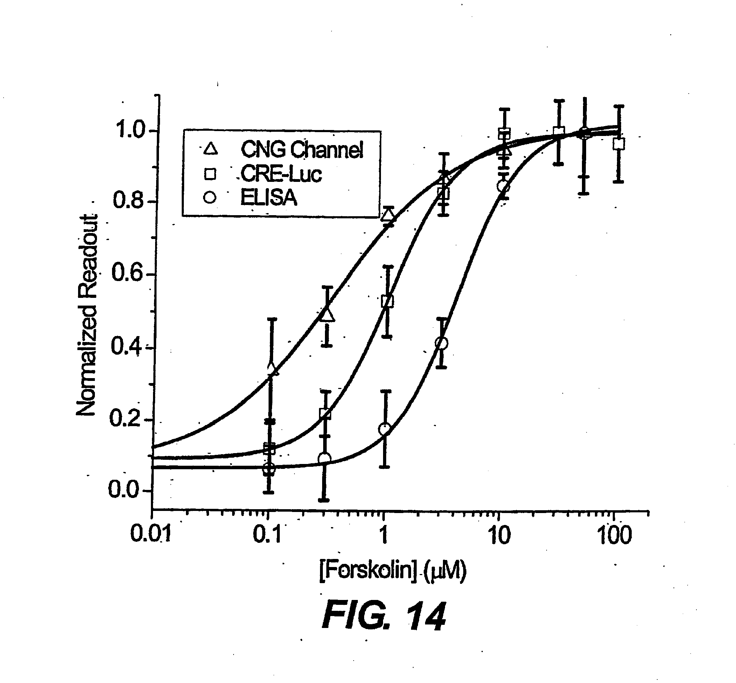

A comparison of the CNG channel assay was made with an ELISA-based anti-cAMP antibody binding assay (Amersham Biotrak kit, used according to kit directions) and a conventional CRE-Luciferase gene reporter assay in 96-well format. Dose-response curves were generated to forskolin using the same ligand concentrations for all 3 assay formats. Fig 14 shows that the response curve is left-shifted in general using the CNG Channel assay and demonstrates that low concentrations of forskolin induced a significantly larger response in CNG channel assay than in the ELISA and gene reporter assay formats, indicating that CNG channel assay is more sensitive than these conventional cAMP assays.

### Example 12

### CNG Channel Assay for Gs-Coupled GPCRs of Different Families

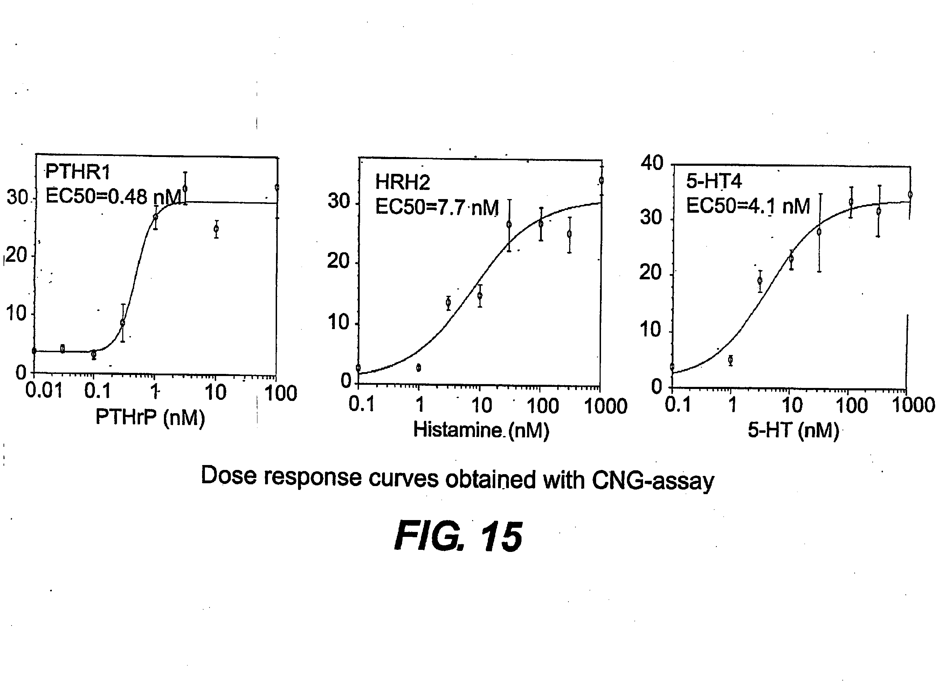

To show the sensitivity of the CNG channel assay as a kinetic assay for Gs-coupled GPCRs, Gs-coupled GPCRs were randomly chosen from class B (the secretin family) and class A (including, for example, biogenic amines, peptides, prostanoids, and adenosine receptors) for assay. Activation of the GPCRs listed in Table 1 by their cognate agonists was examined applying CNG channel assay. All of these GPCRs were read out successfully by CNG channel assay, demonstrating the ability of the CNG assay to provide accurate kinetic measurements of Gs-coupled CPCR activation, regardless of its ligand family. Fig. 15 showed dose-response curves of three GPCRs tested to illustrate their relevance to the other pharmacological analysis.

**Table 1. Gs-coupled GPCRs tested with CNG channel assay**

| **Receptor** | **Ligand Tested** | **Ligand Type** |
|---|---|---|
| Dopamine D1 | Dopamine | Mono amine |
| Beta-Adrenergic | Isoproterenol, Epinephrine | Mono amine |
| Histamine H2 | Histamine | Mono amine |
| 5-Hydroxytryptamine 4 | 5-Hydroxytryptamine | Mono amine |
| Tyramine | Tyramine | Mono amine |
| Prostaglandin E 2 | Prostaglandin E1 | Lipid |
| Prostaglandin D2 | Prostaglandin D2 | Lipid |
| Calcitonin | Calcitonin | Peptide |
| Glucagon | Glucagon | Peptide |
| Parathyroid Hormone 1 | PTH, PTHrP | Peptide |
| Vasoactive Intestinal Peptide 1 | VIP | Peptide |
| Arginine Vasopressin 2 | Arginine Vasopressin | Peptide |
| Melanocortin 1 | α MSH | Peptide |
| Melanocortin 3 | α MSH | Peptide |
| Melanocortin 4 | α MSH | Peptide |
| Melanocortin 5 | α MSH | Peptide |
| Adenosine A2b | NECA | Nucleotide |

### Example 13

### Robustness of CNG channel assay in comparison with assay using promiscuous G protein and G protein chimera

Calcium fluorescence assays using the promiscuous G protein Gα₁₆ and the G protein chimera Gα_{qs} have been previously used to measure intracellular calcium rise. However, measurement of GPCR activation using either Gα₁₆ or Gα_{qs} is indirect, as both re-direct the activity of some Gs-coupled GPCRs to a phospholipase C-mediated intracellular calcium rise. Because the coupling efficiency of Gα₁₆ and Gα_{qs} varies between Gs-coupled GPCRs, the final calcium signal readout varies between receptors.

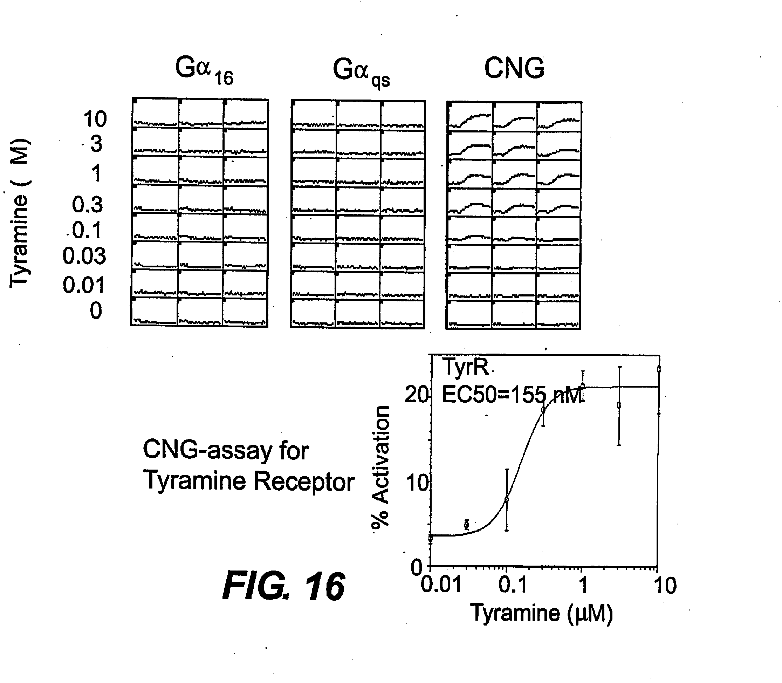

In this example, activation of the tyramine receptor was examined using the CNG channel assay and compared with calcium fluorescence assays using Gα₁₆ and Gα_{qs}. Similar amounts of plasmids comprising CNG channel, Gα₁₆ or Gα_{qs} were used for transient expression in HEK293 cells also transiently expressing tyramine receptor. Calcium assays were performed following the protocol provided by Molecular Devices. For each concentration of tyramine, three recordings were made to obtain an average response in cells expressing Gα₁₆, Gα_{qs} and CNG channel respectively. As shown in Fig. 16, tyramine receptor activation is detectable using the CNG channel assay with membrane potential dye, but not by calcium fluorescence assays using Gα₁₆ or Gα_{qs} and calcium dye (Fig. 16).

### Example 14

### Identification of Agonists and Antagonists of GPCRs Applying CNG Channel Assay

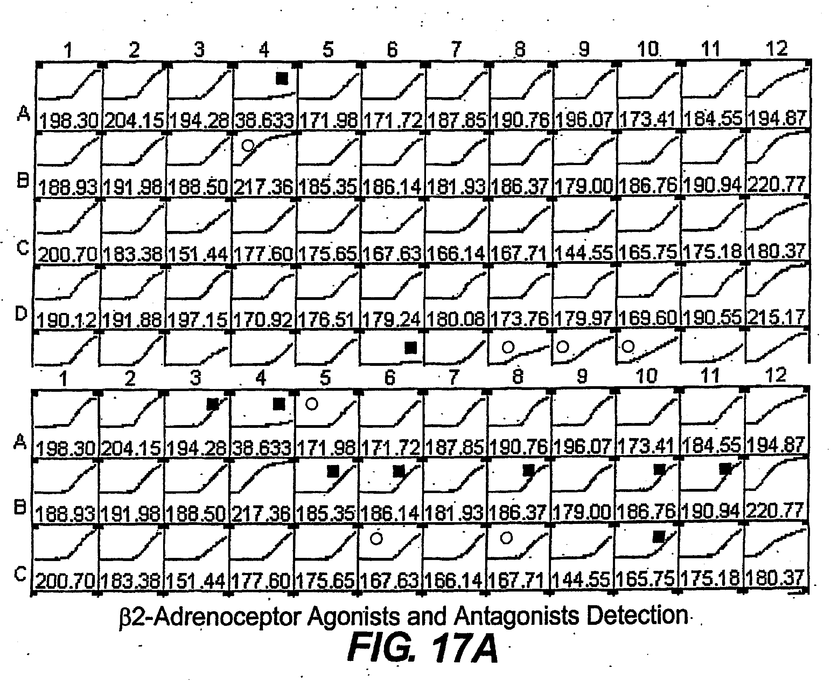

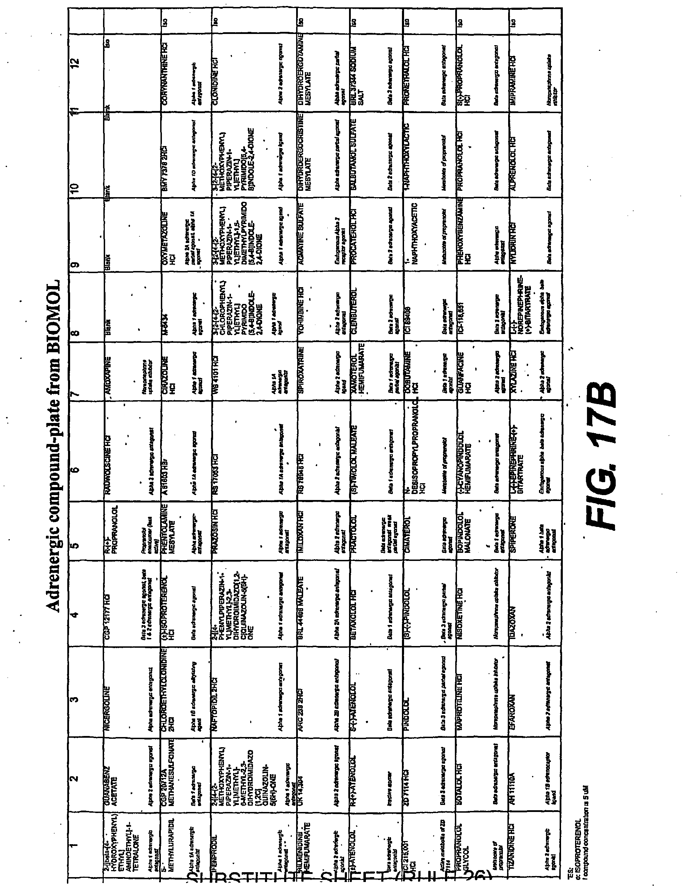

The CNG channel assay can be used to identify GPCR ligands. In this example, the CNG channel assay was used to probe a panel of adrenergic compounds for those that are agonists or antagonists of β-adrenoreceptors. Stably transformed HEK293H cells expressing a CNG channel gene (SEQ ID NO: 7) were seeded into 96 well plates and grown as described in previous examples. Test compounds were arrayed in columns 1-11 of a 96-well plate as shown in Fig. 17B, with buffer only as a control in column 12. Test compounds were added to the cell plates 20 seconds after the start of recordings to a final concentration of 1 µM. Isoproterenol, 10 µM final concentration, was added at 120 seconds to evoke cAMP rise. Time duration of recordings was 230 seconds. Agonists were identified by the detection of a fluorescence rise immediately following the addition of the test compound, before the addition of isoproterenol and are marked by hollow circles in Fig. 17A. Antagonists were identified by delaying or ablating the response of cells to isoproterenol stimulation, as marked by solid squares in Fig 17A.

### Example 15

### Endpoint Assay

The CNG channel assay can also be used to perform endpoint assays. Decay of fluorescence responses results from desensitization of CNG channel was effectively removed by chelating extracellular calcium by EGTA and supplementing inhibitors of phosphodiesterase. Stably transformed cells expressing a CNG channel gene were seeded into 384 well plates and grown as described in previous examples. Forskolin was dissolved at a final concentration of 30 µM in Compound Buffer containing EGTA. Fluorescence intensity values of cells incubated with forskolin or with Compound Buffer only were read using FLEXstation (Molecular Devices) at different time points after forskolin stimulation. Treatment with forskolin resulted in a fluorescence intensity of 5.3±0.5 x 10⁵ RFU (30 µM forskolin, n=192) at 90 minutes after stimulation, versus 1.9±0.1 x 10⁵ RFU (buffer control, n=192), representing a 2.8-fold increase.

### Example 16

### Identification of Fluorescence Quenchers

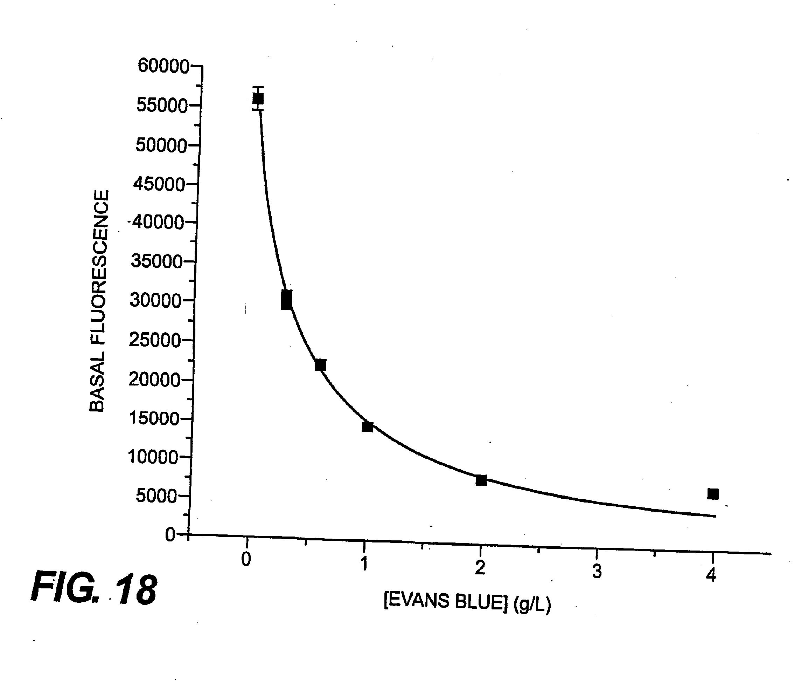

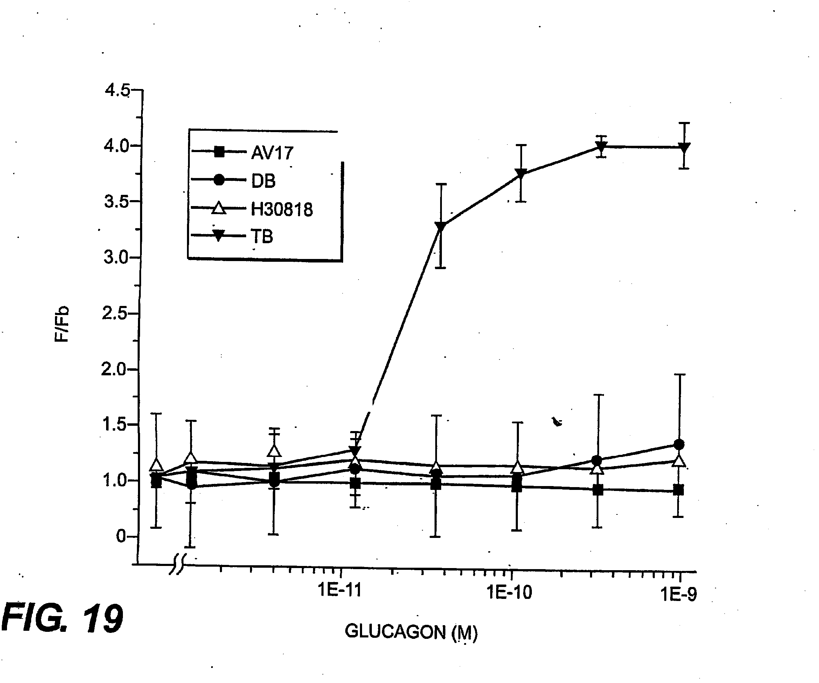

Generally, the pre-selection of dye quenchers (Table 1) is based on their physical chemistry properties: (1) water solubility, (2) absorption spectra overlapping with emission spectra of fluorescent indicators, (3) low autofluorescence, and (4) complete exclusion from cells, i.e. do not affect intracellular fluorescence (Sahlin *et al.,* 1983). Effective quenching of extracellular fluorescence by absorbance dye quenchers is achieved at 0.1-10 mM concentration (Fig. 18) (Sahlin et al., 1983, Differentiation between attached and ingested immune complexes by a fluorescence quenching cytofluorometric assay. J. Immunol. Methods. 60:115-124; Wan et al., 1993, A rapid and simple microfluorometric phagocytosis assay. J. Immunol. Methods. 162:1-7). Positive-charged dyes have large quenching effects on a negative-charged fluorescent voltage dye, HLB30602, in cell-free solution (Table 2). However, many quenchers are not suitable for use in cell-based assays due to their poor exclusion by cells (Sahlin *et al.,* 1983). For instance, cellular responses were totally lost in the presence of many known positive-charged dye quenchers (Fig. 19). Prior to the present invention, it was not known how to select a quencher dye that will have little or no interference on cell-based assays based on the molecular structure of the dye or its physical chemistry properties.

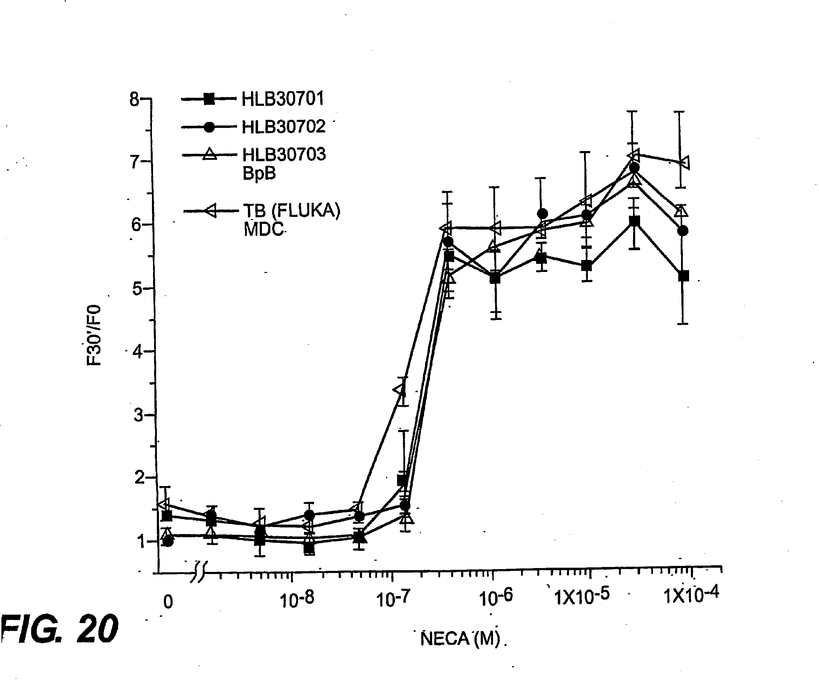

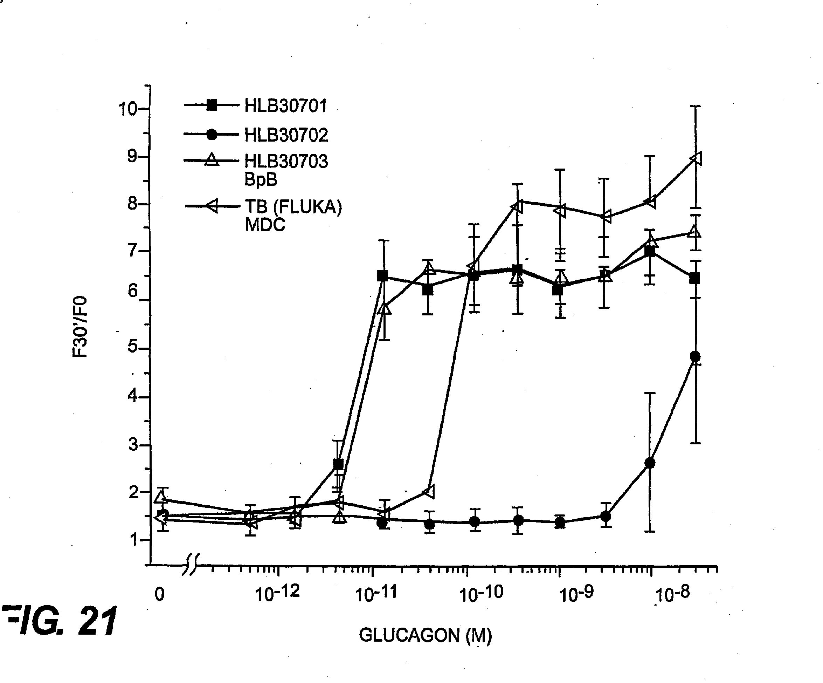

A functional screening of dye quenchers against 20 ligand-receptor pairs was performed in order to identify those candidate quenchers with the least pharmacological interference (Table 3). The chemical composition of the tested ligands include peptides and small organic molecules such as adenosine, lipid, monoamine, etc. Of the quenchers screened using the described invention, many were found to reduce the potency (EC₅₀) of the ligands without effecting the maximum responses. Further quenchers were identified that inhibited maximal response in the assay (bromophenol blue, Fig. 20, 21). Many dye quenchers such as trypan blue, HLB30701, HLB30702, and HLB30703 (Fig. 20, Table 2) are suitable for use in assays in which the ligand is a small organic compound. However, we discovered that several of these quenchers interfered with assay responses to peptide ligands.

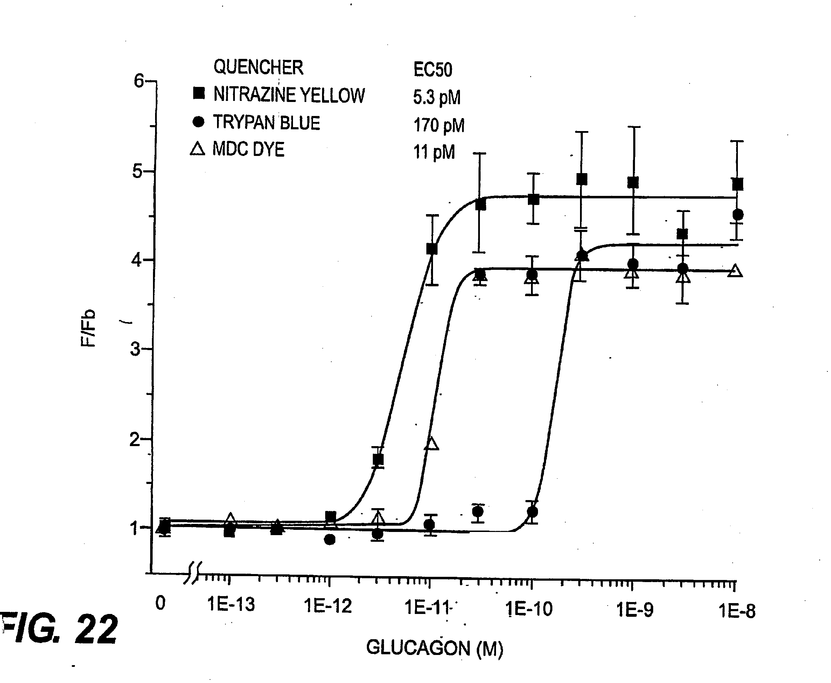

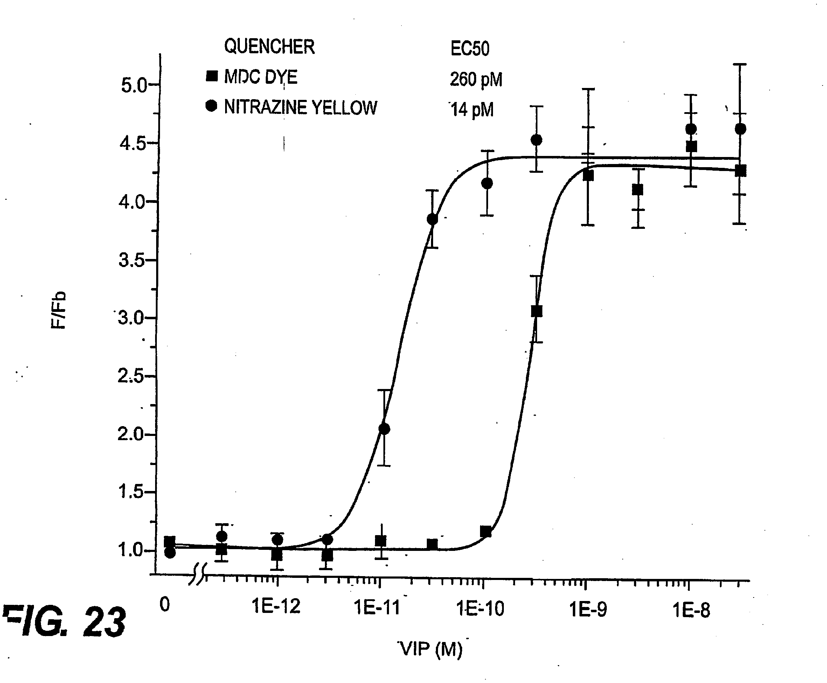

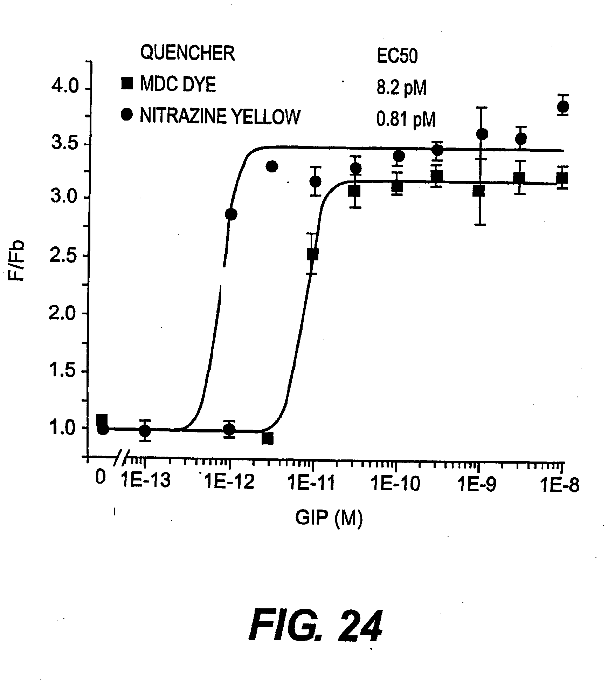

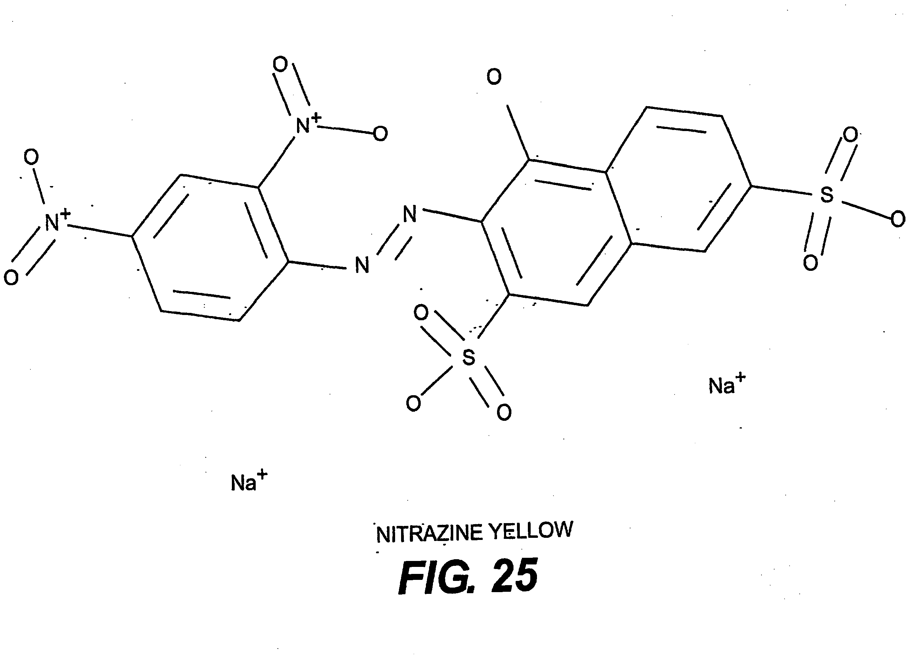

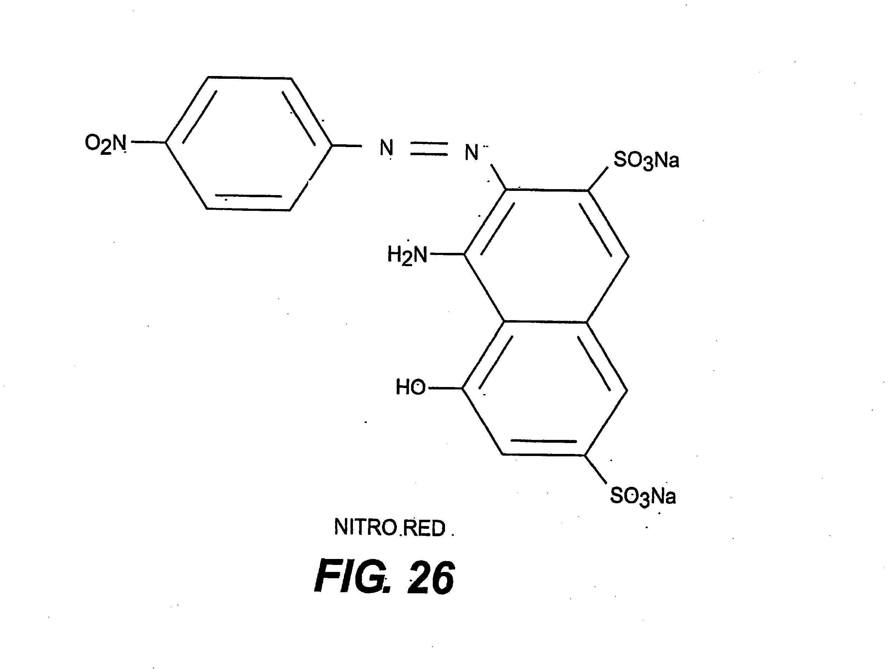

For example, trypan blue significantly reduced glucagon potency (Fig. 22, 23, Table 3). Nitrazine yellow (Fig. 25) and nitro red (Fig.26) were identified in the functional screening of the invention. These quenchers were found to provide the most sensitive assay for peptides, and small molecule ligands (Table 3). For example, the dose-response curves of the peptides VIP and GIP, are left-shifted by about one order of magnitude using nitrazine yellow as the quencher in comparison with a non-disclosed quencher in a commercially available voltage dye assay kit (Molecular Devices, Corp., R-8034) (Fig. 23, 24).

**Table. 2. A list of dye quenchers are pre-selected based on their absorbance spectra. Quenching efficiency was determined by ratioing fluorescence levels of a voltage dye, HLB30602 of 10 µM, in the absence and presence of quencher dyes at concentration of 0.5 g/L (F0/F).**

| Dye Quencher | MW | Absorbance Peak (nm) | Dye Charge | Solubility in Water (g/L) | Quenching Efficiency | |
|---|---|---|---|---|---|---|
| | | | | | Residual F (k) | F0/F |
| Acid Blue 92 | 695.59 | 571 | -3 | 40 | 57.60 | 36 |
| Acid Blue 113 | 681.66 | 566 | -2 | 40 | 112.50 | 19 |
| Acid Violet 17 | 761.94 | 545 | -2, +1 | 30 | 6.90 | 304 |
| Alizarin Red S monohydrate | 360.28 | 556 | -1 | 20 | 335.10 | 6 |
| Brilliant Black BN | 867.69 | 570 | -4 | 30 | 19.30 | 109 |
| Bromochlorophenol Blue | 580.07 | 590 | -1 | 20 | 421.00 | 5 |
| Bromocresol Purple | 562.22 | 585 | -1 | 80 | 115.60 | 18 |
| Bromophenol Blue | 598 | 589 | -1 | 30 | 44.90 | 47 |
| Bromopyrogallol Red | 576.18 | 552 | -1 | 5 | 31.60 | 66 |
| Chlorophenol Red | 423.28 | 572 | -1 | 30 | 62.40 | 34 |
| DIAZINE BLACK | 484.01 | 584 | +1 | | 9.30 | 226 |
| Direct Violet 51 | 719.71 | 549 | -2 | 20 | 152.00 | 14 |
| MELDOLA'S BLUE | 378.93 | 570 | +1 | | 2.10 | 1000 |
| Methylene Violet 3Rax | 378.91 | 557 | +1 | 10 | 87.40 | 24 |
| New Fuchsin | 365.91 | 553 | +1 | 20 | 3.40 | 618 |
| Nitrazine Yellow | 542.37 | 586 | -2 | 20 | 59.80 | 35 |
| Nitro Red | 512.39 | 572 | -2 | 40 | 62.10 | 34 |
| Palatine Chrome Black 6BN (Calcon) | 416.39 | 569 | -1 | 30 | 362.00 | 6 |
| Sulfonazo II | 776.57 | 567 | -4 | 40 | 98.70 | 21 |
| Thymol Blue | 488.58 | 590 | -1 | 60 | 699.00 | 3 |
| Trypan Blue (Fluka) | 960.82 | 607 | -4 | ∼10 | 26.10 | 80 |
| XYLENOL ORANGE | 760.6 | 580 | | | 164.90 | 13 |
| HLB30701 | 776.58 | 573 | | >20 | 52.20 | 40 |
| HLB30702 (Trypan Blue) | 960.82 | 608 | | >20 | 5.50 | 382 |
| HLB30703 | 867.69 | 571 | | >20 | 17.90 | 117 |
| HLB30401P | 941.01 | 542 | | >20 | 17.70 | 119 |
| HLB30818 | 1,447 | 607 | +1 | | 4.40 | 477 |

**Table 3. Quencher dyes were screened against 20 ligand-receptor pairs to determine their effect on cell responses characterized by EC 50 and maximal responses (Fmax/Fb). Nitrazine yellow and nitro red were screened out because peptide and lipid agonists showed higher potency comparing with using other dye quenchers, such as trypan blue and a commercial dye kit from Molecular Devices, Corp. (MDC, Cat # R-8034).**

| **Cell Line** | **Receptor** | **Ligand** | **NY & H30602** | | **MDC dye** | | **TB & H30602** | | **NR& H30602** | |
|---|---|---|---|---|---|---|---|---|---|---|
| | | | **EC50** | **Fmax**/ **Fb** | **EC50** | **Fmax/ Fb** | **EC50** | **Fmax**/ **Fb** | **EC50** | **Fmax**/ **Fb** |
| ASC0089 | GCGR | glucagon | 5.9 pM | 4.9 | 11 pM | 4 | 183 pM | 4.6 | 3.6 pM | 4.2 |
| ASC0089 | Cyclase | forskolin | 0.16 µM | 5.1 | 0.23 µM | 5.3 | | | | |
| ASC0089 | β-adrenoceptor | isoproterenol | 2.6 nM | 5.4 | 2.5 nM | 4.9 | | | | |
| ASC0093 | DP | PGD2 | 7.8 pM | 4.2 | 18 pM | 4.4 | | | | |
| ASC0097 | A2B | NECA | 120 nM | 4.3 | 72 nM | 4.8 | 66 nM | 4.7 | | |
| 4SC0097 | EP2 | PGE1 | 40 nM | 5.3 | | | | | | |
| ASC0106 | GLP1R | GLP | 3.0 pM | 3.7 | 9.3 pM | 4.3 | | | | |
| 4SC0069 | PTH1R | PTH | 20 pM | 3 | 50 pM | 3.4 | | | 18.8 pM | 2.7 |
| ASC0145 | VIP2R | VIP | 14 pM | 4.2 | 258 pM | 4.2 | | | 14.9 pM | 3.8 |
| ASC0164 | IP | iloprost | 5.7 pM | 4.6 | 13.5 pM | 4.4 | 22.5 pM | 3.8 | 6.9 pM | 4.8 |
| ASC0172 | EP4 | PGE2 | 26.5 pM | 4.6 | 33.1 pM | 4.4 | 88 pM | 4.6 | 32.3 pM | 4.8 |
| ASC0182 | DRD1 | dopamine | 234 pM | 3.1 | 292 pM | 2.9 | | | 312 pM | 2.7 |
| ASC0192 | MC5R | [Nle4-D- Phe7]α-MSH | 0.95 µM | 3.4 | 2.9 µM | 3.6 | | | 1.3 µM | 3.2 |
| ASC0143 | VIP1R | VIP | | | 2.4 pM | 4.9 | 5.0 pM | 5 | | |
| ASC0154 | A2A | CGS21680 | | | 3.2 nm | 3.8 | 3.4 nM | 3.4 | | |
| ASC0169 | AVPR2 | AVP | | | 8.3 pM | 5.1 | 6.7 pM | 4 | | |
| ASC0179 | MC1R | α-MSH | | | 0.82 µM | 5 | 0.77 µM | 5.1 | | |
| ASC0133 | GIPR | GIP | 0.81 pM | 3.8 | 8.2 pM | 3 | | | | |
| ASC0156 | CALCRL | hCGRPb | 16 pM | 4.6 | 18 pM | 4.4 | | | | |
| ASC0177 | CRHR1 | CRF | 28 pM | 4.3 | 28 pM | 4.2 | | | | |

| | | | | | | | | | | |
|---|---|---|---|---|---|---|---|---|---|---|
| >= 1 order EC50 shift ∼0.5 order EC50 shift | | | | | | | | | | |

### Example 17

### CNG Channel Pore Mutations

CNG channels from retina and olfactory tissues are highly homologous at the protein level. However, the CNG channels differ significantly in their specificity, responding primarily to cGMP or cAMP, respectively. Several structure-function studies published on retinal CNG channels have disclosed single-site changes that affect these specificities. Other publications have disclosed alternative positions and changes that effect the identity of ions that transit the activated channel, or the sensitivity of the channel toward cyclic nucleotides. These changes provided guidance for introducing a combination of mutations within the rat olfactory channel CNGA2 (formerly designated rOCNC1) in an attempt to increase cAMP specificity, sensitivity, and robustness for application as a cAMP sensor in high-throughput screening. It was not evident that either the site chosen or the identity of the amino acid change would function in the rat CNGA2 channel in a manner similar to those described. Nor was it evident that if mutations were identified with the desired individual effects, that the multiple mutations would function together to provide the cumulative desired effects.

The mutations E342G, and E342N (see SEQ ID NO: 2 for the base sequence of the rat CNGA2) were generated in rat CNGA2 based on electrophysiological data published on rod cGMP-gated channel (Eismann et al., 1994, A single negative charge within the pore region of a cGMP-gated channel controls rectification, Ca blockage, and ionic selectivity. PNAS, 91:1109-1113) and bovine olfactory CNGA2 channel (Gavazzo et al., 2000, A point mutation in the pore region alters gating, Ca blockage, and permeation of olfactory cyclic nucleotide-gated channels. J. Gen. Physiol., 116:311-325) as effecting calcium flow through the channel. Using the CNG of the current invention it was shown for the first time that rat CNGA2 containing mutations at this position provide improved signal-to-noise ratios in high-throughput screening assays when using either voltage potential dyes or calcium detector dyes.

Several amino acid changes were made at other two sites, C460 and E583 (see SEQ ID NO: 2 for the base sequence of the rat CNGA2), of rat CNGA2. Mutations or modifications of amino acids at these two sites have been shown to increase sensitivity and selectivity of the channels toward cAMP (Varnum et al., 1995, Molecular mechanism for ligand discrimination of cyclic nucleotide-gated channel. Neuron, 15:619-25; Brown et al., 1998, Movement of gating machinery during the activation of rod cyclic nucleotide-gated channels. Biophys. J., 75:825-833; Rich et al., 2001, In vivo assessment of local phosphodiesterase activity using tailored cyclic nucleotide-gated channels as cAMP sensors. J. Gen. Physiol., 118:63-77).

In summary, mutations in the rat CNGA2 channel at several sites were generated and found to increase signal and sensitivity of the assay. The tested mutations include: E342G, E342N, E342D, E342Q, and E342K; C460R, C460L, C460W, and C460H; E583Q, E583M, E583V, E583A, E583L, E583F, and E583K. The rat CNGA2 channels containing these mutations have been functionally tested following transient expression in HEK293 cells. E342G, E342N mutations were identified as providing a robust response when cells expressing the adenosine A2B receptor are treated with the agonist NECA in the presence of extracellular calcium.

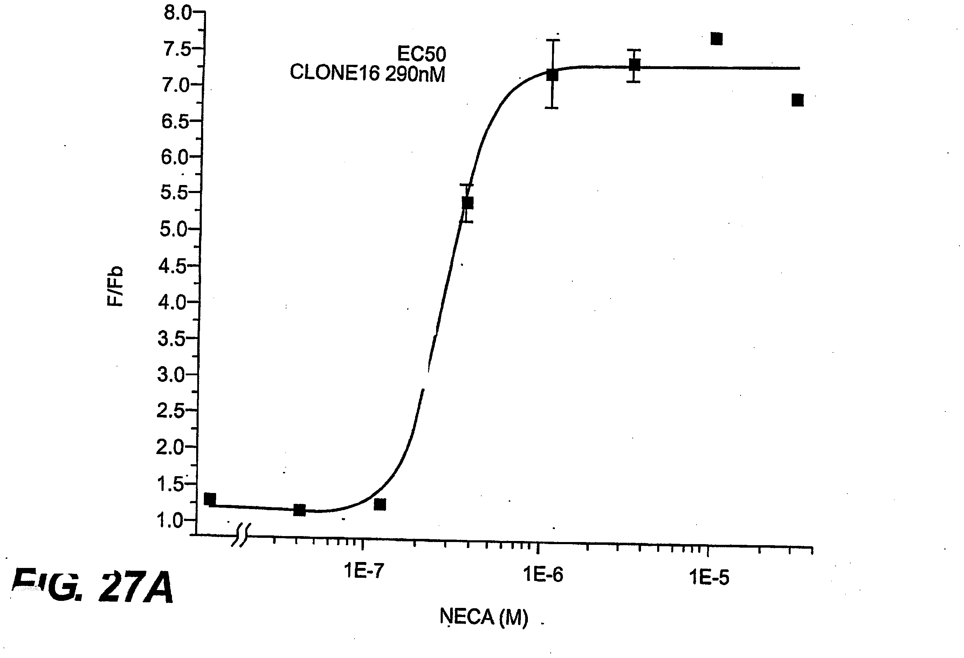

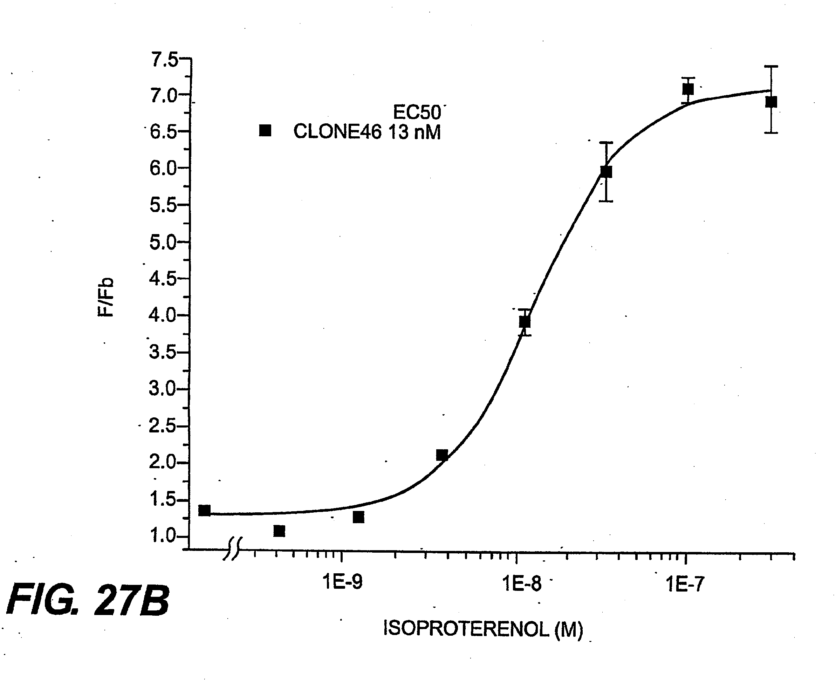

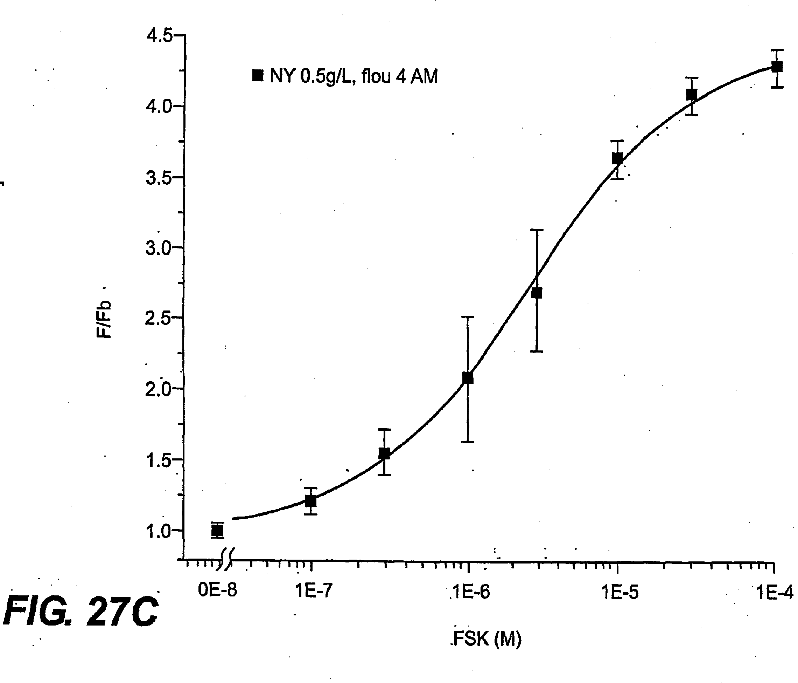

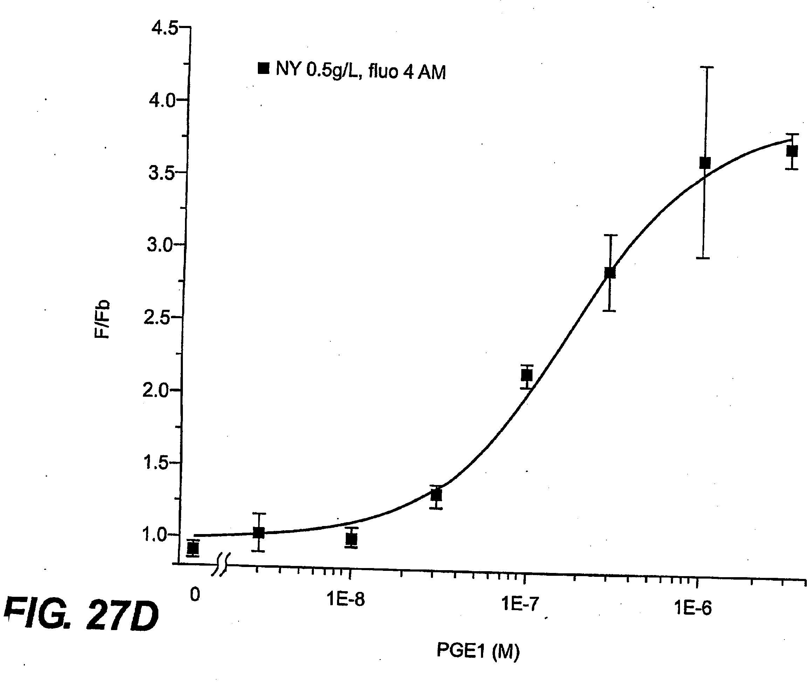

A stable cell line has been generated that contains rat CNGA2 with the E342G mutation. In assays using pore mutants, the signal-to-background ratio was found to be significantly improved when using either membrane potential voltage-sensitive dyes or calcium-sensitive dyes (Fig. 27). In the assay using CNG channel without a pore mutation, EGTA has to be included to chelate calcium ions to reveal voltage-change mediated through activation of CNG channels, because extracellular ions block cation currents through the channel pore. We found that addition of EGTA in the extracellular assay solution slightly depolarizes cell membranes causing fluorescence increases over baseline by about 40%. This effect of EGTA might result from activation of store-operated cation current. In assays using a CNG channel, the baseline drift was eliminated so that signal increases by about 40%. Dose-response relations of NECA and isoproterenol were obtained using a CNG triple mutant (E342G, C460W, E583M) cell line and using a membrane potential dye, HLB30602 (Fig. 27, A, B). EGTA exerts other effects including causing morphological changes of cells. Cells became round-up and started to detach from culture substrate in the presence of EGTA. The use of CNG channel pore mutants has solved these problems associated with using EGTA. The pore mutation also contributes to increase in calcium permeability so that the assay is more robust using calcium dye. Dose-response relations of forskolin and PGE1 were obtained using a CNG triple mutant (E342G, C460W, E583M) cell line and using a calcium dye, fluo-4 (Fig. 27, A, B).

Rat CNGA2 derivatives containing mutations at sites 342, 460, and 583 were further constructed and tested. One such mutant: E342G, C460R, E583L was found to generate functional homomeric channels with several unique properties, including: (1) better sensitivity; (2) better signal-to-noise performance; (3) more physiological assay conditions that avoid the necessity of using Ca-chelator, EGTA; and (4) facilitating delivery of compounds in HTS operation.

### Example 18

### Assay of Gi-coupled GPCRs and use of Gs-i chimera

Gi-coupled receptors comprise approximately 45% of all GPCRs. It is also the most difficult group of receptors for HTS assay development. Because Gi receptors downregulate cAMP levels, assays detecting their activation are typically based on their inhibition of upregulation of cAMP by forskolin or activation of Gs-coupled receptors. This balance of upregulation/ downregulation is both tricky and receptor dependent, and consequently, current cAMP assays often do not provide an adequate detection window for Gi-receptor activation. The traditional functional cAMP assays of Gi-coupled GPCRs are usually carried out by stimulating adenylyl cyclase (AC) with forskolin or by activating a Gs-coupled receptor with its agonist, followed by activating Gi-coupled receptors to decrease the cAMP level. This kind of assay is figuratively dubbed as "upside down assay", as the activation of receptor brings down the fluorescence signal from a highly activated level. Our real-time cAMP assay made it possible to examine the entire process of AC activation, followed by the activation of DRD2 receptor, second by second (Fig. 28 A). Cells were stimulated with forskolin without a PDE inhibitor, followed by the addition of dopamine. The addition of dopamine resulted in an immediate reduction of the forskolin-induced fluorescence signal, nearly returning to the pre-forskolin level within about 7 min (Fig. 28 A). Figure 28A shows the kinetic response to dopamine in a CNG-DRD2 cell line measured in 96-well plates with FLEXstation plate reader. A maximal response to 1 µM dopamine was reached in about 400 seconds.

In addition to the kinetic analysis, end-point data were obtained by simultaneously incubating the cells with forskolin and dopamine for 15 min, followed by reading the fluorescence signals (Fig. 28 B). Figure 28B shows the results of an endpoint assay of CNG-DRD2 cells measured in 96-well plates with FLEXstation plate reader. Cells were treated with 10 µM forskolin and different concentrations of dopamine without a PDE inhibitor. Fluorescence readings were obtained before compound addition for baseline and 15 min after compound addition for induction.

As an alternative approach, we redirect Gi-coupled receptors to activate adenylyl cyclase through artificial Gs-i chimera proteins to form a "right-side up assay" as to Gs-coupled receptors (Komatsuzaki et al., 1997, FEBS Lett. 406:165-70; Drew et al., 2002, Biochim Biophys Acta. 1592:185-92). To accomplish this, we have constructed and introduced a collection of chimeric G-proteins, namely, Gs-i1, Gs-i3, Gs-iz, and Gs-io. This approach, while less physiological, has improved the assay signal and simplified the assay procedures, thereby enable HTS against certain otherwise intractable Gi-targets. The Gs-i approach is an analogous strategy to Gq-i approach that has been widely adopted in HTS (Milligan and Rees, 1999, TiPS 20:118-124). Moreover, the CNG-cAMP technology has several additional advantages over Gq-coupled assays on a FLIPR, including improved signal amplitude and signal stability. Such stable fluorescence signal can be detected with standard fluorescence plate readers, enabling broader adaptation of the technology.

The chimeric Gs-i proteins comprise N-terminal Gs with the last C-terminal 5 amino acid residues replaced by those derived from Gi1 (DCGLF; SEQ ID NO: 20), Gi3 (ECGLY; SEQ ID NO: 21), Gio (GCGLY; SEQ ID NO: 22) and Giz (YIGLC; SEQ ID NO: 23) proteins. In experiments designed to assess the potential of this strategy, we constructed Gs-i3 chimeric G-protein. By co-transfecting Gs-i3 and the somatostatin receptor 5 (SSTR5) into the HEK293-CNGA2*^{CE}* background, we observed SSTR5-dependent increase of fluorescence signal in kinetic study, an indication that the Gi-coupled SSTR5 was redirected to the Gs pathway, resulting in an increase in cellular cAMP (Fig. 28 C). The amplitude of the response for the transiently transfected SSTR5 receptor was similar to that of the Gs-receptors. Figure 28C shows the kinetic response to somatostatin (SST) in HEK293H-CNG cell line transiently co-transfected with a Gi-coupled SSTR5 receptor and Gs-i3 chimeric G-protein. Recording was made in a 96-well plate with FLEXstation. The cells were labeled with a MP dye. Upon stimulation with somatostatin, the fluorescence intensity was recorded for 3 min with FlexStation.

We also established a SSTR5 :: Gs-i3 stable cells line in the HEK293H-CNGA2 cells. Assay of this cell line revealed not only that Gs-i3 could redirect Gi-coupled SSTR5 through the Gs pathway, but also that the amplitude of the signal is comparable with that of Gs-receptor cell lines (Fig. 28 D). Figure 28D is a graph showing the results of an endpoint assay of responses to somatostatin (SST) in a cell line HEK293H-CNG:SSTR5::Gs-i3 stably expressing CNG, SSTR5 and a chimeric Gs-i3 protein. Recording was made in a 384-well plate with FLEXstation. The cells were labeled with a MP dye. The fluorescence intensity was recorded before (F₀) and 10 min (F₁₀,) after addition of somatostatin at different concentrations. Dose-response curve is created by fitting the data to the Hill equation.

### Example 19

### Application of the CNG cAMP Assay to Fluorescence Activated Cell Sorter (FACS)

FACS analysis and cell sorting are widely used in both clinical and basic researches. Measurement of the second messenger cAMP can provide a quick functional assay of biological responses of cells to antigens, hormones, and other varieties of chemical and physical stimuli. There are no effective approaches currently available to determine the cAMP level in the living cells with FACS and there are no small molecule indicators for cAMP. Further, the traditional cAMP competitive immunoassay can not be applied to FACS, because it does not provide single-cell sensitivity and requires cell lysis. Therefore a method is described here to combine use of a CNG channel as a cAMP sensor and use of calcium and membrane potential dyes. Protocols using FACS to monitor [Ca²⁺]i and membrane potential of cells are well established and can be easily adopted to monitor CNG channel activity (Lazzar et al., 1986, J Biol Chem, 261:9710-9713; Shapiro, 2000, Methods, 21:271-279).

To facilitate adaptation of CNG technology to FACS monitoring cAMP in living cells, it is desirable to develop a quick expression protocol of CNG channels in the cells to be assayed. It takes about 2-3 days to express CNG channels using the commercial cDNA liposome transfection kits (e.g. Lipofectamine™ 2000, FuGENE, and etc.). Further, the transfection efficiency using these commercial kits is variable with cell types with these kits. Therefore, these current transfection methods offer very limited use of CNG sensor technology. Using adenovirus to express CNG channel has been explored (Fagan et al., 2001, FEBS Lett. 500:85-90). Cells can be assayed at about 24 hr after infection of the adenovirus CNG channels. Methods enabling faster expression of the CNG channels are potentially more useful in FACS analysis of cAMP in living cells, which will not only shorten the assay period but also avoid potential toxigenic and apoptotic effects of CNG expression. A quantitative expression of GFP protein has been established by RNA transfection using an electroporation protocol (Teruel et al., 1999, J Neurosci Methods, 93:37-48). This RNA transfection method will allow a quick expression of CNG and cAMP assay in cell samples within 8 hrs. Other gene delivery methods can also be developed and applied to express CNG quickly in cells to be assayed (see Kaneda et al., 2002, Mol Ther., 6:219-226).

### Example 20

### Simultaneous Screening of Gs, Gi, and Gq_,GPCR with CNG channel technology for receptor de-orphanization and compound profiling

Of the greater than 750 human GPCR genes, at least 367 are predicted to bind endogenous ligands. Within this group, ligands have been identified for 224 GPCRs leaving 143 orphan receptors for which neither ligands nor functions are known *(*Vassilatis et al. 2003 Vassilatis DK, The G protein-coupled receptor repertoires of human and mouse. Proc Natl Acad Sci USA; 2003; 100:4903-4908). Because about 40% of all the marketed drugs are directed against only 30 GPCRs, it is reasonable to infer that a substantial number of potentially druggable GPCR targets are still present within the pool of functionally uncharacterized GPCRs. Thus, assays that would allow receptor de-orphanization [identification of a ligand (agonist or antagonist), coupling G-protein, and a function for an orphan receptor] are being actively sought in drug discovery. The CNG-technology described herein represents a novel, advantageous tool that enables orphan GPCR receptors to be screened in a universal calcium read-out format.

As demonstrated below, HEK 293-CNG cells expressing orphan GPCR DNAs (either endogeneous receptors, or transiently or stably transfected exogeneous receptors) can be directly tested for functional coupling of the receptor with Gq, Gs, or Gi. Based on the response, in particular the kinetics of the response, it is possible to both identify activated orphan receptors, and determine the G-protein coupling. Further, this approach does not require the need to introduce promiscuous G proteins (e.g. Gα16) that provide "artificial" coupling to Gs/i GPCRs that would not otherwise generate a calcium signal. In a preferred embodiment, this approach can be used to identify and deorphanize and determine the coupling of endogeneous receptors in a broad range of cell types into which a functional CNG can be introduced.

A protocol using HEK 293-CNG (SEQ ID No: 8) cells stably expressing the exogeneous Gi-coupled D2 dopamine receptor (LocusID 1813) was designed to test this model. Cells were plated in 96 well plates (15,000 cell/well), grown overnight, and labeled with the fluorescent calcium indicator Fura-2 (5µM). Single cell kinetic calcium imaging was performed using PathwayHT (Atto Bioscience), an automated imaging platform with liquid dispensing capabilities. Activation of Gq-coupled GPCRs results in release of intracellular calcium stores. In contrast, activation of Gs coupled receptors stimulate adenylyl cyclase, resulting in increasing levels of cyclic AMP (cAMP), while Gi coupled receptors inhibit adenylyl cyclases, reducing levels of cAMP.

As shown in Figure 29, the addition of a first compound (1), in this case carbachol (100µM), that activates the endogeneous Gq-coupled muscarinic receptor, induces a rapid and transient calcium rise (characteristic of Gq-mediated internal calcium release); activation of endogeneous Gs-coupled GPCR, the β-adrenergic receptor (in this case by isoproterenol, 1µM) triggers a slower but sustained calcium response (due to calcium influx through the cAMP-activated CNG channel), while activation of Gi-coupled D2 dopamine receptor (in this case, by 300nM dopamine) or addition of compound buffer alone does not affect calcium levels. A subsequent addition (2) of a known cAMP agonist [in this case 3µM forskolin, which directly activates adenylyl cyclase (AC)] is observed to increase cAMP levels in the control (compound buffer only), but not if the Gi-coupled D2 receptor is also activated (as would be expected if the activated Gi inhibits adenylyl cyclase). The extent of the signal reduction is expected to be proportional to the potency of the first test compound.

While cAMP levels were induced by directly stimulating AC in this example, it will be apparent to those skilled in the art that activation of any Gs-coupled receptor could also be used in this assay to activate AC. It can be further seen that the first transient calcium increase observed for the Gq-GPCRs is followed by a sustained calcium response (due to increasing levels of cAMP as a result of activating ACs, and subsequent activation of the CNG). For Gs-GPCRs, the first calcium response is either not affected (e.g. first ligand exhibits maximal potency/efficacy toward cAMP activation, as in the case shown) or is further enhanced. We have further shown that calcium imaging is unaffected (signal to noise/sensitivity) by the presence of a quenchers. Consequently, Gs/Gi/Gq screening with CNG-293 cells is feasible as a simple, two-step homogeneous format (dye loading and compound additions, no wash required) substantially increasing ease of use and speed. Hence, the current invention allows a simple two-step deorphanization and determination of receptor coupling.

In another preferred embodiment, the current invention is applicable to ligand profiling screens. In this application, compounds are tested for their effect (agonist/antagonist/allosteric modulators) on collections of GPCRs, typically receptor families. As described above, agonists, inhibitors of agonists, or modulators of agonists or antagonists can be surveyed by contacting cells expressing receptors of interest with compounds and assessing the response. Because the kinetics of each response is indicative not only of agonist/antagonist or modulator, but also the coupling, combinatorial assays that in addition generate deorphanization and receptor coupling results can be developed.

In yet another embodiment, imaging-based deorphanization or ligand profiling screens using the current invention can be done in 96 well plates, 384 well plates, 1536 well plates or in implemented on a cell microarray platform (where multiple GPCR genes are introduced in spatially discrete locations, and cells are grown over the DNA to provide an array of cell populations, wherein each population within a spot can contain a different GPCR. Said arrays can be generated within the same well of a microtiter plate). This approach provides unprecedented speed and multiplexing for receptor assays using the current invention, including de-orphanization and ligand profiling screening.

## Claims

1. An isolated polynucleotide comprising a sequence encoding a mutant cyclic nucleotide-gated (CNG) channel comprising:
(a) at least one mutation that decreases divalent cation-mediated blockage of cation flux; and
(b) at least one mutation that makes the channel more sensitive to cAMP than a channel that does not comprise the mutation,
wherein said mutant CNG channel is a mutant of rat CNGA2 comprising SEQ ID NO:2, wherein said at least one mutation that decreases divalent cation-mediated blockage of cation flux comprises a mutation at position E342, and wherein said at least one mutation that makes the channel more sensitive to cAMP than a channel that does not comprise the mutation comprises a mutation at position C460 and/or E583.

2. The isolated polynucleotide of claim 1, wherein said at least one mutation that decreases divalent cation-mediated blockage of cations is selected from the group consisting of E342G and E342N.

3. The isolated polynucleotide of claim 1 or claim 2, wherein said at least one mutation that makes the channel more sensitive to cAMP than a channel that does not comprise the mutation is selected from the group consisting of C460R, C460H and E583L.

4. A vector comprising the isolated polynucleotide of claim 1 operably linked to a promoter.

5. The vector of claim 4, wherein said vector is selected from the group consisting of expression plasmids, retroviruses and adenoviruses.

6. A host cell comprising a first nucleic acid comprising the isolated polynucleotide of any one of claims 1-3.

7. The host cell of claim 6, wherein said isolated polynucleotide is stably integrated into in a host chromosome.

8. The host cell of claim 6, wherein the cell expresses at least a first endogeneous GPCR.

9. The host cell of claim 8, further comprising a second nucleic acid comprising a promoter operably linked to a second polynucleotide wherein the second polynucleotide comprises a sequence encoding an heterologous G protein-coupled receptor (GPCR).

10. A host cell according to claim 8, wherein the endogenous GPCR is overexpressed.

11. A host cell according to claim 9, wherein the first nucleic acid and the second nucleic acid are part of one molecule or different molecules.

12. A host cell according to claim 9, wherein at least one of the first and second nucleic acids are selected from the group consisting of viruses and plasmids.

13. A host cell according to claim 9, wherein at least one of the first and the second nucleic acids is stably integrated in the genome of the cell.

14. A host cell according to claim 9, wherein at least one of the first and the second nucleic acids is not part of the genome of the cell.

15. A host cell according to claim 8, wherein the host cell is a mammalian cell.

16. A host cell according to claim 6 or claim 15, wherein the cell is selected from the group consisting of BHK cells, mouse L cells, Jurkat cells, 153DG44 cells, HEK cells, CHO cells, PC12 cells, human T-lymphocyte cells, Cos-7 cells and primary cells.

17. A host cell according to claim 8, wherein the mutant cyclic nucleotide-gated channel comprises mutations at both positions C460 and E583.

18. A host cell according to claim 17, wherein the mutant cyclic nucleotide-gated channel comprises at least three mutations that make the channel more sensitive to cAMP than a channel that does not comprise the mutations.

19. A host cell according to claim 6, wherein said host cell further expresses at least one additional CNG subunit selected from the group consisting of CNGA4 and CNG4.3.

20. A host cell according to claim 9, wherein the second polynucleotide comprises a sequence encoding a mutated or truncated G protein-coupled receptor.

21. A host cell according to claim 8 or claim 9, further comprising a nucleic acid comprising a promoter operably linked to a polynucleotide comprising a sequence encoding a G protein that interacts with at least one GPCR in said host cell.

22. A host cell according to claim 21, wherein the G protein is selected from a group consisting of Gₛ, Gᵢ, G_{q}, G_{olf}, Gₒ, G₁₂ and Gₛ₋ᵢ chimeric G proteins.

23. A host cell according to claim 8 or 9 further comprising at least one of a polynucleotide comprising a sequence encoding a chimeric G-protein, a heterologous G-protein, and a heterologous adenylyl cyclase.

24. A host cell according to claim 23 wherein at least two of said polynucleotide coding sequences are on a single vector.

25. A host cell according to claim 24 wherein the vector is selected from the group consisting of a plasmid or a virus.

26. A host cell according to claim 23 wherein the adenylyl cyclase is selected from the group consisting of adenylyl cyclase type V and type VI.

27. A method of detecting changes in intracellular levels of cAMP, comprising:
(a) expressing in a cell the polynucleotide of claim 1; and
(b) measuring activity of the channel wherein activity of the channel is indicative of changes in intracellular cAMP.

28. The method of claim 27, wherein said activity is measured using an indicator selected from the group consisting of membrane potential indicators and cation-sensitive indicators.

29. The method of claim 28, wherein said indicator is selected from the group consisting of fluorescent and luminescent indicators.

30. The method of claim 28, wherein said cation is selected from the group consisting of calcium, sodium and potassium.

31. The method of claim 27, wherein the method is selected from the group consisting of microplate assays, cell imaging analysis, FACS analysis, radioisotope analysis and electrophysiology platforms.

32. A method of detecting activity of a GPCR, comprising:
(a) expressing the CNG channel and the GPCR in the host cell of claim 8 or 9; and
(b) measuring activity of the channel wherein activity of the channel indicates activity of the GPCR.

33. A method of identifying a ligand for a receptor, comprising:
(a) contacting a cell with a compound wherein the cell expresses the receptor and the mutant CNG channel of any one of claims 1 to 3; and
(b) measuring activation of the CNG channel, wherein activation of the CNG channel indicates that the compound is a ligand for the receptor.

34. A method of identifying an agent that modulates an activity mediated by a GPCR comprising:
(a) contacting the host cell of claim 8 with the agent and a ligand for the receptor;
(b) measuring activation of the CNG channel.

35. A method according to claim 34, further comprising:
(c) comparing activation of the CNG channel to activation of the channel in the absence of the agent, wherein a difference in activation of the CNG channel indicates the agent modulates the activity.

36. A kit comprising a container containing a cell according to claim 6.

37. A kit according to claim 36, further comprising at least one reagent selected from a group consisting of buffers, salts, quenchers and indicators.

38. A kit according to claim 37, further comprising at least one indicator selected from a group consisting of voltage sensitive indicators and cation sensitive indicators.

## Patentansprüche

1. Isoliertes Polynucleotid, das eine Sequenz umfasst, die für einen mutierten HCN-Kanal ("cyclic nucleotide-gated channel") kodiert, der Folgendes umfasst:
(a) zumindest eine Mutation, die die durch zweiwertige Kationen vermittelte Blockade eines Kationenflusses vermindert,
(b) zumindest eine Mutation, die den Kanal gegenüber cAMP empfindlicher macht als einen Kanal, der die Mutation nicht umfasst,
worin der mutierte HCN-Kanal eine Mutante von Ratten-CNGA2 ist, die Seq.-ID Nr. 2 umfasst, worin die zumindest eine Mutation, die die durch zweiwertige Kationen vermittelte Blockade eines Kationenflusses vermindert, eine Mutation an Position E342 umfasst und worin die zumindest eine Mutation, die den Kanal gegenüber cAMP empfindlicher macht als einen Kanal, der die Mutation nicht umfasst, eine Mutation an Position C460 und/oder E583 umfasst.

2. Isoliertes Polynucleotid nach Anspruch 1, worin die zumindest eine Mutation, die die durch zweiwertige Kationen vermittelte Blockade von Kationen vermindert, aus der aus E342G und E342N bestehenden Gruppe ausgewählt ist.

3. Isoliertes Polynucleotid nach Anspruch 1 oder Anspruch 2, worin die zumindest eine Mutation, die den Kanal gegenüber cAMP empfindlicher macht als einen Kanal, der die Mutation nicht umfasst, aus der aus C460R, C460H und E583L bestehenden Gruppe ausgewählt ist.

4. Vektor, der das isolierte Polynucleotid nach Anspruch 1 operabel gebunden an einen Promotor umfasst.

5. Vektor nach Anspruch 4, worin der Vektor aus der aus Expressionsplasmiden, Retroviren und Adenoviren bestehenden Gruppe ausgewählt ist.

6. Wirtszelle, die eine erste Nucleinsäure umfasst, die das isolierte Polynucleotid nach einem der Ansprüche 1 bis 3 umfasst.

7. Wirtszelle nach Anspruch 6, worin das isolierte Polynucleotid stabil in ein Wirtschromosom integriert ist.

8. Wirtszelle nach Anspruch 6, worin die Zelle zumindest einen ersten endogenen GPCR exprimiert.

9. Wirtszelle nach Anspruch 8, weiters umfassend eine zweite Nucleinsäure, die einen operabel an ein zweites Polynucleotid gebundenen Promotor umfasst, worin das zweite Polynucleotid eine Sequenz umfasst, die für einen heterologen G-Protein-gekoppelten Rezeptor (GPCR) kodiert.

10. Wirtszelle nach Anspruch 8, worin der endogene GPCR überexprimiert wird.

11. Wirtszelle nach Anspruch 9, worin die erste Nucleinsäure und die zweite Nucleinsäure beide zu einem Molekül oder zu jeweils verschiedenen Molekülen gehören.

12. Wirtszelle nach Anspruch 9, worin zumindest eine von erster Nucleinsäure und zweiter Nucleinsäure aus der aus Viren und Plasmiden bestehenden Gruppe ausgewählt ist.

13. Wirtszelle nach Anspruch 9, worin zumindest eine von erster Nucleinsäure und zweiter Nucleinsäure stabil in das Genom der Zelle integriert ist.

14. Wirtszelle nach Anspruch 9, worin zumindest eine von erster Nucleinsäure und zweiter Nucleinsäure nicht Teil des Genoms der Zelle ist.

15. Wirtszelle nach Anspruch 8, worin die Wirtszelle eine Säugetierzelle ist.

16. Wirtszelle nach Anspruch 6 oder Anspruch 15, worin die Zelle aus der aus BHK-Zellen, murinen L-Zellen, Jurkat-Zellen, 153DG44-Zellen, HEK-Zellen, CHO-Zellen, PC12-Zellen, menschlichen T-Lymphozytenzellen, Cos-7-Zellen und Primärzellen bestehenden Gruppe ausgewählt ist.

17. Wirtszelle nach Anspruch 8, worin der mutierte HCN-Kanal Mutationen an den beiden Positionen C460 und E583 umfasst.

18. Wirtszelle nach Anspruch 17, worin der mutierte HCN-Kanal zumindest drei Mutationen umfasst, die den Kanal gegenüber cAMP empfindlicher machen als einen Kanal, der die Mutationen nicht umfasst.

19. Wirtszelle nach Anspruch 6, worin die Wirtszelle weiters zumindest eine zusätzliche aus der aus CNGA4 und CNG4.3 bestehenden Gruppe ausgewählte CNG-Untereinheit umfasst.

20. Wirtszelle nach Anspruch 9, worin das zweite Polynucleotid eine Sequenz umfasst, die für einen mutierten oder trunkierten G-Protein-gekoppelten Rezeptor kodiert.

21. Wirtszelle nach Anspruch 8 oder Anspruch 9, die weiters eine Nucleinsäure umfasst, die einen Promotor umfasst, der operabel an ein Polynucleotid gebunden ist, das eine Sequenz umfasst, welche für ein G-Protein kodiert, das mit zumindest einem GPCR in der Wirtszelle wechselwirkt.

22. Wirtszelle nach Anspruch 21, worin das G-Protein aus einer aus den chimären G-Proteinen Gₛ, Gᵢ, G_{q}, G_{olf}, Gₒ, G₁₂ und Gₛ₋ᵢ bestehenden Gruppe ausgewählt ist.

23. Wirtszelle nach Anspruch 8 oder 9, die weiters zumindest eines von einem Polynucleotid, das eine Sequenz umfasst, die für ein chimäres G-Protein kodiert, einem heterologen G-Protein und einer heterologen Adenylylcyclase umfasst.

24. Wirtszelle nach Anspruch 23, worin zumindest zwei der Polynucleotid-Kodiersequenzen sich auf einem einzigen Vektor befinden.

25. Wirtszelle nach Anspruch 24, worin der Vektor aus der aus einem Plasmid und einem Virus bestehenden Gruppe ausgewählt ist.

26. Wirtszelle nach Anspruch 23, worin die Adenylylcyclase aus der aus Adenylylcyclase des Typs V und des Typs VI bestehenden Gruppe ausgewählt ist.

27. Verfahren zur Detektion von Änderungen der intrazellulären Mengen von cAMP, umfassend:
(a) das Exprimieren des Polynucleotids nach Anspruch 1 in einer Zelle und
(b) das Messen der Aktivität des Kanals, worin Aktivität des Kanals ein Indikator für Änderungen des intrazellulären cAMP ist.

28. Verfahren nach Anspruch 27, worin die Aktivität unter Verwendung eines aus der aus Membranpotenzialindikatoren und kationenempfindlichen Indikatoren bestehenden Gruppe ausgewählten Indikators gemessen wird.

29. Verfahren nach Anspruch 28, worin der Indikator aus der aus fluoreszierenden und lumineszierenden Indikatoren bestehenden Gruppe ausgewählt ist.

30. Verfahren nach Anspruch 28, worin das Kation aus der aus Calcium, Natrium und Kalium bestehenden Gruppe ausgewählt ist.

31. Verfahren nach Anspruch 27, worin das Verfahren aus der aus Mikroplattentests, Zellbildgebungstests, FACS-Analyse, Radioisotopenanalyse und Elektrophysiologieplattformen bestehenden Gruppe ausgewählt ist.

32. Verfahren zur Detektion der Aktivität eines GPCR, umfassend:
(a) das Exprimieren eines HCN-Kanals und des GPCR in einer Wirtszelle nach Anspruch 8 oder 9 und
(b) das Messen der Aktivität des Kanals, worin Aktivität des Kanals ein Indikator für Aktivität des GPCR ist.

33. Verfahren zur Identifizierung eines Liganden für einen Rezeptor, umfassend:
(a) das Kontaktieren einer Zelle mit einer Verbindung, worin die Zelle einen Rezeptor und einen mutierten HCN-Kanal nach einem der Ansprüche 1 bis 3 exprimiert, und
(b) das Messen der Aktivierung des HCN-Kanals, worin die Aktivierung des HCN-Kanals ein Indikator dafür ist, dass die Verbindung ein Ligand für den Rezeptor ist.

34. Verfahren zur Identifizierung eines Mittels, das eine durch einen GPCR vermittelte Aktivität moduliert, umfassend:
(a) das Kontaktieren einer Wirtszelle nach Anspruch 8 mit dem Mittel und einem Liganden für den Rezeptor,
(b) das Messen der Aktivierung des HCN-Kanals.

35. Verfahren nach Anspruch 34, weiters umfassend:
(c) das Vergleichen der Aktivierung des HCN-Kanals mit der Aktivierung des Kanals in Abwesenheit des Mittels, worin ein Unterschied bei der Aktivierung des HCN-Kanals ein Indikator dafür ist, dass das Mittel die Aktivität moduliert.

36. Set, das einen Behälter umfasst, der eine Zelle nach Anspruch 6 enthält.

37. Set nach Anspruch 36, das weiters zumindest ein aus der aus Puffern, Salzen, Quenchern und Indikatoren bestehenden Gruppe ausgewähltes Reagens umfasst.

38. Set nach Anspruch 37, das weiters zumindest einen aus der aus spannungsempfindlichen Indikatoren und kationenempfindlichen Indikatoren bestehenden Gruppe ausgewählten Indikator umfasst.

## Revendications

1. Polynucléotide isolé comprenant une séquence codant pour un canal déclenché par un nucléotide cyclique mutant (CNG), comprenant:
(a) au moins une mutation qui diminue un blocage divalent régulé par cations du flux de cations; et
(b) au moins une mutation qui rend le canal plus sensible à cAMP qu'un canal qui ne comprend pas la mutation,
où ledit canal CNG mutant est un mutant de rat CNGA2 comprenant la SEQ ID NO:2, où ladite au moins une mutation qui diminue le blocage divalent régulé par cations du flux de cations comprend une mutation à la position E342, et où ladite au moins une mutation qui rend le canal plus sensible à cAMP qu'un canal qui ne comprend pas la mutation, comprend une mutation à la position C460 et/ou E583.

2. Polynucléotide isolé selon la revendication 1, où ladite au moins une mutation qui diminue le blocage divalent régulé par cations des cations est sélectionnée dans le groupe consistant en E342G et E342N.

3. Polynucléotide isolé selon la revendication 1 ou la revendication 2, où ladite au moins une mutation qui rend le canal plus sensible à cAMP qu'un canal qui ne comprend pas la mutation est sélectionnée dans le groupe consistant en C460R, C460H et E583L.

4. Vecteur comprenant le polynucléotide isolé selon la revendication 1, fonctionnellement lié à un promoteur.

5. Vecteur selon la revendication 4, où ledit vecteur est sélectionné dans le groupe consistant en plasmides d'expression, rétrovirus et adénovirus.

6. Cellule hôte comprenant un premier acide nucléique comprenant le polynucléotide isolé selon l'une quelconque des revendications 1-3.

7. Cellule hôte selon la revendication 6, où ledit polynucléotide isolé est intégré d'une manière stable dans un chromosome hôte.

8. Cellule hôte selon la revendication 6, où la cellule exprime au moins un premier GPCR endogène.

9. Cellule hôte selon la revendication 8, comprenant en outre un deuxième acide nucléique comprenant un promoteur fonctionnellement lié à un deuxième polynucléotide, où le deuxième polynucléotide comprend une séquence codant pour une récepteur hétérologue couplé à une protéine G (GPCR).

10. Cellule hôte selon la revendication 8, où le GPCR endogène est surexprimé.

11. Cellule hôte selon la revendication 9, où le premier acide nucléique et le deuxième acide nucléique font partie d'une molécule ou de molécules différentes.

12. Cellule hôte selon la revendication 9, où au moins un des premier et deuxième acides nucléiques sont sélectionnés dans le groupe consistant en virus et plasmides.

13. Cellule hôte selon la revendication 9, où au moins un des premier et deuxième acides nucléiques est intégré d'une manière stable dans le génome de la cellule.

14. Cellule hôte selon la revendication 9, où au moins un des premier et deuxième acides nucléiques ne fait pas partie du génome de la cellule.

15. Cellule hôte selon la revendication 8, où la cellule hôte est une cellule mammalienne.

16. Cellule hôte selon la revendication 6 ou la revendication 15, où la cellule est sélectionnée dans le groupe consistant en cellules BHK, cellules L de souris, cellules Jurkat, cellules 153DG44, cellules HEK, cellules CHO, cellules PC12, cellules de lymphocytes-T humaines, cellules Cos-7 et cellules primaires.

17. Cellule hôte selon la revendication 8, où le canal déclenché par un nucléotide cyclique mutant comprend des mutations à la fois aux positions C460 et E583.

18. Cellule hôte selon la revendication 17, où le canal déclenché par un nucléotide cyclique mutant comprend au moins trois mutations qui rendent le canal plus sensible au cAMP qu'un canal qui ne comprend pas les mutations.

19. Cellule hôte selon la revendication 6, où ladite cellule hôte exprime en outre au moins une sous-unité CNG additionnelle sélectionnée dans le groupe consistant en CNGA4 et CNG4.3.

20. Cellule hôte selon la revendication 9, où le deuxième polynucléotide comprend une séquence codant pour un récepteur muté ou tronqué couplé à la protéine G.

21. Cellule hôte selon la revendication 8 ou la revendication 9, comprenant en outre un acide nucléique comprenant un promoteur fonctionnellement lié à un polynucléotide comprenant une séquence codant pour une protéine G qui est en interaction avec au moins un GPCR dans ladite cellule hôte.

22. Cellule hôte selon la revendication 21, où la protéine G est sélectionnée d'un groupe consistant en protéine Gₛ, Gᵢ, G_{q}, G_{olf}, Gₒ, G₁₂ et Gₛ₋ᵢ chimériques.

23. Cellule hôte selon la revendication 8 ou 9, comprenant en outre au moins un d'un polynucléotide comprenant une séquence codant pour une protéine G chimérique, une protéine G hétérologue et une adénylyle cyclase hétérologue.

24. Cellule hôte selon la revendication 23, où au moins deux desdites séquences de codage de polynucléotide sont sur un seul vecteur.

25. Cellule hôte selon la revendication 24, où le vecteur est sélectionné dans le groupe consistant en un plasmide ou un virus.

26. Cellule hôte selon la revendication 23, où l'adénylyle cyclase est sélectionnée dans le groupe consistant en adénylyle cyclase de type V et de type VI.

27. Méthode pour détecter des changements dans des niveaux intracellulaires de cAMP, comprenant:
(a) exprimer dans une cellule le polynucléotide de la revendication 1; et
(b) mesurer l'activité du canal, où l'activité du canal est indicative de changements dans le cAMP intracellulaire.

28. Méthode selon la revendication 27, où ladite activité est mesurée en utilisant un indicateur sélectionné dans le groupe consistant en indicateurs de potentiel de membrane et indicateurs réagissant aux cations.

29. Méthode selon la revendication 28, où ledit indicateur est sélectionné dans le groupe consistant en indicateurs fluorescents et luminescents.

30. Méthode selon la revendication 28, où ledit cation est sélectionné dans le groupe consistant en calcium, sodium et potassium.

31. Méthode selon la revendication 27, où la méthode est sélectionnée dans le groupe consistant en essais sur microplaque, l'analyse de l'imagerie de la cellule, l'analyse FACS, l'analyse radioisotopique et des plateformes d'électrophysiologie.

32. Méthode de détection de l'activité d'un GPCR, comprenant:
(a) exprimer le canal CNG et le GPCR dans la cellule hôte de la revendication 8 ou 9; et
(b) mesurer l'activité du canal, où l'activité du canal indique l'activité du GPCR.

33. Méthode d'identification d'un ligand pour un récepteur, comprenant:
(a) mettre en contact une cellule avec un composé, où la cellule exprime le récepteur et le canal CNG mutant selon l'une quelconque des revendications 1 à 3; et
(b) mesurer l'activation du canal CNG, où l'activation du canal CNG indique que le composé est un ligand pour le récepteur.

34. Méthode d'identification d'un agent qui module une activité régulée par un GPCR comprenant:
(a) mettre en contact la cellule hôte de la revendication 8 avec l'agent et un ligand pour le récepteur;
(b) mesurer l'activation du canal CNG.

35. Méthode selon la revendication 34, comprenant en outre:
(c) comparer l'activation du canal CNG avec l'activation du canal en l'absence de l'agent, où une différence dans l'activation du canal CNG indique que l'agent module l'activité.

36. Kit comprenant un contenant qui contient une cellule selon la revendication 6.

37. Kit selon la revendication 36, comprenant en outre au moins un réactif sélectionné dans un groupe consistant en tampons, sels, refroidisseurs brusques et indicateurs.

38. Kit selon la revendication 37, comprenant en outre au moins un indicateur sélectionné dans un groupe consistant en indicateurs réagissant à la tension et indicateurs réagissant aux cations.
